(19) Europäisches Patentamt
European Patent Office
Office européen des brevets

(11) EP 3 573 973 B1

(12) EUROPÄISCHE PATENTSCHRIFT

(45) Veröffentlichungstag und Bekanntmachung des Hinweises auf die Patenterteilung:
**06.12.2023 Patentblatt 2023/49**

(21) Anmeldenummer: **18700516.0**

(22) Anmeldetag: **22.01.2018**

(51) Internationale Patentklassifikation (IPC):
***C07D 405/14*** (2006.01) ***C07F 9/6584*** (2006.01)
***C07D 401/04*** (2006.01) ***C07D 403/04*** (2006.01)
***C07D 403/10*** (2006.01) ***C07D 403/14*** (2006.01)
***C07D 405/10*** (2006.01) ***C07D 409/14*** (2006.01)
***C07D 471/04*** (2006.01) ***C07D 471/16*** (2006.01)
***C07D 487/06*** (2006.01) ***C07D 491/06*** (2006.01)
***C07D 491/16*** (2006.01) ***C07D 491/22*** (2006.01)
***C07D 495/06*** (2006.01) ***C07D 495/16*** (2006.01)
***C07D 209/80*** (2006.01) ***C07F 7/08*** (2006.01)
***C09K 11/06*** (2006.01)

(52) Gemeinsame Patentklassifikation (CPC):
**C07D 209/80; C07D 401/04; C07D 403/04; C07D 403/10; C07D 403/14; C07D 405/10; C07D 405/14; C07D 409/14; C07D 471/04; C07D 471/06; C07D 471/16; C07D 487/06; C07D 491/06; C07D 491/16; C07D 491/22;** (Forts.)

(86) Internationale Anmeldenummer:
**PCT/EP2018/051407**

(87) Internationale Veröffentlichungsnummer:
**WO 2018/138039 (02.08.2018 Gazette 2018/31)**

(54) **CARBAZOLDERIVATE**

CARBAZOLE DERIVATIVES

DÉRIVÉS DE CARBAZOLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.01.2017 EP 17152962**

(43) Veröffentlichungstag der Anmeldung:
**04.12.2019 Patentblatt 2019/49**

(73) Patentinhaber: **Merck Patent GmbH**
**64293 Darmstadt (DE)**

(72) Erfinder:
- **PARHAM, Amir**
  **60486 Frankfurt am Main (DE)**
- **LUDEMANN, Aurélie**
  **60322 Frankfurt am Main (DE)**
- **JOOSTEN, Dominik**
  **60487 Frankfurt am Main (DE)**
- **GROSSMANN, Tobias**
  **64297 Darmstadt (DE)**
- **KROEBER, Jonas**
  **60311 Frankfurt am Main (DE)**

(56) Entgegenhaltungen:
**CN-A- 104 513 246** **KR-A- 20110 041 726**
**KR-A- 20110 113 468** **KR-A- 20120 081 539**
**KR-A- 20130 080 827** **KR-A- 20150 065 383**
**KR-A- 20160 062 603** **US-A1- 2015 380 661**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 573 973 B1

- **MOSS G P ET AL: "Glossary of class names of organic compounds and reactive intermediates based on structure (IUPAC Recommendations 1995)", PURE & APPLIED CHEMISTRY, PERGAMON PRESS, OXFORD, GB, vol. 67, no. 8/9, 1 January 1995 (1995-01-01), pages 1307-1375, XP007914773, ISSN: 0033-4545, DOI: 10.1351/PAC199567081307**

(52) Gemeinsame Patentklassifikation (CPC): (Forts.)
**C07D 495/06; C07D 495/16; C07D 519/00; C07F 7/0816; C07F 9/65685; C09K 11/06; H10K 85/40; H10K 85/654; H10K 85/657; H10K 85/6572; H10K 85/6574;** H10K 50/11; H10K 50/165; H10K 85/342; H10K 2101/10; Y02E 10/549

**Beschreibung**

Die vorliegende Erfindung beschreibt Carbazolderivate, insbesondere zur Verwendung in elektronischen Vorrichtungen. Die Erfindung betrifft ferner ein Verfahren zur Herstellung der erfindungsgemäßen Verbindungen sowie elektronische Vorrichtungen enthaltend diese Verbindungen.

In organischen Elektrolumineszenzvorrichtungen (OLEDs) werden als emittierende Materialien häufig metallorganische Komplexe eingesetzt, die Phosphoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Phosphoreszenz zeigen, immer noch Verbesserungsbedarf, beispielsweise im Hinblick auf Effizienz, Betriebsspannung und Lebensdauer.

Die Eigenschaften organischer elektrolumineszierender Vorrichtungen werden nicht nur durch die eingesetzten Emitter bestimmt. Hier sind insbesondere auch die anderen verwendeten Materialien, wie Host-/Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können zu deutlichen Verbesserungen elektrolumineszierender Vorrichtungen führen.

Gemäß dem Stand der Technik werden als Matrixmaterialien für phosphoreszierende Verbindungen beispielsweise Carbazolderivate verwendet, wobei auch Verbindungen bekannt sind, die sowohl Carbazol-Strukturen als auch Strukturen aufweisen, die von Benzoxanthen abgeleitet sind. Je nach Substitution können diese Verbindungen als Elektronentransportmaterialien eingesetzt werden. Beispielsweise sind in KR 2015/0065383 entsprechende Verbindungen beschrieben. Allerdings umfassen die beschriebenen Benzoxanthenderivate eine Naphthalinstruktur, mit der eine Carbazolgruppe gebildet wird. Weitere entsprechende Verbindungen, bei denen eine Indolgruppe an eine Naphthylgruppe ankondensiert ist, sind in KR 2016 0062603 A und KR 2011 0113468 A beschrieben.

Generell besteht bei diesen Materialien, beispielsweise für die Verwendung als Matrixmaterialien, Lochleitermaterialien oder Elektronentransportmaterialien noch Verbesserungsbedarf, insbesondere in Bezug auf die Lebensdauer, aber auch in Bezug auf die Effizienz und die Betriebsspannung der Vorrichtung. Ferner sollten die Verbindungen eine hohe Farbreinheit aufweisen.

Aufgabe der vorliegenden Erfindung ist daher die Bereitstellung von Verbindungen, welche sich für den Einsatz in einer organischen elektronischen Vorrichtung, insbesondere in einer organischen Elektrolumineszenzvorrichtung, eignen und welche bei Verwendung in dieser Vorrichtung zu guten Device-Eigenschaften führen, sowie die Bereitstellung der entsprechenden elektronischen Vorrichtung.

Insbesondere ist es die Aufgabe der vorliegenden Erfindung, Verbindungen zur Verfügung zu stellen, die zu hoher Lebensdauer, guter Effizienz und geringer Betriebsspannung führen. Gerade auch die Eigenschaften der Matrixmaterialien, der Lochleitermaterialien oder der Elektronentransportmaterialien haben einen wesentlichen Einfluss auf die Lebensdauer und die Effizienz der organischen Elektrolumineszenzvorrichtung.

Eine weitere Aufgabe der vorliegenden Erfindung kann darin gesehen werden, Verbindungen bereitzustellen, welche sich für den Einsatz in einer phosphoreszierenden oder fluoreszierenden OLED eignen, insbesondere als Matrixmaterial. Insbesondere ist es eine Aufgabe der vorliegenden Erfindung, Matrixmaterialien bereitzustellen, welche sich für rot, gelb und grün phosphoreszierende OLEDs eignen.

Weiterhin sollten die Verbindungen, insbesondere bei ihrem Einsatz als Matrixmaterialien, als Lochleitermaterialien oder als Elektronentransportmaterialien in organischen Elektrolumineszenzvorrichtung zu Vorrichtungen führen, die eine ausgezeichnete Farbreinheit aufweisen.

Weiterhin sollten sich die Verbindungen möglichst einfach verarbeiten lassen, insbesondere eine gute Löslichkeit und Filmbildung zeigen. Beispielsweise sollten die Verbindungen eine erhöhte Oxidationsstabilität und eine verbesserte Glasübergangstemperatur zeigen.

Eine weitere Aufgabe kann darin gesehen werden, elektronische Vorrichtungen mit einer ausgezeichneten Leistungsfähigkeit möglichst kostengünstig und in konstanter Qualität bereitzustellen

Weiterhin sollten die elektronischen Vorrichtungen für viele Zwecke eingesetzt oder angepasst werden können. Insbesondere sollte die Leistungsfähigkeit der elektronischen Vorrichtungen über einen breiten Temperaturbereich erhalten bleiben.

Überraschend wurde gefunden, dass bestimmte, nachfolgend näher beschriebene Verbindungen diese Aufgaben lösen und den Nachteil aus dem Stand der Technik beseitigen. Die Verwendung der Verbindungen führt zu sehr guten Eigenschaften organischer elektronischer Vorrichtungen, insbesondere von organischen Elektrolumineszenzvorrichtungen, insbesondere hinsichtlich der Lebensdauer, der Effizienz und der Betriebsspannung. Elektronische Vorrichtungen, insbesondere organische Elektrolumineszenzvorrichtungen, welche derartige Verbindungen enthalten, sowie die entsprechenden bevorzugten Ausführungsformen sind daher Gegenstand der vorliegenden Erfindung.

Gegenstand der vorliegenden Erfindung ist daher eine Verbindung, umfassend mindestens eine Struktur gemäß der folgenden Formel (I),

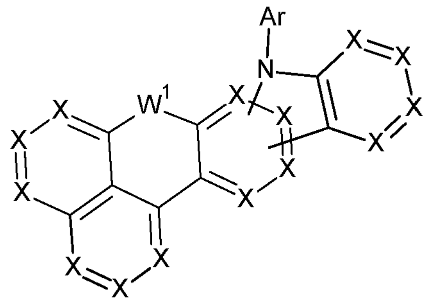

Formel (I)

wobei für die verwendeten Symbole gilt:

X ist bei jedem Auftreten gleich oder verschieden N oder $CR^1$, vorzugsweise $CR^1$, oder C, falls an X die Indologruppe gebunden ist;

$W^1$ ist O, S, $C(R^1)_2$, oder $Si(R^1)_2$, vorzugsweise O, S, oder $C(R^1)_2$, besonders bevorzugt O, S oder $C(R^1)_2$, ganz besonders bevorzugt O oder S;

$R^1$ ist bei jedem Auftreten gleich oder verschieden H, D, F, Cl, Br, I, CN, $NO_2$, $N(Ar^1)_2$, $N(R^2)_2$, $C(=O)Ar^1$, $C(=O)R^2$, $P(=O)(Ar^1)_2$, $P(Ar^1)_2$, $B(Ar^1)_2$, $B(OR^2)_2$, $Si(Ar^1)_3$, $Si(R^2)_3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Alkenylgruppe mit 2 bis 40 C-Atomen, worunter auch Cyclopentenyl, Cyclohexenyl, Cycloheptenyl, Cyclooctenyl und Cyclooctadienyl fallen, die jeweils mit einem oder mehreren Resten $R^2$ substituiert sein kann, wobei eine oder mehrere nicht benachbarte $CH_2$-Gruppen durch $-R^2C=CR^2-$, -C=C-, $Si(R^2)_2$, $Ge(R^2)_2$, $Sn(R^2)_2$, C=O, C=S, C=Se, $C=NR^2$, -C(=O)O-, $-C(=O)NR^2-$, $NR^2$, $P(=O)(R^2)$,-O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^2$ substituiert sein kann, oder eine Aryloxy- oder Heteroaryloxygruppe mit 5 bis 40 aromatischen Ringatomen, die durch einen oder mehrere Reste $R^2$ substituiert sein kann, oder eine Aralkyl- oder Heteroaralkylgruppe mit 5 bis 40 aromatischen Ringatomen, die mit einem oder mehreren Resten $R^2$ substituiert sein kann, oder eine Kombination dieser Systeme;

Ar ist bei jedem Auftreten gleich oder verschieden ein aromatisches oder heteroaromatisches Ringsystem mit 5 bis 60 aromatischen Ringatomen, das mit einem oder mehreren, vorzugsweise nichtaromatischen Resten $R^1$ substituiert sein kann;

$Ar^1$ ist bei jedem Auftreten gleich oder verschieden ein aromatisches oder heteroaromatisches Ringsystem mit 5 bis 30 aromatischen Ringatomen, das mit einem oder mehreren, vorzugsweise nichtaromatischen Resten $R^2$ substituiert sein kann, dabei können zwei Reste $Ar^1$, welche an dasselbe Si-Atom, N-Atom, P-Atom oder B-Atom binden, auch durch eine Einfachbindung oder eine Brücke, ausgewählt aus $B(R^2)$, $C(R^2)_2$, $Si(R^2)_2$, C=O, $C=NR^2$, $C=C(R^2)_2$, O, S, S=O, $SO_2$, $N(R^2)$, $P(R^2)$ und $P(=O)R^2$, miteinander verbrückt sein;

$R^2$ ist bei jedem Auftreten gleich oder verschieden H, D, F, Cl, Br, I, CN, $B(OR^3)_2$, $NO_2$, $C(=O)R^3$, $CR^3=C(R^3)_2$, $C(=O)OR^3$, $C(=O)N(R^3)_2$, $Si(R^3)_3$, $P(R^3)_2$, $B(R^3)_2$, $N(R^3)_2$, $NO_2$, $P(=O)(R^3)_2$, $OSO_2R^3$, $OR^3$, $S(=O)R^3$, $S(=O)_2R^3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $-R^3C=CR^3-$, -C=C-, $Si(R^3)_2$, $Ge(R^3)_2$, $Sn(R^3)_2$, C=O, C=S, $C=NR^3$, -C(=O)O-, $-C(=O)NR^3-$, $NR^3$, $P(=O)(R^3)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^3$ substituiert sein kann, oder eine Aryloxy- oder Heteroaryloxygruppe mit 5 bis 40 aromatischen Ringatomen, die durch einen oder mehrere Reste $R^3$ substituiert sein kann, oder eine Kombination dieser Systeme; dabei können zwei oder mehrere, vorzugsweise benachbarte Substituenten $R^2$ auch miteinander ein mono- oder polycyclisches, aliphatisches, heteroaliphatisches, aromatisches oder heteroa-

romatisches Ringsystem bilden;

$R^3$ ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen, worunter auch Cyclopropyl und Cyclobutyl fallen, substituiert sein kann, dabei können zwei oder mehrere, vorzugsweise benachbarte Substituenten $R^3$ auch miteinander ein mono- oder polycyclisches, aliphatisches, heteroaliphatisches, aromatisches oder heteroaromatisches Ringsystem bilden.

Dabei bindet die Indologruppe in Formel (I) an zwei benachbarte Atome X. Weiterhin kann die Indologruppe in zwei Ausrichtungen binden, so dass das N-Atom der Indologruppe und die Gruppe $W^1$ entweder in cis-Position oder in trans-Position bezüglich dem zentralen Phenylring stehen.

Benachbarte Kohlenstoffatome im Sinne der vorliegenden Erfindung sind Kohlenstoffatome, die direkt miteinander verknüpft sind. Weiterhin bedeutet "benachbarte Reste" in der Definition der Reste, dass diese Reste an dasselbe Kohlenstoffatom oder an benachbarte Kohlenstoffatome gebunden sind. Diese Definitionen gelten entsprechend unter anderem für die Begriffe "benachbarte Gruppen" und "benachbarte Substituenten".

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

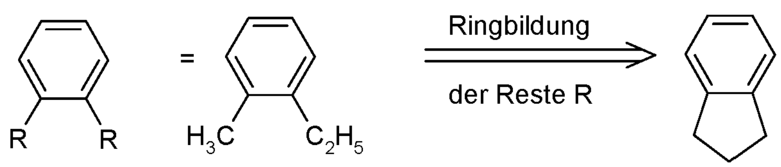


Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

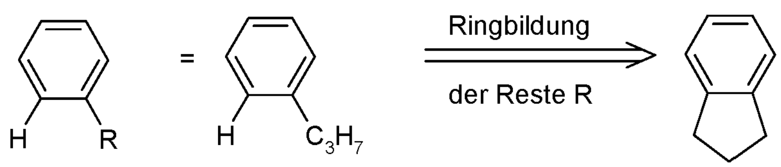


Eine kondensierte Arylgruppe, ein kondensiertes aromatisches Ringsystem oder ein kondensiertes heteroaromatisches Ringsystem im Sinne der vorliegenden Erfindung ist eine Gruppe, in der zwei oder mehr aromatische Gruppen über eine gemeinsame Kante aneinander ankondensiert, d. h. anelliert, sind, so dass beispielsweise zwei C-Atome zu den mindestens zwei aromatischen oder heteroaromatischen Ringen zugehören, wie beispielsweise im Naphthalin. Dagegen ist beispielsweise Fluoren keine kondensierte Arylgruppe im Sinne der vorliegenden Erfindung, da im Fluoren die beiden aromatischen Gruppen keine gemeinsame Kante aufweisen. Entsprechende Definitionen gelten für Heteroarylgruppen sowie für kondensierte Ringsysteme, die auch Heteroatome enthalten können, jedoch nicht müssen.

Eine Arylgruppe im Sinne dieser Erfindung enthält 6 bis 60 C-Atome, vorzugsweise 6 bis 40 C-Atome, besonders bevorzugt 6 bis 30 C-Atome; eine Heteroarylgruppe im Sinne dieser Erfindung enthält 2 bis 60 C-Atome, vorzugsweise 2 bis 40 C-Atome, besonders bevorzugt 2 bis 30 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 60 C-Atome, vorzugsweise 6 bis 40 C-Atome, besonders bevorzugt 6 bis 30 C-Atome im Ringsystem. Ein heteroaromatisches Ringsystem im Sinne dieser Erfindung enthält 1 bis 60 C, vorzugsweise 1 bis 40 C-Atome, besonders bevorzugt 1 bis 30 C-Atome und mindestens

ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nichtaromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl- oder Heteroarylgruppen direkt aneinander gebunden sind, wie z. B. Biphenyl, Terphenyl, Quaterphenyl oder Bipyridin, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer $C_1$- bis $C_{20}$-Alkylgruppe, in der auch einzelne H-Atome oder $CH_2$-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer $C_1$- bis $C_{40}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 bis 60, vorzugsweise 5 - 40 aromatischen Ringatomen, besonders bevorzugt 5 bis 30 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

In einer bevorzugten Ausgestaltung können die erfindungsgemäßen Verbindungen mindestens eine Struktur der Formel (IIa), (IIb), (IIc), (IId), (IIe) oder (IIf) umfassen,

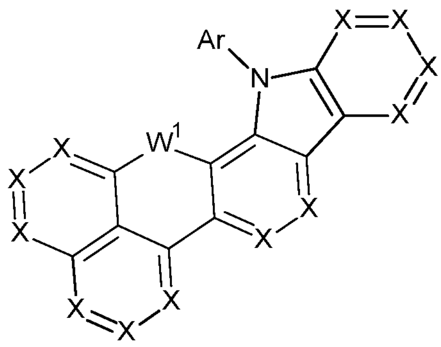

Formel (IIa)

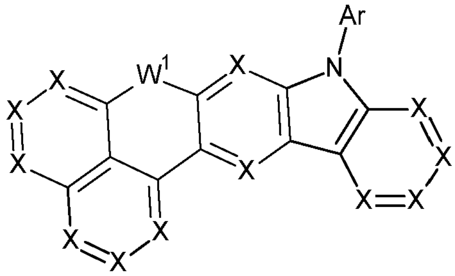

Formel (IIb)

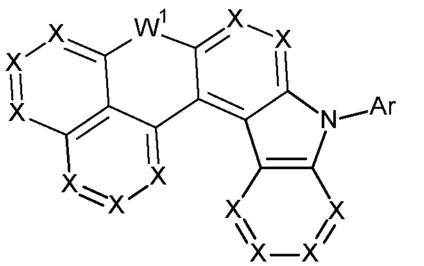

Formel (IIc)

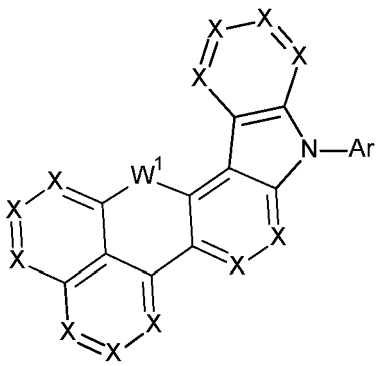

Formel (IId)

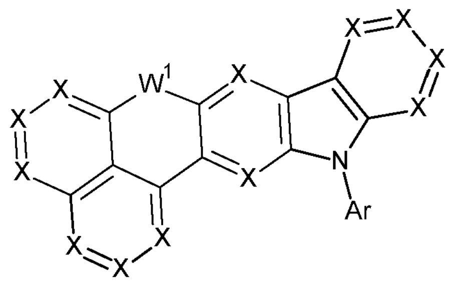

Formel (IIe)

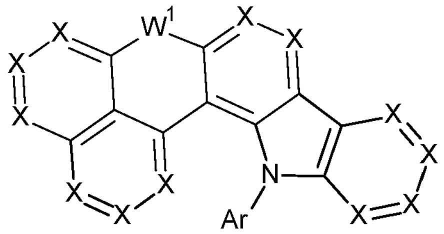

Formel (IIf)

wobei die verwendeten Symbole Ar, $W^1$ und X die zuvor, insbesondere für Formel (I) genannte Bedeutung haben.

Vorzugsweise können die erfindungsgemäßen Verbindungen mindestens eine Struktur der Formel (III) umfassen,

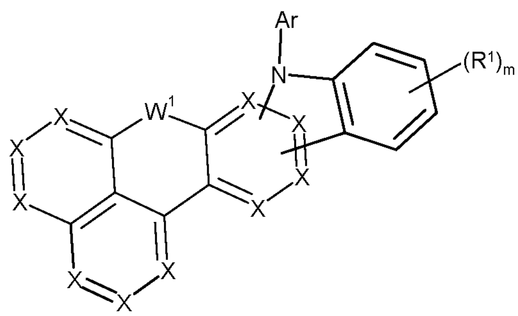

Formel (III)

wobei die Symbole $R^1$, Ar, $W^1$ und X die zuvor, insbesondere für Formel (I) genannte Bedeutung aufweisen und m 0, 1, 2, 3 oder 4, vorzugsweise 0, 1, 2 oder 3 ist.

Vorzugsweise können die erfindungsgemäßen Verbindungen mindestens eine Struktur der Formel (IIIa), (IIIb), (IIIc), (IIId), (IIIe) oder (IIIf) umfassen,

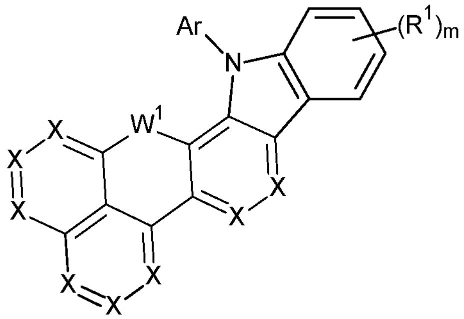

Formel (IIIa)

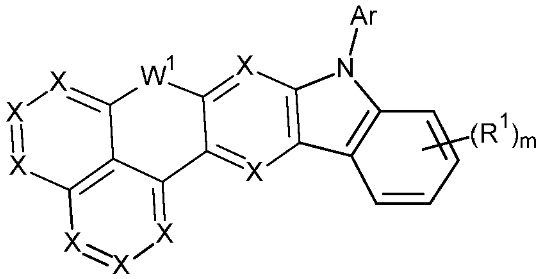

Formel (IIIb)

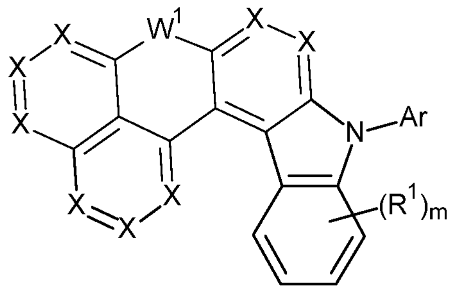

Formel (IIIc)

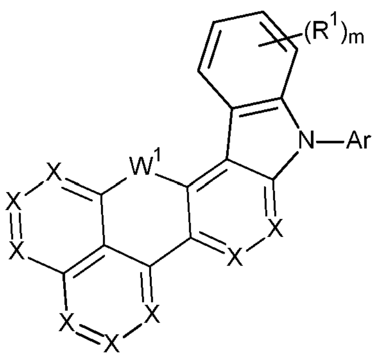

Formel (IIId)

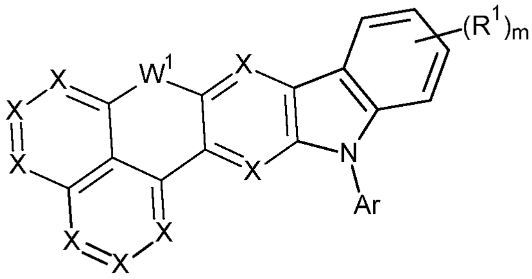

Formel (IIIe)

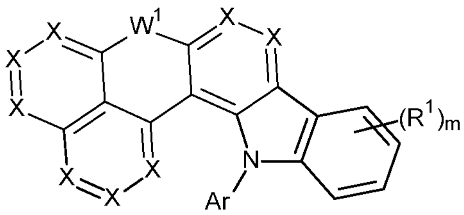

Formel (IIIf)

wobei die Symbole $R^1$, Ar, $W^1$ und X die zuvor, insbesondere für Formel (I) genannte Bedeutung aufweisen und m 0, 1, 2, 3 oder 4, vorzugsweise 0, 1, 2 oder 3 ist.

Gemäß einer weiteren bevorzugten Ausfühurngsform können die erfindungsgemäßen Verbindungen mindestens eine Struktur der Formel (IV) umfassen,

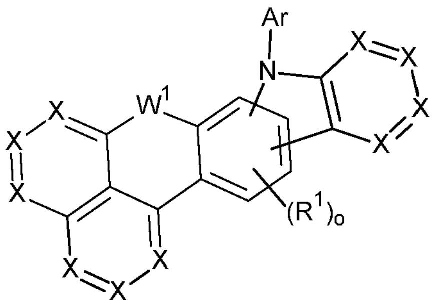

Formel (IV)

wobei die Symbole $R^1$, Ar, $W^1$ und X die zuvor, insbesondere für Formel (I) genannte Bedeutung aufweisen und und o 0, 1 oder 2, vorzugsweise 0 oder 1 ist.

Vorzugsweise können die erfindungsgemäßen Verbindungen mindestens eine Struktur der Formel (IVa), (IVb), (IVc), (IVd), (IVe) oder (IVf) umfassen,

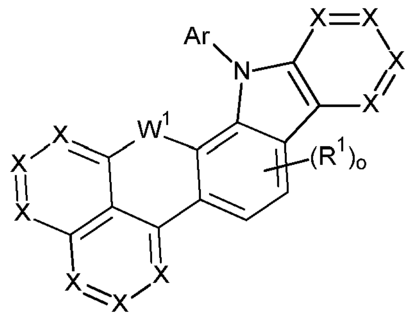

Formel (IVa)

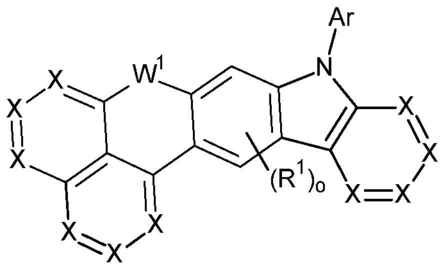

Formel (IVb)

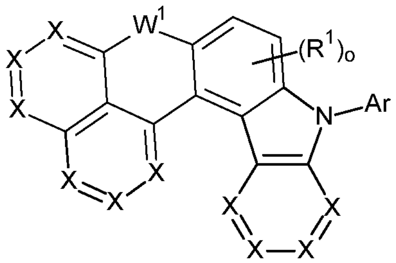

Formel (IVc)

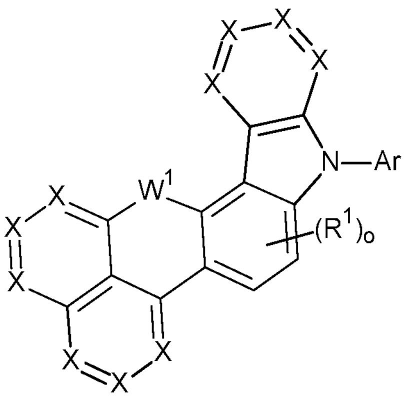

Formel (IVd)

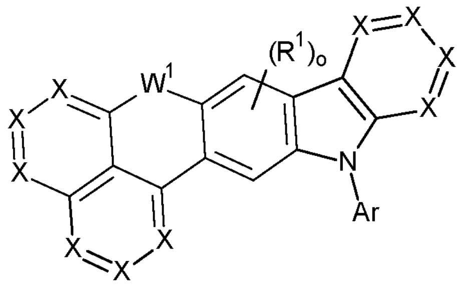

Formel (IVe)

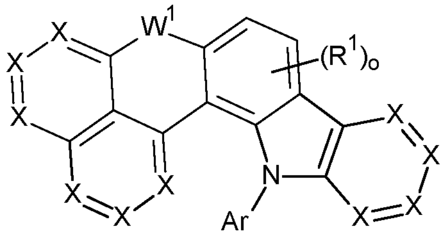

Formel (IVf)

wobei die verwendeten Symbole $R^1$, Ar, $W^1$ und X die zuvor, insbesondere für Formel (I) dargelegte Bedeutung aufweisen und o 0, 1 oder 2, vorzugsweise 0 oder 1 ist.

Gemäß einer weiteren bevorzugten Ausgestaltung können die erfindungsgemäßen Verbindungen mindestens eine Struktur der Formel (V) umfassen,

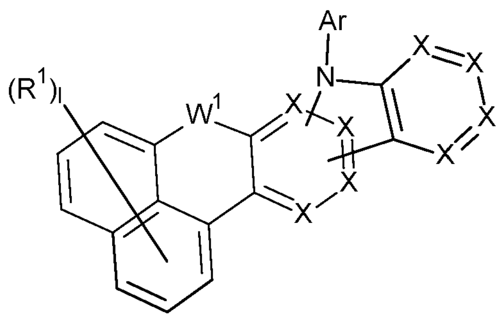

Formel (V)

wobei die Symbole $R^1$, Ar, $W^1$ und X die zuvor, insbesondere für Formel (I) genannte Bedeutung aufweisen und l 0, 1, 2, 3, 4, 5 oder 6, vorzugsweise 0, 1, 2, 3 oder 4, besonders bevorzugt 0, 1 oder 2 ist.

Bevorzugt können die erfindungsgemäßen Verbindungen mindestens eine Struktur der Formel (Va), (Vb), (Vc), (Vd), (Ve) oder (Vf) umfassen,

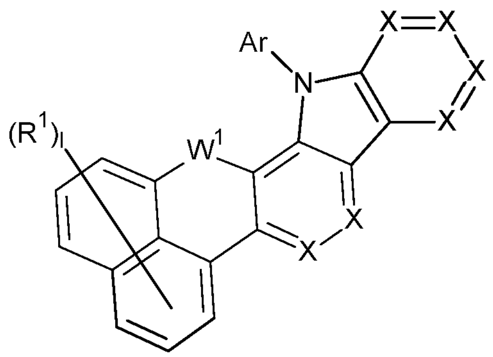

Formel (Va)

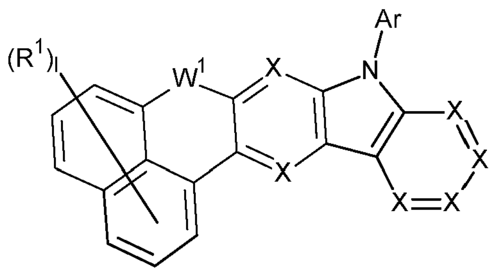

Formel (Vb)

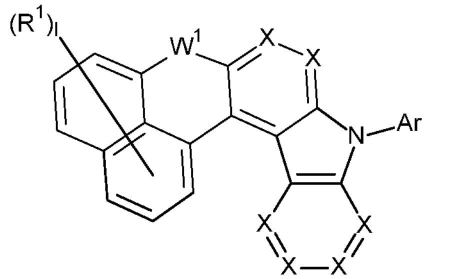

Formel (Vc)

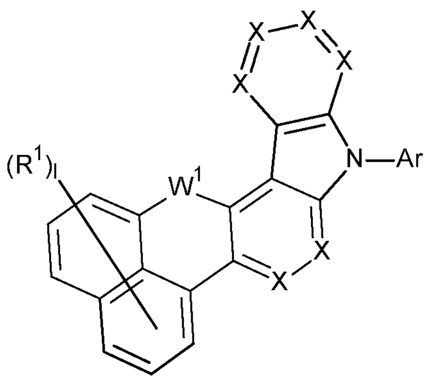

Formel (Vd)

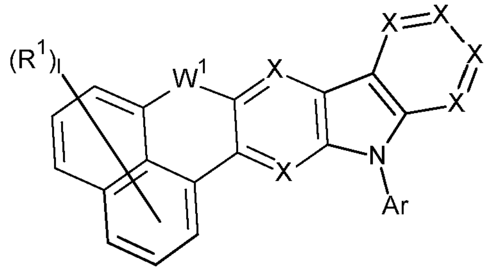

Formel (Ve)

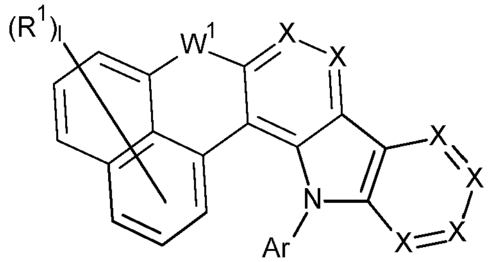

Formel (Vf)

wobei die verwendeten Symbole $R^1$, Ar, $W^1$ und X die zuvor, insbesondere für Formel (I) dargelegte Bedeutung aufweisen und I 0, 1, 2, 3, 4, 5 oder 6, vorzugsweise 0, 1, 2, 3 oder 4, besonders bevorzugt 0, 1 oder 2 ist.

Ferner sind Verbindungen mit Strukturen bevorzugt, in denen höchstens zwei Gruppen X pro Ring für N stehen und bevorzugt mindestens eine, besonders bevorzugt mindestens zwei der Gruppen X pro Ring ausgewählt sind aus C-H und C-D.

Darüber hinaus sind Verbindungen mit Strukturen gemäß Formel (I), (IIa), (IIb), (IIc), (IId), (IIe), (IIf), (III), (IIIa), (IIIb), (IIIc), (IIId), (IIIe), (IIIf), (IV), (IVa), (IVb), (IVc), (IVd), (IVe), (IVf), (V), (Va), (Vb), (Vc), (Vd), (Ve) und/oder (Vf) bevorzugt, in denen nicht mehr als vier Gruppen X, vorzugsweise nicht mehr als zwei Gruppen X und insbesondere nicht mehr als eine Gruppe X für N stehen, und besonders bevorzugt alle Gruppen X, an die nicht die Indologruppe gebunden ist, für $CR^1$ stehen, wobei vorzugsweise höchstens vier, besonders bevorzugt höchstens drei und speziell bevorzugt höchstens zwei der Gruppen $CR^1$, für die X steht, ungleich der Gruppe CH ist.

Vorzugsweise können die erfindungsgemäßen Verbindungen mindestens eine Struktur der Formel (VI) umfassen,

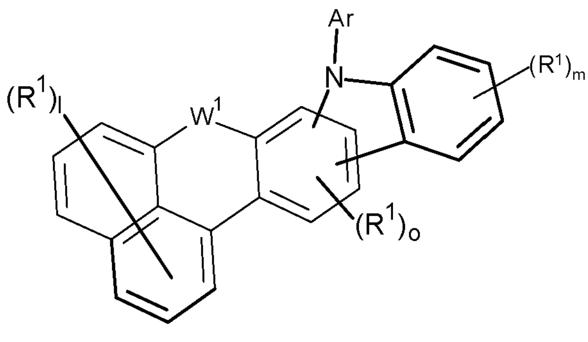

Formel (VI)

wobei die Symbole $R^1$, Ar und $W^1$ die zuvor, insbesondere für Formel (I) genannte Bedeutung aufweisen und I 0, 1, 2, 3, 4, 5 oder 6, vorzugsweise 0, 1, 2, 3 oder 4, besonders bevorzugt 0, 1 oder 2, m 0, 1, 2, 3 oder 4, vorzugsweise 0, 1, 2 oder 3 und o 0, 1 oder 2, vorzugsweise 0 oder 1 ist.

In einer weiterhin bevorzugten Ausführungsform können die erfindungsgemäßen Verbindungen mindestens eine Struktur der Formeln (VIa), (VIb), (VIc), (VId), (VIe) oder (VIf) aufweisen,

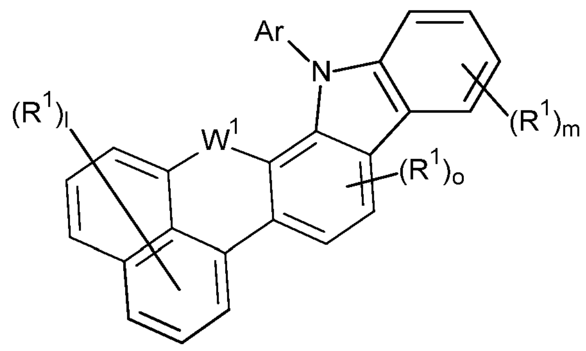

Formel (VIa)

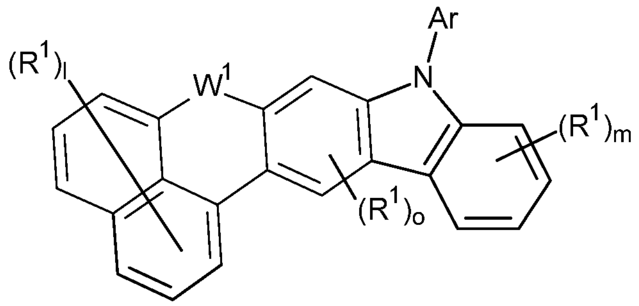

Formel (VIb)

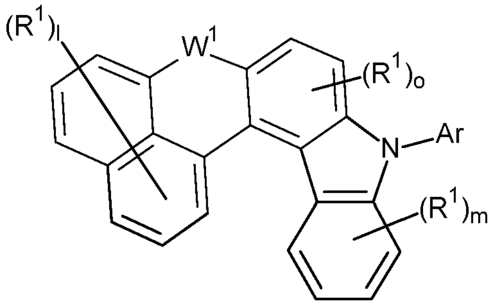

Formel (VIc)

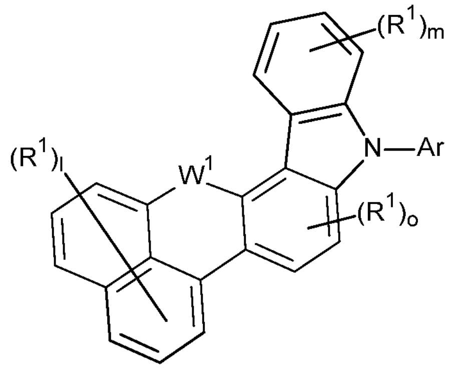

Formel (VId)

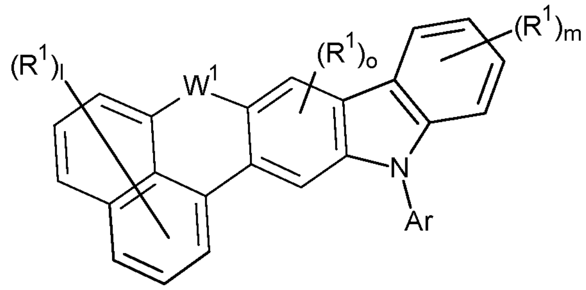

Formel (VIe)

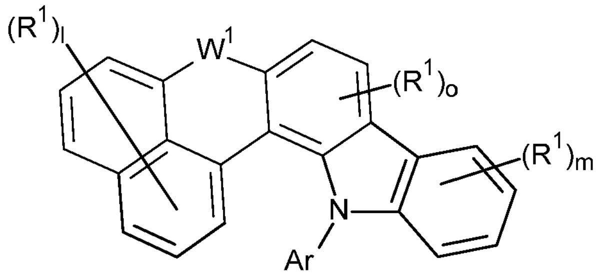

Formel (VIf)

wobei die verwendeten Symbole $R^1$, Ar und $W^1$ die zuvor, insbesondere für Formel (I) genannte Bedeutung aufweisen, l 0, 1, 2, 3, 4, 5 oder 6, vorzugsweise 0, 1, 2, 3 oder 4, besonders bevorzugt 0, 1 oder 2, m 0, 1, 2, 3 oder 4, vorzugsweise 0, 1, 2 oder 3 und o 0, 1 oder 2, vorzugsweise 0 oder 1 ist.

Ferner kann in den Strukturen gemäß den Formeln (VI), (VIa), (VIb), (VIc), (VId), (VIe) und/oder (VIf) vorgesehen sein, dass die Summe der Indices l, m und o höchstens 6, vorzugsweise höchstens 4 und besonders bevorzugt höchstens 2 beträgt.

Ferner kann vorgesehen sein, dass in den Formeln (I), (IIa), (IIb), (IIc), (IId), (IIe), (IIf), (III), (IIIa), (IIIb), (IIIc), (IIId), (IIIe), (IIIf), (IV), (IVa), (IVb), (IVc), (IVd), (IVe), (IVf), (V), (Va), (Vb), (Vc), (Vd), (Ve), (Vf), (VI), (VIa), (VIb), (VIc), (VId), (VIe) und/oder (VIf) genau ein Rest $R^1$ vorhanden ist, der ungleich H oder D ist, so dass die anderen Gruppen X für N, CH oder CD stehen, beziehungsweise die Summe der Indices l, m und o genau eins ist. Vorzugsweise ist dieser Rest $R^1$ an die Carbazolgruppe in diesen Formeln gebunden, wobei der Rest $R^1$ besonders bevorzugt an den Ring des Carbazols gebunden ist, der nicht unmittelbar eine Bindung mit der Gruppe $W^1$ aufweist. Dieser Substituent $R^1$ steht vorzugsweise in para-Position zum Stickstoffatom der Carbazoleinheit.

Vorzugsweise können die erfindungsgemäßen Verbindungen mindestens eine Struktur der Formel (VII) umfassen,

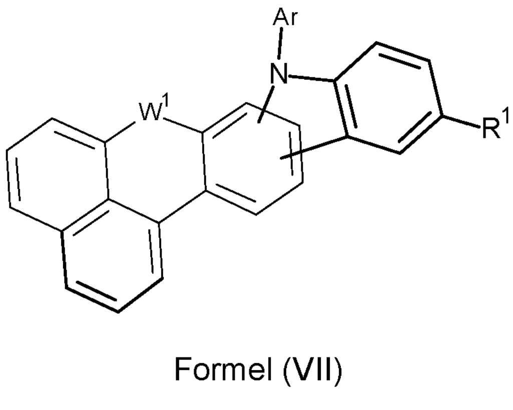

Formel (VII)

wobei die Symbole $R^1$, Ar und $W^1$ die zuvor, insbesondere für Formel (I) genannte Bedeutung aufweisen. Dabei ist $R^1$ bevorzugt ungleich H oder D.

In einer weiterhin bevorzugten Ausführungsform können die erfindungsgemäßen Verbindungen mindestens eine Struktur der Formeln (VIIa), (VIIb), (VIIc), (VIId), (VIIe) und/oder (VIIf) umfassen,

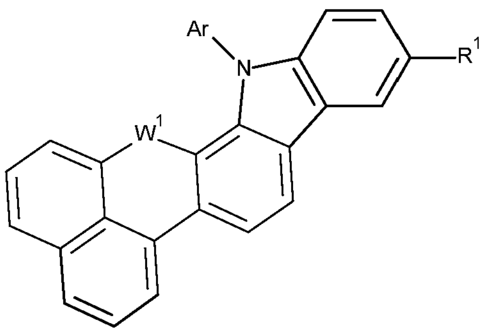

Formel (VIIa)

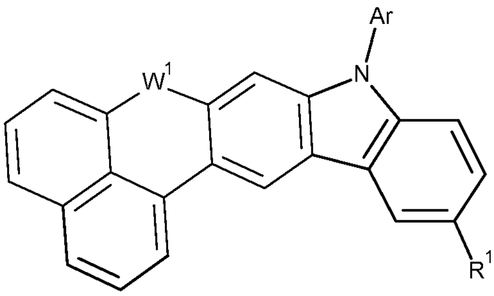

Formel (VIIb)

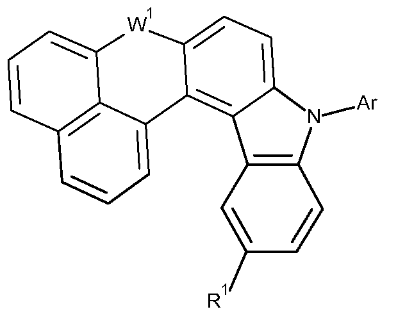

Formel (VIIc)

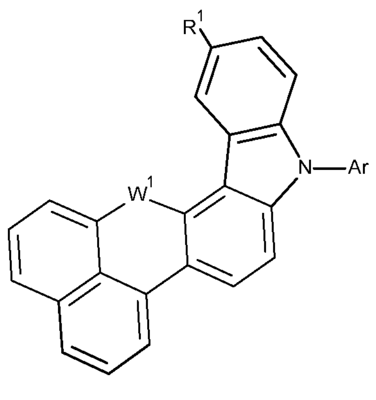

Formel (VIId)

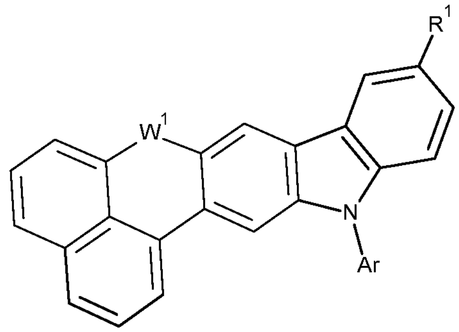

Formel (VIIe)

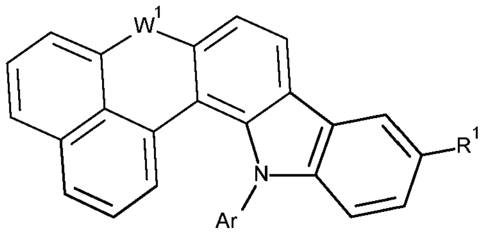

Formel (VIIf)

wobei die verwendeten Symbole Ar, $W^1$ und $R^1$ die zuvor, insbesondere für Formel (I) dargelegte Bedeutung aufweisen. Dabei ist $R^1$ bevorzugt ungleich H oder D.

Vorzugsweise werden die Verbindungen umfassend mindestens eine Struktur gemäß einer der Formeln (I) bis (VII) bzw. den entsprechenden bevorzugten Ausführungsformen durch eine Struktur gemäß einer der Formeln (I) bis (VII) bzw. die bevorzugten Ausführungsformen dargestellt.

Vorzugsweise steht der an die Carbazolgruppe in den Formeln (VII), (VIIa), (VIIb), (VIIc), (VIId), (VIIe) und/oder (VIIf) gebundene Rest $R^1$ für ein aromatisches oder heteroaromatisches Ringsystem mit 5 bis 24 aromatischen Ringatomen, vorzugsweise mit 6 bis 13 aromatischen Ringatomen, das jeweils mit einem oder mehreren Resten $R^2$ substituiert sein kann, bevorzugt aber unsubstituiert ist. Zu den bevorzugten Gruppen, für die der an die Carbazolgruppe in den Formeln (VII), (VIIa), (VIIb), (VIIc), (VIId), (VIIe) und/oder (VIIf) gebundene Rest $R^1$ stehen kann, gehören insbesondere Phenyl, ortho-, meta- oder para-Biphenyl, Terphenyl, insbesondere verzweigtes Terphenyl, Quaterphenyl, insbesondere verzweigtes Quaterphenyl, 1- oder 2-Naphthyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl, 1-, 2-, 3- oder 4-Carbazolyl und Indenocarbazolyl, die jeweils durch einen oder mehrere Reste $R^2$ substituiert sein können, bevorzugt aber unsubstituiert sind. Die Fluoren- bzw. Indenocarbazolylreste sind bevorzugt am Indenokohlenstoffatom durch zwei Reste $R^2$ substituiert. Die Carbazol- bzw. Indenocarbazolreste sind bevorzugt am Stickstoffatom durch einen Rest $R^2$ ungleich H oder D substituiert, bevorzugt durch einen aromatischen oder heteroaromatischen Rest $R^2$, wenn sie nicht über das Stickstoffatom gebunden sind.

Weiterhin kann vorgesehen sein, dass die Substitutenten $R^1$ des heteroaromatischen Ringssystems gemäß den Formeln (I), (IIa), (IIb), (IIc), (IId), (IIe), (IIf), (III), (IIIa), (IIIb), (IIIc), (IIId), (IIIe), (IIIf), (IV), (IVa), (IVb), (IVc), (IVd), (IVe), (IVf), (V), (Va), (Vb), (Vc), (Vd), (Ve), (Vf), (VI), (VIa), (VIb), (VIc), (VId), (VIe), (VIf), (VII), (VIIa), (VIIb), (VIIc), (VIId), (VIIe) und/oder (VIIf) mit den Ringatomen des heteroaromatischen Ringssystems der Grundstruktur kein kondensiertes aromatisches oder heteroaromatisches Ringsystem, vorzugsweise kein kondensiertes Ringsystem bilden. Dies schließt die Bildung eines kondensierten Ringsystems mit möglichen Substituenten $R^2$ bzw. $R^3$ ein, die an die Reste $R^1$ gebunden sein können.

Gemäß einer bevorzugten Ausgestaltung sind erfindungsgemäße Verbindungen durch Strukturen der Formel (I), (IIa), (IIb), (IIc), (IId), (IIe), (IIf), (III), (IIIa), (IIIb), (IIIc), (IIId), (IIIe), (IIIf), (IV), (IVa), (IVb), (IVc), (IVd), (IVe), (IVf), (V), (Va), (Vb), (Vc), (Vd), (Ve), (Vf), (VI), (VIa), (VIb), (VIc), (VId), (VIe), (VIf), (VII), (VIIa), (VIIb), (VIIc), (VIId), (VIIe) und/oder (VIIf) darstellbar. Vorzugsweise weisen Verbindungen, umfassend Strukturen gemäß Formel (I), (IIa), (IIb), (IIc), (IId), (IIe), (IIf), (III), (IIIa), (IIIb), (IIIc), (IIId), (IIIe), (IIIf), (IV), (IVa), (IVb), (IVc), (IVd), (IVe), (IVf), (V), (Va), (Vb), (Vc), (Vd), (Ve), (Vf), (VI), (VIa), (VIb), (VIc), (VId), (VIe), (VIf), (VII), (VIIa), (VIIb), (VIIc), (VIId), (VIIe) und/oder (VIIf), ein Molekulargewicht von kleiner oder gleich 5000 g/mol, bevorzugt kleiner oder gleich 4000 g/mol, insbesondere bevorzugt kleiner oder gleich 3000 g/mol, speziell bevorzugt kleiner oder gleich 2000 g/mol und ganz besonders bevorzugt kleiner oder

gleich 1200 g/mol auf.

Weiterhin zeichnen sich bevorzugte erfindungsgemäße Verbindungen dadurch aus, dass diese sublimierbar sind. Diese Verbindungen weisen im Allgemeinen eine Molmasse von weniger als ca. 1200 g/mol auf.

Vorzugsweise ist die aromatische oder heteroaromatische Gruppe des durch das Symbol Ar beziehungsweise $Ar^1$ dargestellten aromatischen oder heteroaromatischen Ringsystems direkt, d.h. über ein Atom der aromatischen oder heteroaromatischen Gruppe, an das jeweilige Atom der weiteren Gruppe gebunden.

Gemäß einer Ausführungsform kann die Gruppe Ar eine Lochtransportgruppe umfassen oder darstellen. Lochtransportgruppen sind in der Fachwelt bekannt, wobei diese vorzugsweise Triarylamin- oder Carbazolgruppen umfassen.

Vorzugsweise kann vorgesehen sein, dass die Lochtransportgruppe eine Gruppe umfasst und bevorzugt für eine Gruppe steht, die ausgewählt ist aus den Formeln (H-1) bis (H-3),

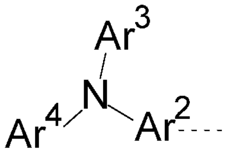

Formel (H-1)

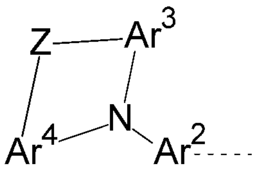

Formel (H-2)

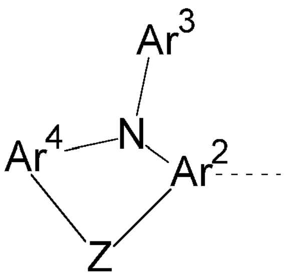

Formel (H-3)

wobei die gestrichelte Bindung die Anbindungsposition an das Stickstoffatom markiert und weiterhin gilt:

$Ar^2$, $Ar^3$, $Ar^4$ ist jeweils unabhängig eine Arylgruppe mit 6 bis 40 C-Atomen oder eine Heteroarylgruppe mit 3 bis 40 C-Atomen, welche jeweils durch einen oder mehrere Reste $R^1$ substituiert sein kann; und

Z steht für $C(R^1)_2$, $Si(R^1)_2$, C=O, N-$Ar^1$, $BR^1$, $PR^1$, $PO(R^1)$, SO, $SO_2$, Se, O oder S, vorzugsweise für $C(R^1)_2$, N-$Ar^1$, O oder S, wobei die Symbole $Ar^1$ und $R^1$ die zuvor, insbesondere für Formel (I) genannte Bedeutung aufweisen.

Demgemäß kann die Gruppe Ar einen Rest der Formeln (H-1), (H-2) und/oder (H3) umfassen und bevorzugt für einen Rest der Formeln (H-1), (H-2) oder (H3) stehen.

Weiterhin kann vorgesehen sein, dass die Gruppe Ar eine Gruppe umfasst und bevorzugt für eine Gruppe steht, die ausgewählt ist aus den Formeln (H-4) bis (H-26),

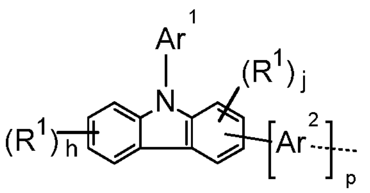

Formel (H-4)

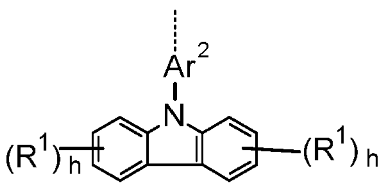

Formel (H-5)

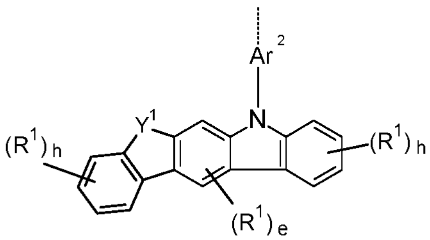

Formel (H-6)

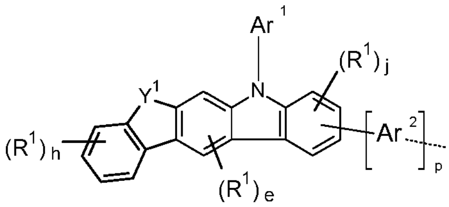

Formel (H-7)

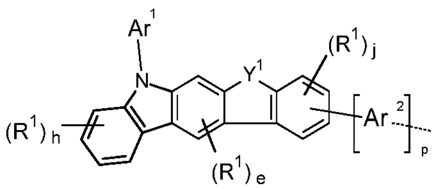

Formel (H-8)

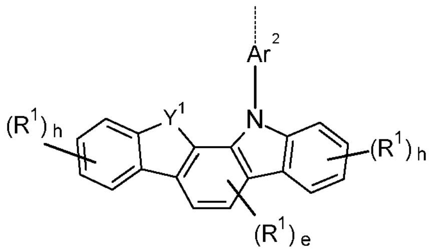

Formel (H-9)

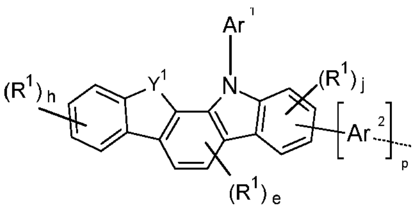

Formel (H-10)

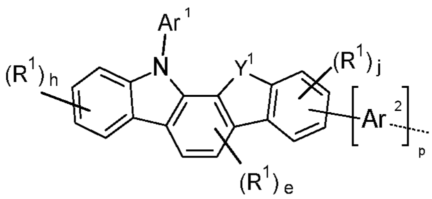

Formel (H-11)

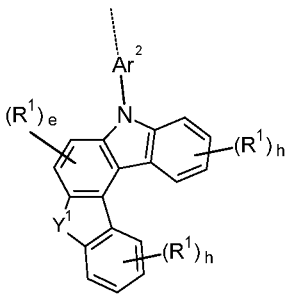

Formel (H-12)

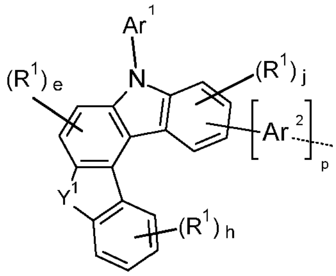

Formel (H-13)

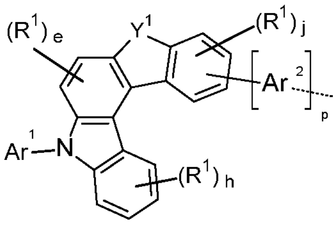

Formel (H-14)

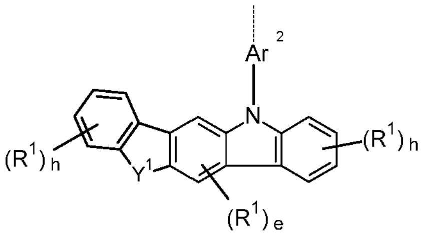

Formel (H-15)

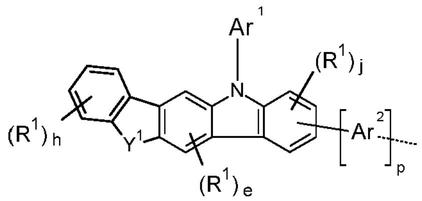

Formel (H-16)

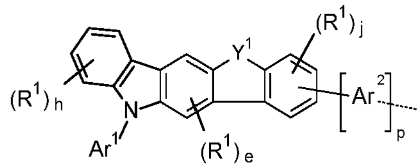

Formel (H-17)

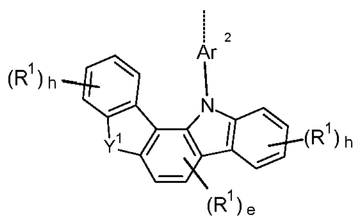

Formel (H-18)

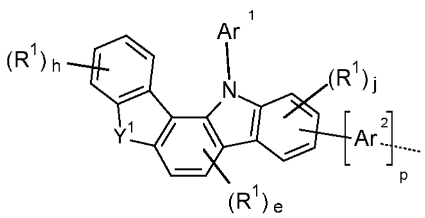

Formel (H-19)

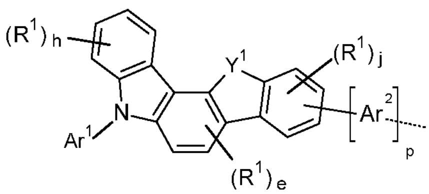

Formel (H-20)

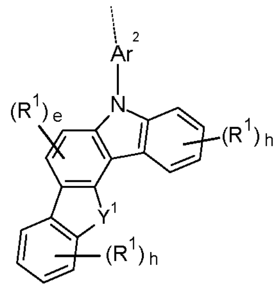

Formel (H-21)

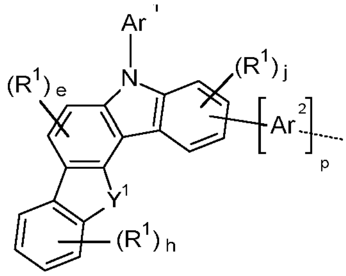

Formel (H-22)

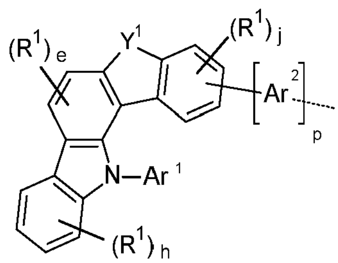

Formel (H-23)

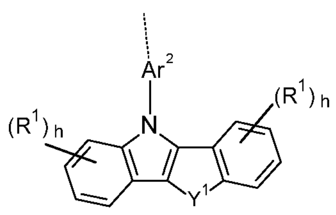

Formel (H-24)

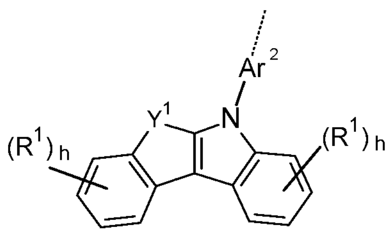

Formel (H-25)

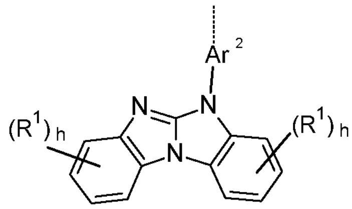

Formel (H-26)

wobei $Y^1$ O, S, $C(R^1)_2$ oder $NAr^1$ darstellt, die gestrichelte Bindung die Anbindungsposition an das Stickstoffatom markiert, e 0, 1 oder 2 ist, j 0, 1, 2 oder 3 ist, h gleich oder verschieden bei jedem Auftreten 0, 1, 2, 3 oder 4 ist, p 0 oder 1 ist, $Ar^1$ und $R^1$ die zuvor, insbesondere für Formel (I) und $Ar^2$ die zuvor, insbesondere für Formel (H-1) oder (H-2) genannte Bedeutung aufweist.

Die zuvor dargelegten Lochtransportgruppen der Formeln (H-1) bis (H-26) stellen auch bevorzugte Reste $R^1$ in den Verbindungen gemäß Formel (I) oder bevorzugte Ausführungsformen dieser Formel dar, wobei in diesem Fall die in den Formeln (H-1) bis (H-26) dargelegten Gruppen $R^1$ durch Reste $R^2$ zu ersetzen sind.

Aus der obigen Formulierung ist ersichtlich, dass, falls der Index p = 0 ist, die entsprechende Gruppe $Ar^2$ nicht vorhanden ist und eine Bindung gebildet wird.

Bevorzugt kann die Gruppe $Ar^2$ mit dem aromatischen oder heteroaromatischen Rest oder dem Stickstoffatom, an den die Gruppe $Ar^2$ gemäß den Formeln (H-1) bis (H-26) gebunden sein kann, eine durchgängige Konjugation ausbilden.

In einer weiteren bevorzugten Ausführungsform der Erfindung steht $Ar^2$ für ein aromatisches oder heteroaromatisches Ringsystem mit 5 bis 14 aromatischen oder heteroaromatischen Ringatomen, vorzugsweise ein aromatisches Ringsystem mit 6 bis 12 Kohlenstoffatomen, welches durch einen oder mehrere Reste $R^1$ substituiert sein kann, bevorzugt aber unsubstituiert ist, wobei $R^1$ die zuvor, insbesondere für Formel (I) genannte Bedeutung aufweisen kann. Besonders bevorzugt steht $Ar^2$ für ein aromatisches Ringsystem mit 6 bis 10 aromatischen Ringatomen oder ein heteroaromatisches Ringsystem mit 6 bis 13 heteroaromatischen Ringatomen, das jeweils durch einen oder mehrere Reste $R^1$ substituiert sein kann, bevorzugt aber unsubstituiert ist, wobei $R^1$ die zuvor, insbesondere für Formel (I) genannte Bedeutung aufweisen kann.

Weiterhin bevorzugt steht das unter anderem in Formeln (H-1) bis (H-26) dargelegte Symbol $Ar^2$ für einen Arylen- oder Heteroarylenrest mit 5 bis 14 Ringatomen, vorzugsweise 6 bis 13 Ringatomen, besonders bevorzugt 6 bis 10 Ringatomen, so dass eine aromatische oder heteroaromatische Gruppe eines aromatischen oder heteroaromatische Ringsystems direkt, d.h. über ein Atom der aromatischen oder heteroaromatische Gruppe, an das jeweilige Atom der weiteren Gruppe gebunden ist.

Weiterhin kann vorgesehen sein, dass die in Formeln (H-1) bis (H-26) dargelegte Gruppe $Ar^2$ ein aromatisches Ringsystem mit höchstens zwei kondensierten aromatischen und/oder heteroaromatischen Sechsringen, vorzugsweise kein kondensiertes aromatisches oder heteroaromatisches Ringsystem mit kondensierten Sechsringen umfasst. Demgemäß sind Naphthylstrukturen gegenüber Anthracenstrukturen bevorzugt. Weiterhin sind Fluorenyl-, Spirobifluorenyl-, Dibenzofuranyl- und/oder Dibenzothienyl-Strukturen gegenüber Naphthylstrukturen bevorzugt. Besonders bevorzugt sind Strukturen, die keine Kondensation aufweisen, wie beispielsweise Phenyl-, Biphenyl-, Terphenyl- und/oder Quaterphenyl-Strukturen.

Ferner kann vorgesehen sein, dass die unter anderem in Formeln (H-1) bis (H-26) dargelegte Gruppe $Ar^2$ höchstens 1 Stickstoffatom, bevorzugt höchstens 2 Heteroatome, insbesondere bevorzugt höchstens ein Heteroatom und ganz besonders bevorzugt kein Heteroatom aufweist.

In einer weiteren bevorzugten Ausführungsform der Erfindung steht $Ar^3$ und/oder $Ar^4$ gleich oder verschieden bei jedem Auftreten für ein aromatisches oder heteroaromatisches Ringsystem mit 6 bis 24 aromatischen Ringatomen, bevorzugt mit 6 bis 18 aromatischen Ringatomen, besonders bevorzugt für ein aromatisches Ringsystem mit 6 bis 12 aromatischen Ringatomen bzw. ein heteroaromatisches Ringsystem mit 6 bis 13 aromatischen Ringatomen, das jeweils durch einen oder mehrere Reste $R^1$ substituiert sein kann, bevorzugt aber unsubstituiert ist, wobei $R^1$ die zuvor, insbesondere in Formel (I) dargestellte Bedeutung aufweisen kann.

In einer weiteren bevorzugten Ausführungsform kann die Gruppe Ar eine Elektronentransportgruppe umfassen, vorzugsweise darstellen. Elektronentransportgruppen sind in der Fachwelt weithin bekannt und fördern die Fähigkeit von Verbindungen, Elektronen zu transportieren und/oder zu leiten. Hierfür eignen sich insbesondere elektronenarme Heteroarylgruppen. Diese sind dadurch definiert, dass sie einen heteroaromatischen Sechsring enthalten, der mindestens ein Stickstoffatom aufweist, und/oder dass sie einen heteroaromatischen Fünfring enthalten, der mindestens zwei Heteroatome aufweist, von denen mindestens ein Heteroatom Stickstoff ist. An diese Strukturen können auch weitere Aryl- oder Heteroarylgruppen ankondensiert sein, wie beispielsweise im Chinazolin oder Benzimidazol.

In diesem Zusammenhang ist festzuhalten, dass erfindungsgemäße Verbindungen mit Elektronentransportgruppen als Matrixmaterialien und/oder Elektronentransportmaterialen besonders bevorzugt sind, insbesondere auch gegenüber erfindungsgemäßen Verbindungen, die zwar eine Lochtransportgruppe, jedoch keine Elektronentransportgruppe aufweisen.

Weiterhin zeigen Verbindungen, umfassend mindestens eine Struktur gemäß Formel (I) oder deren bevorzugte Ausführungsformen überraschende Vorteile, bei denen die Gruppe Ar mindestens eine Struktur umfasst, die aus der Gruppe Pyridine, Pyrimidine, Pyrazine, Pyridazine, Triazine, Chinazoline, Chinoxaline, Chinoline, Isochinoline, Imidazole und/oder Benzimidazole ausgewählt ist, wobei Pyrimidine, Triazine und Chinazoline besonders bevorzugt sind.

In einer bevorzugten Ausgestaltung der vorliegenden Erfindung kann vorgesehen sein, dass die Gruppe Ar für eine Gruppe steht, die durch die Formel (QL) darstellbar ist,

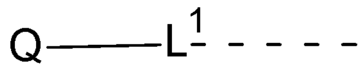

Formel (QL)

worin L' eine Bindung oder ein aromatisches oder heteroaromatisches Ringsystem mit 5 bis 40, bevorzugt 5 bis 30 aromatischen Ringatomen darstellt, welches durch einen oder mehrere Reste $R^1$ substituiert sein kann, und Q eine Elektronentransportgruppe ist, wobei $R^1$ die zuvor, insbesondere für Formel (I) genannte Bedeutung aufweist. Dabei ist die Elektronentransportgruppe wie oben definiert.

Bevorzugt kann die Gruppe $L^1$ mit der Gruppe Q und dem Stickstoffatom, an den die Gruppe L' gemäß Formel (QL) gebunden ist, eine durchgängige Konjugation ausbilden. Eine durchgängige Konjugation der aromatischen beziehungsweise heteroaromatischen Systeme wird ausgebildet, sobald direkte Bindungen zwischen benachbarten aromatischen oder heteroaromatischen Ringen gebildet werden. Eine weitere Verknüpfung zwischen den zuvor genannten konjugierten Gruppen, die beispielsweise über ein S-, N- oder O-Atom oder eine Carbonylgruppe erfolgt, schadet einer Konjugation nicht. Bei einem Fluorensystem sind die beiden aromatischen Ringe unmittelbar gebunden, wobei das $sp^3$-hybridi-sierte Kohlenstoffatom in 9-Position zwar eine Kondensation dieser Ringe unterbindet, jedoch eine Konjugation erfolgen kann, da dieses $sp^3$-hybridi-sierte Kohlenstoffatom in 9-Position nicht zwingend zwischen der elektronentransportierenden Gruppe Q und dem Stickstoffatom liegt. Im Gegensatz hierzu kann bei einer zweiten Spirobifluorenstruktur eine durchgängige Konjugation ausgebildet werden, falls die Verbindung zwischen der Gruppe Q und dem aromatischen oder heteroaromatischen Rest, an den die Gruppe L' gemäß Formel (QL) gebunden ist, über die gleiche Phenylgruppe der Spirobifluorenstruktur oder über Phenylgruppen der Spirobifluorenstruktur, die unmittelbar aneinander gebunden sind und in einer Ebene liegen, erfolgt. Falls die Verbindung zwischen der Gruppe Q und dem aromatischen oder heteroaromatischen Rest, an den die Gruppe L' gemäß Formel (QL) gebunden ist, über verschiedene Phenylgruppen der zweiten Spirobifluorenstruktur erfolgt, die über das $sp^3$-hybridisierte Kohlenstoffatom in 9-Position verbunden sind, ist die Konjugation unterbrochen.

In einer bevorzugten Ausführungsform der Erfindung steht L' für eine Bindung oder für ein aromatisches oder heteroaromatisches Ringsystem mit 5 bis 14 aromatischen oder heteroaromatischen Ringatomen, vorzugsweise ein aromatisches Ringsystem mit 6 bis 12 Kohlenstoffatomen, welches durch einen oder mehrere Reste $R^1$ substituiert sein kann, bevorzugt aber unsubstituiert ist, wobei $R^1$ die zuvor, insbesondere für Formel (I) genannte Bedeutung aufweisen kann. Besonders bevorzugt steht L' für ein aromatisches Ringsystem mit 6 bis 10 aromatischen Ringatomen oder ein heteroaromatisches Ringsystem mit 6 bis 13 heteroaromatischen Ringatomen, das jeweils durch einen oder mehrere Reste $R^1$ substituiert sein kann, bevorzugt aber unsubstituiert ist, wobei $R^1$ die zuvor, insbesondere für Formel (I) genannte Bedeutung aufweisen kann.

Weiterhin bevorzugt steht das unter anderem in Formel (QL) dargelegte Symbol L' gleich oder verschieden bei jedem Auftreten für eine Bindung oder einen Arylen- oder Heteroarylenrest mit 5 bis 24 Ringatomen, vorzugsweise 6 bis 13 Ringatomen, besonders bevorzugt 6 bis 10 Ringatomen, so dass eine aromatische oder heteroaromatische Gruppe eines aromatischen oder heteroaromatische Ringsystems direkt, d.h. über ein Atom der aromatischen oder heteroaromatische Gruppe, an das jeweilige Atom der weiteren Gruppe gebunden ist.

Weiterhin kann vorgesehen sein, dass die in Formel (QL) dargelegte Gruppe $L^1$ ein aromatisches Ringsystem mit höchstens zwei kondensierten aromatischen und/oder heteroaromatischen Sechsringen, vorzugsweise kein kondensiertes aromatisches oder heteroaromatisches Ringsystem umfasst. Demgemäß sind Naphthylstrukturen gegenüber Anthracenstrukturen bevorzugt. Weiterhin sind Fluorenyl-, Spirobifluorenyl-, Dibenzofuranyl- und/oder Dibenzothienyl-Strukturen gegenüber Naphthylstrukturen bevorzugt.

Besonders bevorzugt sind Strukturen, die keine Kondensation aufweisen, wie beispielsweise Phenyl-, Biphenyl-, Terphenyl- und/oder Quaterphenyl-Strukturen.

Beispiele für geeignete aromatische oder heteroaromatische Ringsysteme L’ sind ausgewählt aus der Gruppe bestehend aus ortho-, meta- oder para-Phenylen, ortho-, meta- oder para-Biphenylen, Terphenylen, insbesondere verzweigtes Terphenylen, Quaterphenylen, insbesondere verzweigtes Quaterphenylen, Fluorenylen, Spirobifluorenylen, Dibenzofuranylen, Dibenzothienylen und Carbazolylen, die jeweils durch einen oder mehrere Reste $R^1$ substituiert sein können, bevorzugt aber unsubstituiert sind. Wenn L’ für Carbazolylen steht, ist dieses bevorzugt am Stickstoff durch einen Rest $R^1$ ungleich H oder D substituiert, bevorzugt durch einen aromatischen oder heteroaromatischen Rest $R^1$, wenn das Carbazolylen nicht über das Stickstoffatom verknüpft ist.

Ferner kann vorgesehen sein, dass die unter anderem in Formel (QL) dargelegte Gruppe L’ höchstens 1 Stickstoffatom, bevorzugt höchstens 2 Heteroatome, insbesondere bevorzugt höchstens ein Heteroatom und besonders bevorzugt kein Heteroatom aufweist.

Vorzugsweise kann die unter anderem in den Formel (QL) dargelegte Gruppe Q beziehungsweise die Elektronentransportgruppe ausgewählt sein aus Strukturen der Formeln (Q-1), (Q-2), (Q-3), (Q-4), (Q-5), (Q-6), (Q-7), (Q-8), (Q-9) und/oder (Q-10),

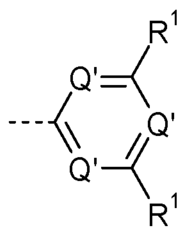
Formel (Q-1)

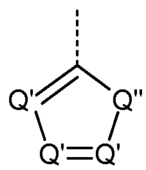
Formel (Q-2)

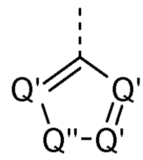
Formel (Q-3)

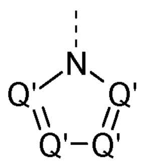
Formel (Q-4)

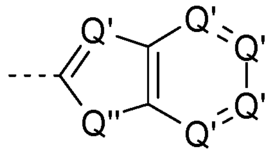
Formel (Q-5)

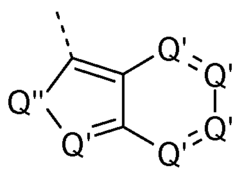
Formel (Q-6)

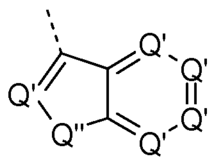
Formel (Q-7)

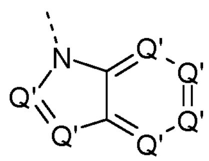
Formel (Q-8)

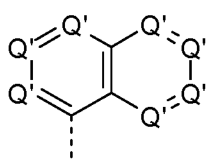
Formel (Q-9)

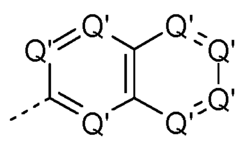
Formel (Q-10),

wobei die gestrichelte Bindung die Anbindungsposition markiert,
Q’ bei jedem Auftreten gleich oder verschieden $CR^1$ oder N darstellt, und
Q" $NR^1$, O oder S darstellt;
wobei wenigstens ein Q’ gleich N ist, und
$R^1$ wie zuvor, insbesondere in Formel (I) definiert ist.

Weiterhin kann die unter anderem in der Formel (QL) dargelegte Gruppe Q, beziehungsweise die Elektronentransportgruppe vorzugsweise ausgewählt sein aus einer Struktur der Formeln (Q-11), (Q-12), (Q-13), (Q-14) und/oder (Q-15),

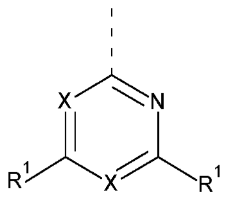

Formel (Q-11)

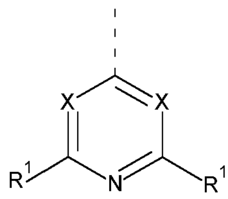

Formel (Q-12)

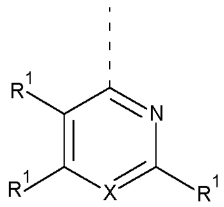

Formel (Q-13)

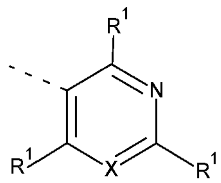

Formel (Q-14)

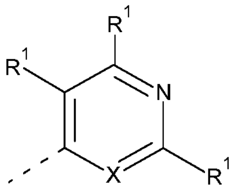

Formel (Q-15)

wobei das Symbol $R^1$ die zuvor unter anderem für Formel (I) genannte Bedeutung aufweist, X N oder $CR^1$ ist und die gestrichelte Bindung die Anbindungsposition markiert, wobei X vorzugsweise ein Stickstoffatom darstellt.

In einer weiteren Ausführungsform kann die unter anderem in der Formel (QL) dargelegte Gruppe Q, beziehungsweise die Elektronentransportgruppe ausgewählt sein aus Strukturen der Formeln (Q-16), (Q-17), (Q-18), (Q-19), (Q-20), (Q-21) und/oder (Q-22),

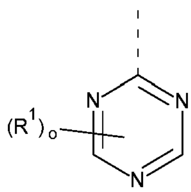

Formel (Q-16)

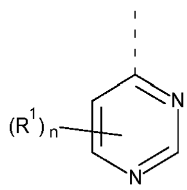

Formel (Q-17)

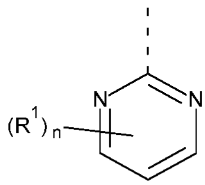

Formel (Q-18)

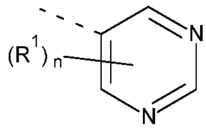

Formel (Q-19)

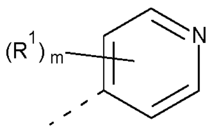

Formel (Q-20)

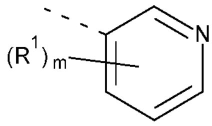

Formel (Q-21)

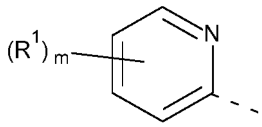

Formel (Q-22)

worin das Symbol $R^1$ die zuvor unter anderem für Formel (I) dargelegte Bedeutung aufweist, die gestrichelte Bindung die Anbindungsposition markiert und m 0, 1, 2, 3 oder 4, vorzugsweise 0, 1 oder 2, n 0, 1, 2 oder 3, vorzugsweise 0, 1 oder 2 und o 0, 1 oder 2, vorzugsweise 1 oder 2 ist. Hierbei sind die Strukturen der Formeln (Q-16), (Q-17), (Q-18) und (Q-19) bevorzugt.

In einer weiteren Ausführungsform kann die unter anderem in der Formel (QL) dargelegte Gruppe Q, beziehungsweise die Elektronentransportgruppe ausgewählt sein aus Strukturen der Formeln (Q-23), (Q-24) und/oder (Q-25),

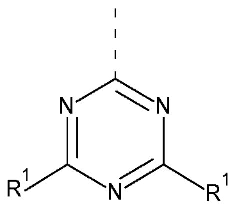

Formel (Q-23)

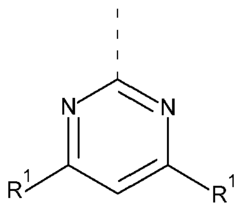

Formel (Q-24)

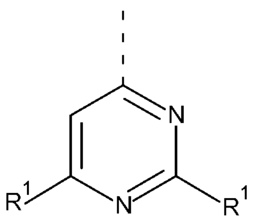

Formel (Q-25)

worin das Symbol $R^1$ die zuvor unter anderem für Formel (I) dargelegte Bedeutung aufweist und die gestrichelte Bindung die Anbindungsposition markiert.

In einer weiteren Ausführungsform kann die unter anderem in der Formel (QL) dargelegte Gruppe Q, beziehungsweise die Elektronentransportgruppe ausgewählt sein aus Strukturen der Formeln (Q-26), (Q-27), (Q-28), (Q-29)

und/oder (Q-30),

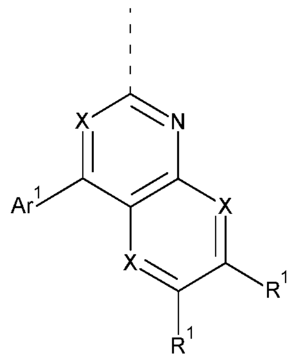

Formel (Q-26)

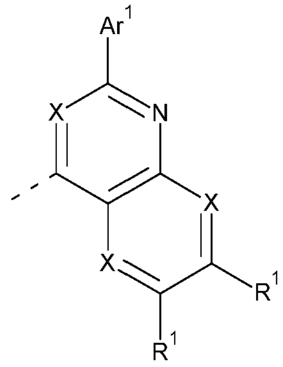

Formel (Q-27)

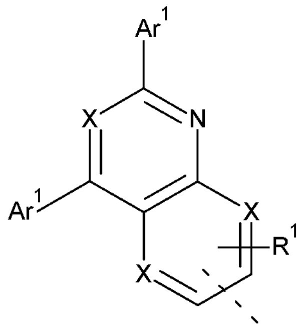

Formel (Q-28)

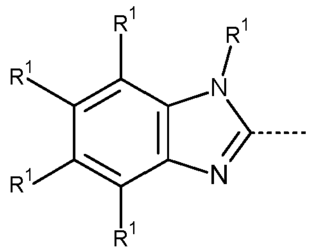

Formel (Q-29)

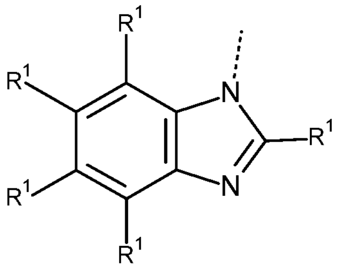

Formel (Q-30)

wobei Symbole X, $Ar^1$ und $R^1$ die zuvor unter anderem für Formel (I) genannte Bedeutung aufweisen und die gestrichelte Bindung die Anbindungsposition markiert. Vorzugsweise stellt in den Strukturen der Formeln (Q-26), (Q-27) und (Q-28) genau ein X ein Stickstoffatom dar.

Vorzugsweise kann die unter anderem in der Formel (QL) dargelegte Gruppe Q, beziehungsweise die Elektronentransportgruppe ausgewählt sein aus Strukturen der Formeln (Q-31), (Q-32), (Q-33), (Q-34), (Q-35), (Q-36), (Q-37), (Q-38), (Q-39), (Q-40), (Q-41), (Q-42), (Q-43) und/oder (Q-44),

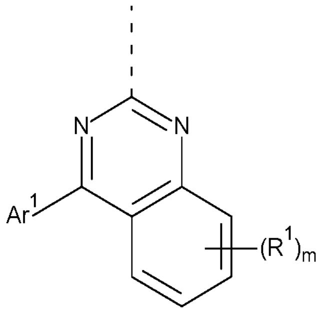

Formel (Q-31)

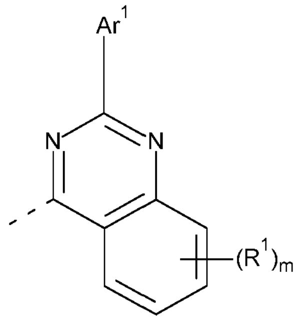

Formel (Q-32)

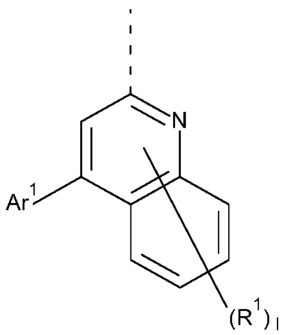

Formel (Q-33)

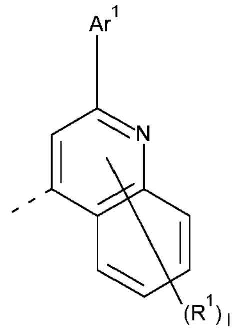

Formel (Q-34)

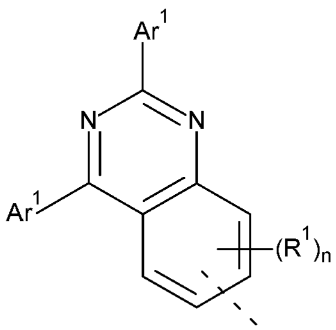

Formel (Q-35)

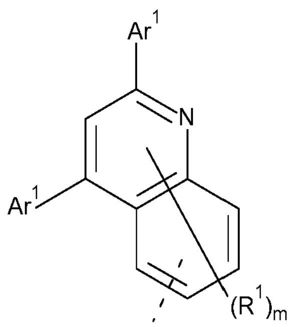

Formel (Q-36)

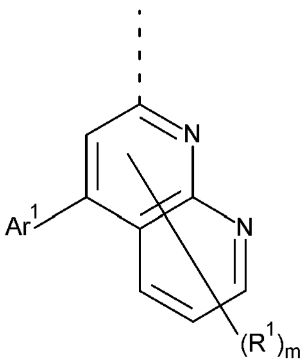

Formel (Q-37)

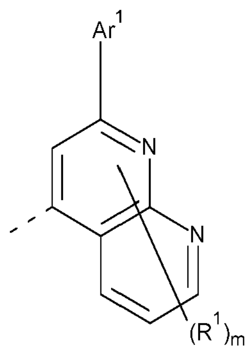

Formel (Q-38)

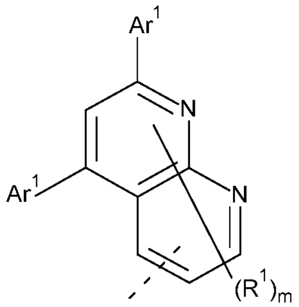

Formel (Q-39)

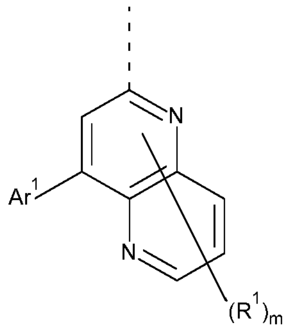

Formel (Q-40)

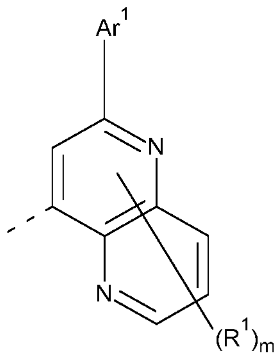

Formel (Q-41)

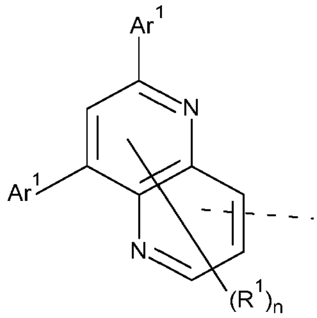

Formel (Q-42)

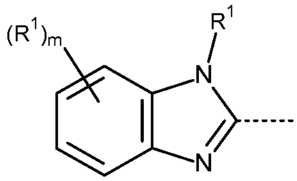

Formel (Q-43)

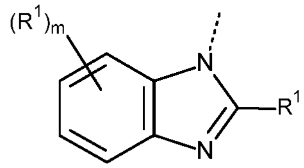

Formel (Q-44)

worin die Symbole $Ar^1$ und $R^1$ die zuvor unter anderem für Formel (I) dargelegte Bedeutung aufweisen, die gestrichelte Bindung die Anbindungsposition markiert und m 0, 1, 2, 3 oder 4, vorzugsweise 0, 1 oder 2, n 0, 1, 2 oder 3, vorzugsweise 0 oder 1, n 0, 1, 2 oder 3, vorzugsweise 0, 1 oder 2 und I 1, 2, 3, 4 oder 5, vorzugsweise 0, 1 oder 2 ist.

Die zuvor dargelegten Elektronentransportgruppen der Formeln (Q-1) bis (Q-44) stellen auch bevorzugte Reste $R^1$ in den Verbindungen gemäß Formel (I) oder bevorzugte Ausführungsformen dieser Formel dar, wobei in diesem Fall die in den Formeln (Q-1) bis (Q-44) dargelegten Gruppen $R^1$ durch Reste $R^2$ zu ersetzen sind.

Ferner kann vorgesehen sein, dass die Gruppe Ar eine Lochtransport- und eine Elektronentransportgruppe umfasst. Je nach Ausgestaltung können bevorzugte Gruppen aus den zuvor dargelegten Formeln (H-1) bis (H-26) beziehungsweise (Q-1) bis (Q-44) gebildet werden, wobei beispielsweise die Gruppen $R^1$ eine Lochtransport- oder eine Elektronentransportgruppe darstellen kann, wobei die in den Formeln (H-1) bis (H-26) beziehungsweise (Q-1) bis (Q-44) dargelegten Reste $R^1$ durch entsprechende Reste $R^2$ ersetzt werden können.

In einer weiteren bevorzugten Ausführungsform der Erfindung steht $Ar^1$ gleich oder verschieden bei jedem Auftreten für ein aromatisches oder heteroaromatisches Ringsystem, vorzugsweise einen Aryl- oder Heteroarylrest mit 5 bis 24 aromatischen Ringatomen, bevorzugt mit 6 bis 18 aromatischen Ringatomen, besonders bevorzugt für ein aromatisches Ringsystem, vorzugsweise einen Arylrest mit 6 bis 10 aromatischen Ringatomen bzw. ein heteroaromatisches Ringsystem, vorzugsweise eine Heteroarylgruppe mit 5 bis 13 aromatischen Ringatomen, das jeweils durch einen oder mehrere Reste $R^2$ substituiert sein kann, bevorzugt aber unsubstituiert ist, wobei $R^2$ die zuvor, insbesondere in Formel (I) dargestellte Bedeutung aufweisen kann.

Vorzugsweise steht das Symbol $Ar^1$ für einen Aryl- oder Heteroarylrest, so dass eine aromatische oder heteroaromatische Gruppe eines aromatischen oder heteroaromatischen Ringsystems direkt, d. h. über ein Atom der aromatischen oder heteroaromatischen Gruppe, an das jeweilige Atom der weiteren Gruppe gebunden ist, beispielweise ein C- oder N-Atom der zuvor dargestellten Gruppen (H-1) bis (H-26) oder (Q-26) bis (Q-44).

Mit Vorteil stellt $Ar^1$ in den Formeln (H-1) bis (H-26) oder (Q-26) bis (Q-44) ein aromatisches Ringsystem mit 6 bis 12 aromatischen Ringatomen dar, welches mit einem oder mehreren Resten $R^2$ substituiert sein kann, vorzugsweise aber unsubstituiert ist, wobei $R^2$ die zuvor, insbesondere für Formel (I) dargestellte Bedeutung aufweisen kann.

Bevorzugt bilden die Reste $R^1$ oder $R^2$ in den Formeln (H-1) bis (H-26) oder (Q-1) bis (Q-44) mit den Ringatomen der Arylgruppe oder Heteroarylgruppe $Ar^1$, $Ar^2$, $Ar^3$ und/oder $Ar^4$, an die die Reste $R^1$ oder $R^2$ gebunden sind, kein kondensiertes Ringsystem. Dies schließt die Bildung eines kondensierten Ringsystems mit möglichen Substituenten $R^2$ bzw. $R^3$ ein, die an die Reste $R^1$ oder $R^2$ gebunden sein können.

Ferner kann vorgesehen sein, dass die Gruppe Ar, $Ar^1$, $Ar^2$, $Ar^3$ und/oder $Ar^4$ ausgewählt ist aus der Gruppe bestehend aus Phenyl, ortho-, meta- oder para-Biphenyl, Terphenyl, insbesondere ortho-, meta-, para- oder verzweigtes Terphenyl, Quaterphenyl, insbesondere ortho-, meta-, para- oder verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl, Pyrenyl, Triazinyl, Imidazolyl, Benzimidazolyl, Benzoxazolyl, Benzthiazolyl, 1-, 2-, 3- oder 4-Carbazolyl, 1- oder 2-Naphthyl, Anthracenyl, vorzugsweise 9-Anthracenyl, Phenanthrenyl und/oder Triphenylenyl, die jeweils durch einen oder mehrere Reste $R^2$ substituiert sein können, bevorzugt aber unsubstituiert sind, wobei Phenyl, Spirobifluoren-, Fluoren-, Dibenzofuran-, Dibenzothiophen-, Anthracen-, Phenanthren-, Triphenylen-Gruppen besonders bevorzugt sind.

Wenn X für $CR^1$ steht bzw. wenn die aromatische und/oder heteroaromatische Gruppen durch Substituenten $R^1$ substituiert sind, dann sind diese Substituenten $R^1$ bevorzugt gewählt aus der Gruppe bestehend aus H, D, F, CN, $N(Ar^1)_2$, $C(=O)Ar^1$, $P(=O)(Ar^1)_2$, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten $R^2$ substituiert sein kann, wobei eine oder mehrere nicht-benachbarte $CH_2$-Gruppen durch O ersetzt sein können und wobei ein oder mehrere H-Atome durch D oder F ersetzt sein können, einem aromatischen oder heteroaromatischen Ringsystem mit 5 bis 24 aromatischen Ringatomen, das jeweils mit einem oder mehreren Resten $R^2$ substituiert sein kann, bevorzugt aber unsubstituiert ist, oder einer Aralkyl- oder Heteroaralkylgruppe mit 5 bis 25 aromatischen Ringatomen, die mit einem oder mehreren Resten $R^2$ substituiert sein kann; wobei die Gruppe $Ar^1$ die zuvor, insbesondere für Formel (I) genannte Bedeutung aufweist.

Besonders bevorzugt sind diese Substituenten $R^1$ ausgewählt aus der Gruppe bestehend aus H, D, F, CN, $N(Ar^1)_2$, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3 oder 4 C-Atomen, oder einer Alkenylgruppe mit 2 bis 8 C-Atomen, bevorzugt mit 2, 3 oder 4 C-Atomen, die jeweils mit einem oder mehreren Resten $R^2$ substituiert sein kann, bevorzugt aber unsubstituiert ist, oder einem aromatischen oder heteroaromatischen Ringsystem mit 5 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren nicht-aromatischen Resten $R^1$ substituiert sein kann, bevorzugt aber unsubstituiert ist; wobei $Ar^1$ die zuvor dargelegte Bedeutung aufweisen kann.

Ganz besonders bevorzugt sind die Substituenten $R^1$ ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten $R^2$ substituiert sein kann, bevorzugt aber unsubstituiert ist. Beispiele für geeignete Substituenten $R^1$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind.

Weiterhin kann vorgesehen sein, dass in einer Struktur gemäß Formel (I), (IIa), (IIb), (IIc), (IId), (IIe), (IIf), (III), (IIIa), (IIIb), (IIIc), (IIId), (IIIe), (IIIf), (IV), (IVa), (IVb), (IVc), (IVd), (IVe), (IVf), (V), (Va), (Vb), (Vc), (Vd), (Ve), (Vf), (VI), (VIa), (VIb), (VIc), (VId), (VIe), (VIf), (VII), (VIIa), (VIIb), (VIIc), (VIId), (VIIe) und/oder (VIIf) mindestens ein Rest $R^1$ oder $Ar^1$ für eine Gruppe steht, die ausgewählt ist aus den Formeln ($R^1$-1) bis ($R^1$- 92), beziehungsweise in einer Struktur gemäß Formel (H-1) bis (H-26), (Q-1) bis (Q-44) mindestens ein Rest $Ar^1$ oder $R^1$ für eine Gruppe steht, die ausgewählt ist aus den Formeln ($R^1$-1) bis ($R^1$- 92),

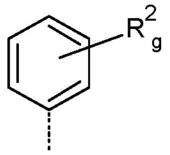
Formel ($R^1$-1)

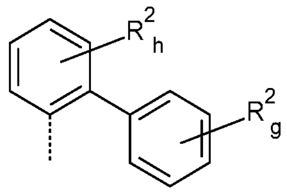
Formel ($R^1$-2)

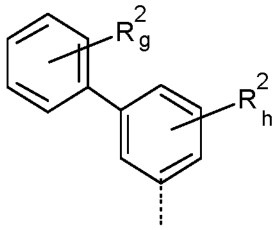
Formel ($R^1$-3)

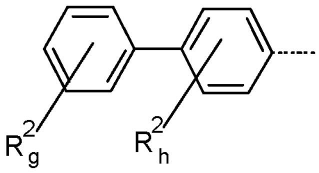
Formel ($R^1$-4)

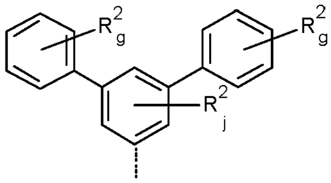
Formel ($R^1$-5)

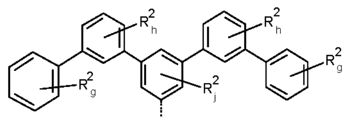
Formel ($R^1$-6)

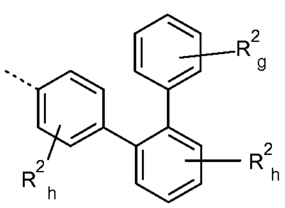
Formel ($R^1$-7)

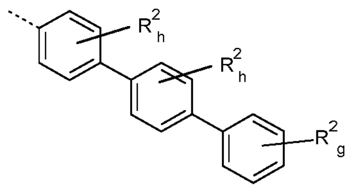
Formel ($R^1$-8)

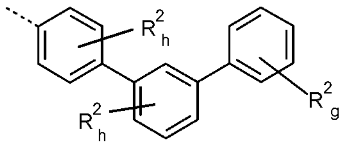
Formel ($R^1$-9)

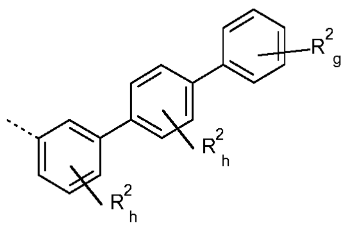
Formel ($R^1$-10)

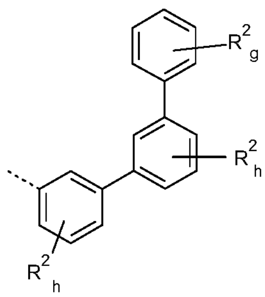
Formel ($R^1$-11)

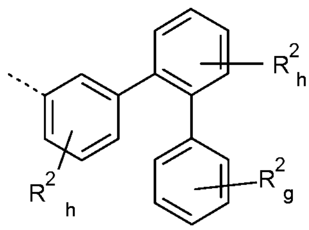
Formel ($R^1$-12)

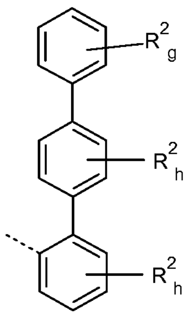

Formel ($R^1$-13)

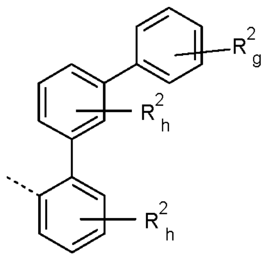

Formel ($R^1$-14)

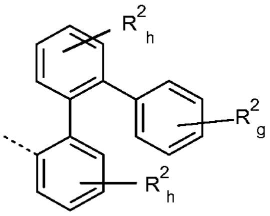

Formel ($R^1$-15)

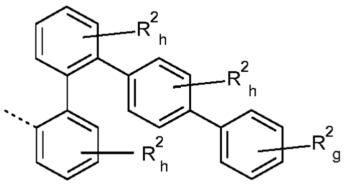

Formel ($R^1$-16)

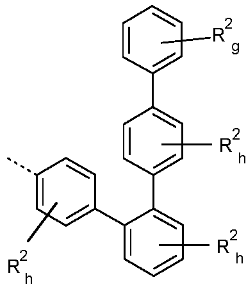

Formel ($R^1$-17)

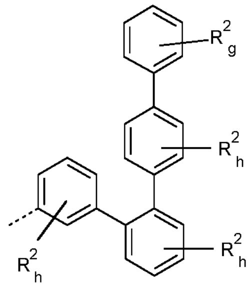

Formel ($R^1$-18)

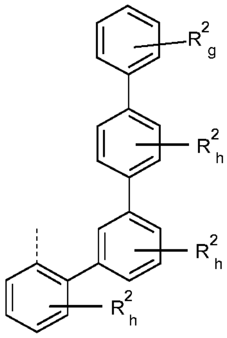

Formel ($R^1$-19)

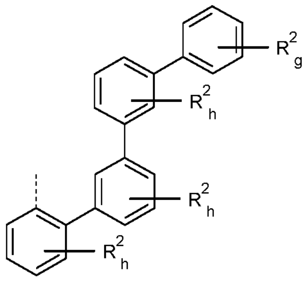

Formel ($R^1$-20)

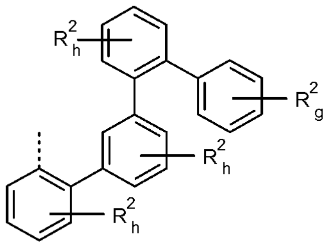

Formel ($R^1$-21)

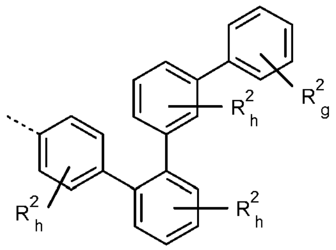

Formel ($R^1$-22)

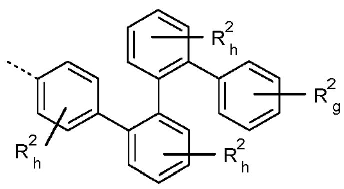

Formel ($R^1$-23)

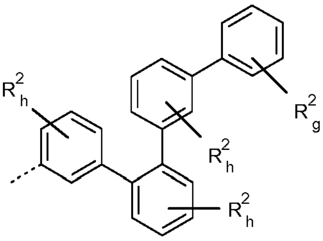

Formel ($R^1$-24)

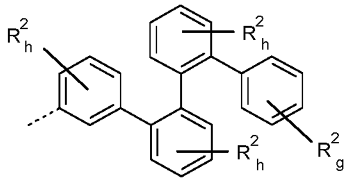

Formel ($R^1$-25)

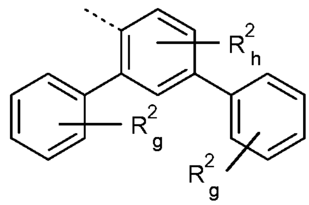

Formel ($R^1$-26)

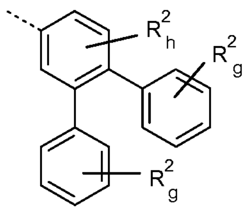

Formel ($R^1$-27)

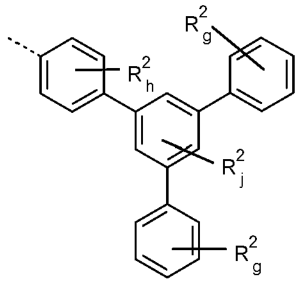

Formel ($R^1$-28)

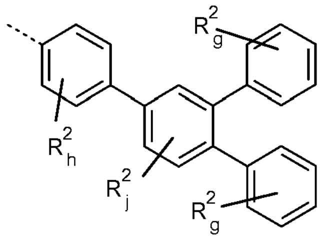

Formel ($R^1$-29)

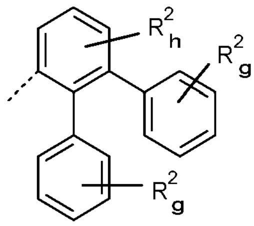

Formel ($R^1$-30)

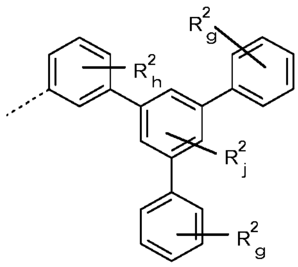

Formel ($R^1$-31)

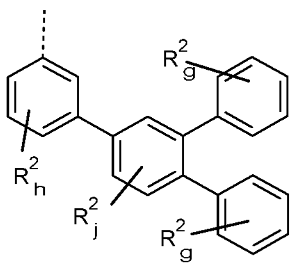

Formel ($R^1$-32)

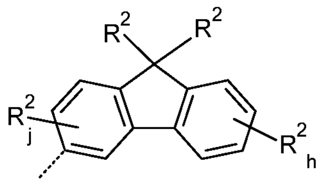

Formel ($R^1$-33)

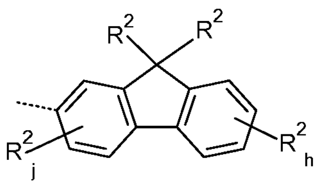

Formel ($R^1$-34)

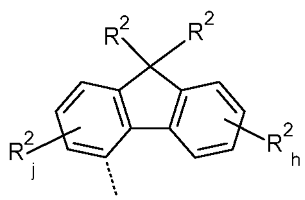

Formel ($R^1$-35)

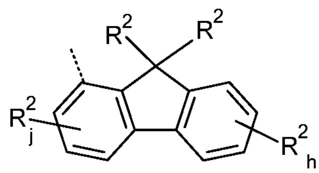

Formel ($R^1$-36)

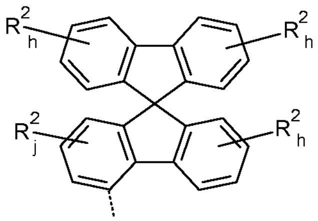

Formel ($R^1$-37)

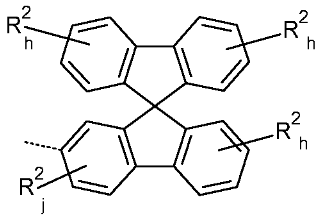

Formel ($R^1$-38)

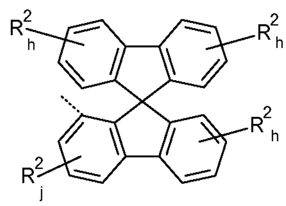

Formel ($R^1$-39)

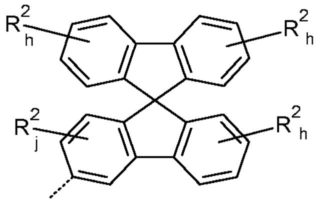
Formel ($R^1$-40)

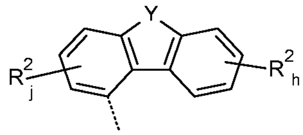
Formel ($R^1$-41)

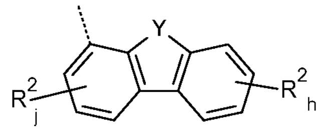
Formel ($R^1$-42)

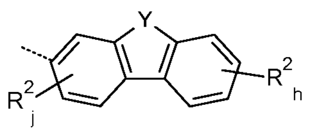
Formel ($R^1$-43)

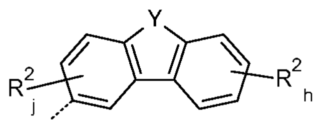
Formel ($R^1$-44)

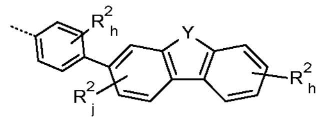
Formel ($R^1$-45)

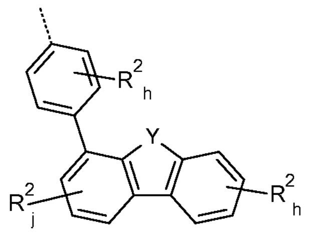
Formel ($R^1$-46)

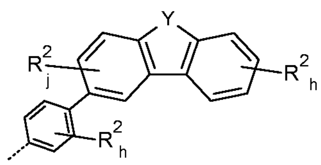
Formel ($R^1$-47)

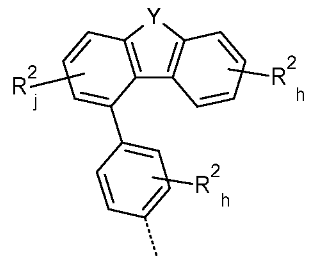
Formel ($R^1$-48)

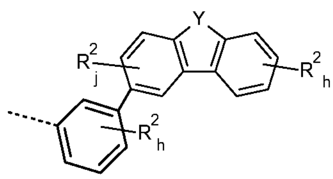
Formel ($R^1$-49)

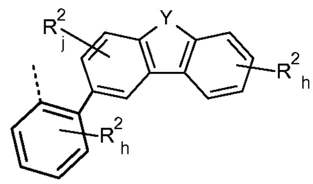
Formel ($R^1$-50)

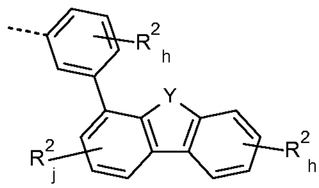
Formel ($R^1$-51)

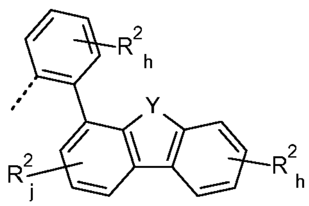
Formel ($R^1$-52)

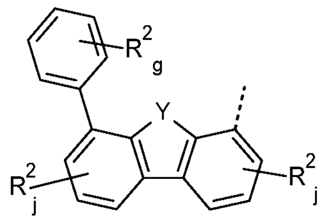
Formel ($R^1$-53)

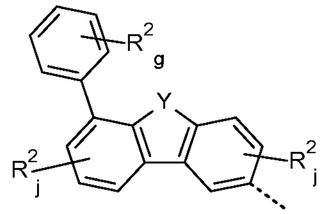
Formel ($R^1$-54)

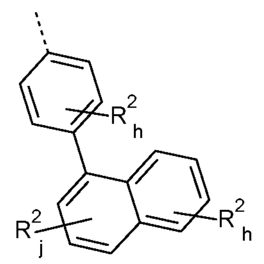

Formel ($R^1$-55)

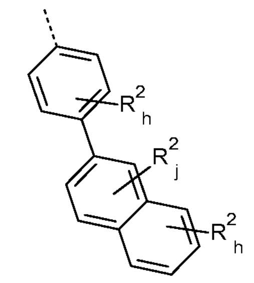

Formel ($R^1$-56)

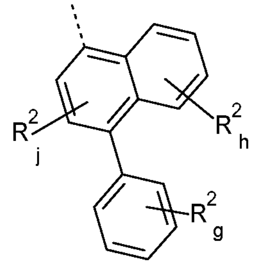

Formel ($R^1$-57)

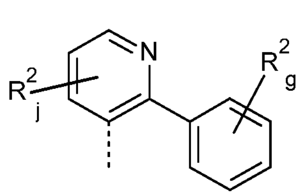

Formel ($R^1$-58)

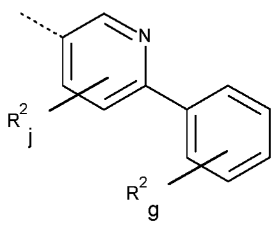

Formel ($R^1$-59)

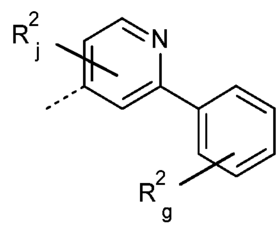

Formel ($R^1$-60)

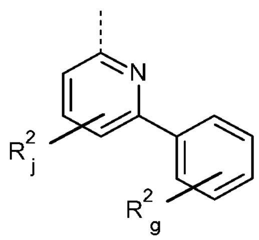

Formel ($R^1$-61)

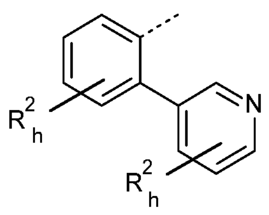

Formel ($R^1$-62)

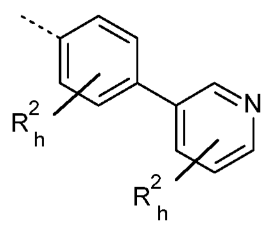

Formel ($R^1$-63)

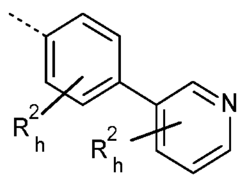

Formel ($R^1$-64)

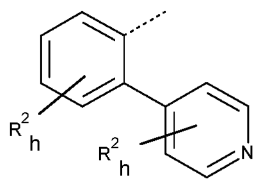

Formel ($R^1$-65)

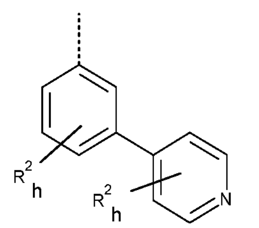

Formel ($R^1$-66)

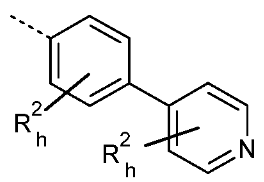

Formel ($R^1$-67)

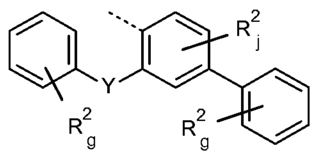

Formel ($R^1$-68)

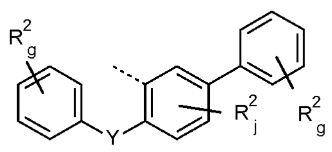

Formel ($R^1$-69)

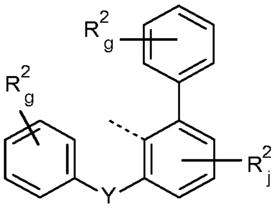

Formel ($R^1$-70)

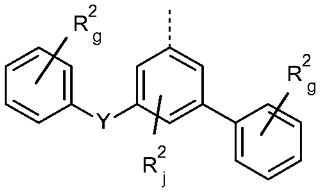

Formel ($R^1$-71)

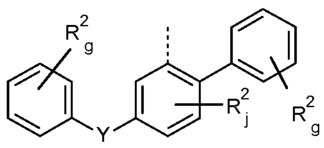

Formel ($R^1$-72)

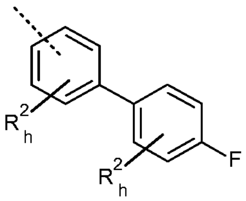

Formel ($R^1$-73)

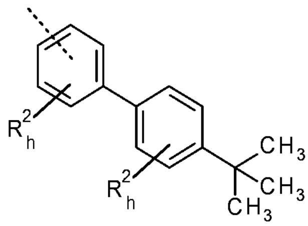

Formel ($R^1$-74)

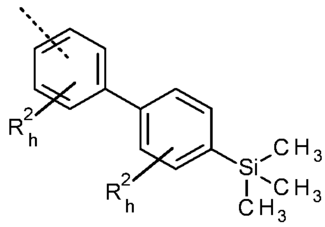

Formel ($R^1$-75)

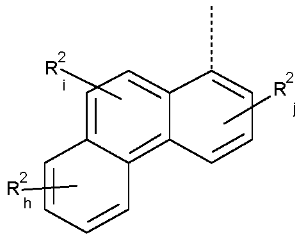

Formel ($R^1$-76)

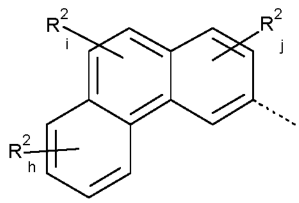

Formel ($R^1$-77)

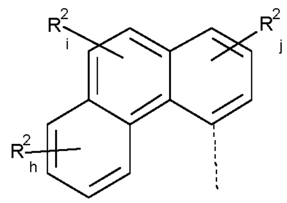

Formel ($R^1$-78)

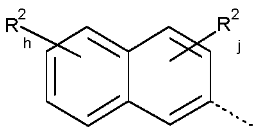

Formel ($R^1$-79)

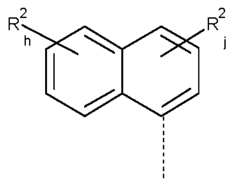

Formel ($R^1$-80)

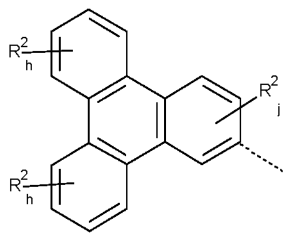

Formel ($R^1$-81)

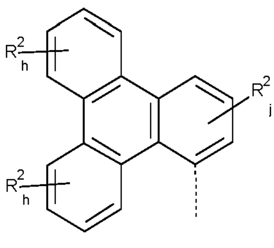

Formel ($R^1$-82)

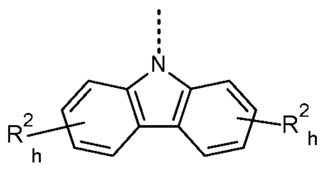

Formel ($R^1$-83)

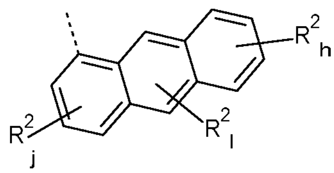

Formel ($R^1$-84)

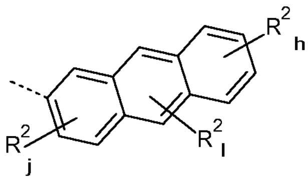
Formel ($R^1$-85)

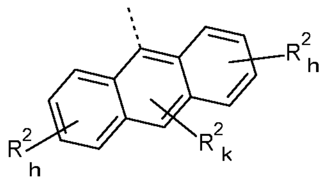
Formel ($R^1$-86)

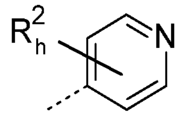
Formel ($R^1$-87)

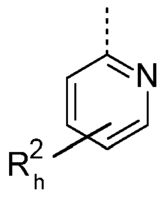
Formel ($R^1$-88)

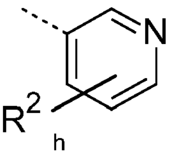
Formel ($R^1$-89)

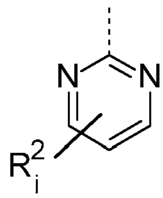
Formel ($R^1$-90)

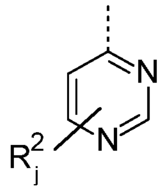
Formel ($R^1$-91)

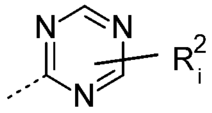
Formel ($R^1$-92)

wobei für die verwendeten Symbole gilt:

- Y ist O, S oder $NR^2$, vorzugsweise O oder S;
- k ist bei jedem Auftreten unabhängig 0 oder 1;
- i ist bei jedem Auftreten unabhängig 0, 1 oder 2;
- j ist bei jedem Auftreten unabhängig 0, 1, 2 oder 3;
- h ist bei jedem Auftreten unabhängig 0, 1, 2, 3 oder 4;
- g ist bei jedem Auftreten unabhängig 0, 1, 2, 3, 4 oder 5;
- $R^2$ kann die zuvor genannte, insbesondere für Formel (I) genannte Bedeutung aufweisen;

die gestrichelte Bindung markiert die Anbindungsposition.

Hierbei sind die Gruppen der Formeln $R^1$-1 bis $R^1$-56 bevorzugt, wobei die Gruppen $R^1$-1, $R^1$-3, R’-5, R’-6, R’-15, $R^1$-29, $R^1$-30, $R^1$-31, $R^1$-32, $R^1$-33, $R^1$-38, $R^1$-39, $R^1$-40, $R^1$-41, $R^1$-42, $R^1$-43, $R^1$-44 und/oder $R^1$-45 besonders bevorzugt sind.

Vorzugsweise kann vorgesehen sein, dass die Summe der Indices k, i, j, h und g in den Strukturen der Formel ($R^1$-1) bis ($R^1$-92) jeweils höchstens 3, vorzugsweise höchstens 2 und besonders bevorzugt höchstens 1 beträgt.

Bevorzugt bilden die Reste $R^2$ in den Formeln ($R^1$-1) bis ($R^1$-92) mit den Ringatomen der Arylgruppe oder Heteroarylgruppe, an die die Reste $R^2$ gebunden sind, kein kondensiertes aromatisches oder heteroaromatisches Ringsystem, vorzugsweise kein kondensiertes Ringsystem. Dies schließt die Bildung eines kondensierten Ringsystems mit möglichen Substituenten $R^3$ ein, die an die Reste $R^2$ gebunden sein können.

Die zuvor dargelegten Reste der Formeln ($R^1$-1) bis ($R^1$-92) stellen bevorzugte Reste Ar gemäß Formel (I) beziehungsweise $Ar^3$, $Ar^4$ gemäß Formeln (H-1) bis (H-3) oder bevorzugte Ausführungsformen dieser Formeln dar, wobei in diesem Fall die in den Formeln ($R^1$-1) bis ($R^1$-92) dargelegten Gruppen $R^2$ durch Reste $R^1$ zu ersetzen sind. Die zuvor dargelegten Bevorzugungen hinsichtlich der Formeln ($R^1$-1) bis ($R^1$-92) gelten entsprechend.

Bevorzugt sind Verbindungen, umfassend mindestens eine Struktur der Formeln (H-1) bis (H-26), in denen die Gruppe $Ar^2$ für eine Gruppe steht, die ausgewählt ist aus den Formeln ($L^1$-1) bis ($L^1$-108), und/oder Verbindungen, umfassend Strukturen der Formel (QL), in denen die Gruppe L’ für eine Bindung oder für eine Gruppe steht, die ausgewählt ist aus den Formeln ($L^1$-1) bis ($L^1$-108),

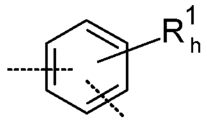

Formel ($L^1$-1)

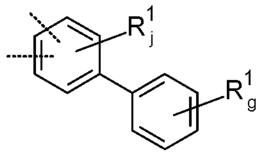

Formel ($L^1$-2)

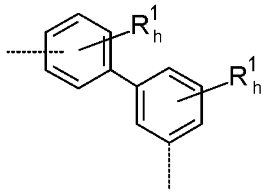

Formel ($L^1$-3)

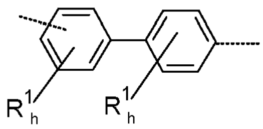

Formel ($L^1$-4)

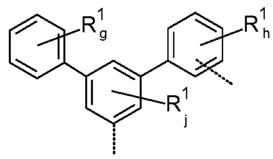

Formel ($L^1$-5)

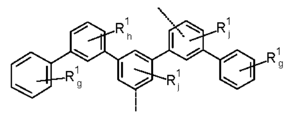

Formel ($L^1$-6)

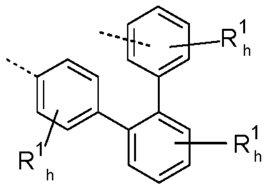

Formel ($L^1$-7)

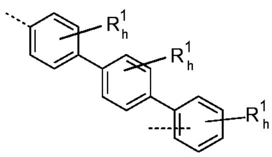

Formel ($L^1$-8)

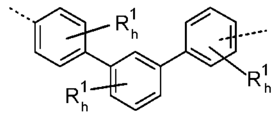

Formel ($L^1$-9)

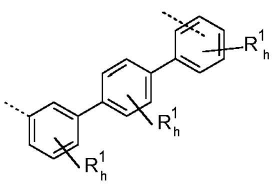

Formel ($L^1$-10)

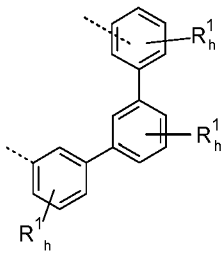

Formel ($L^1$-11)

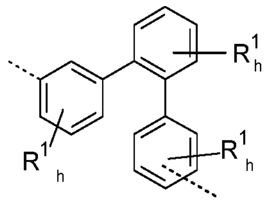

Formel ($L^1$-12)

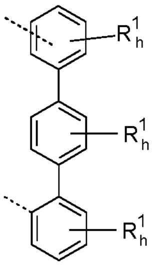

Formel ($L^1$-13)

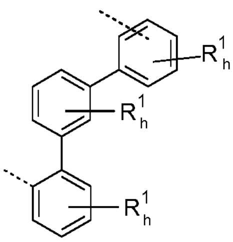

Formel ($L^1$-14)

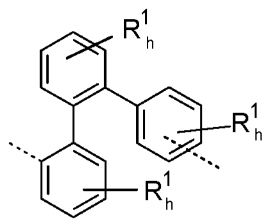

Formel ($L^1$-15)

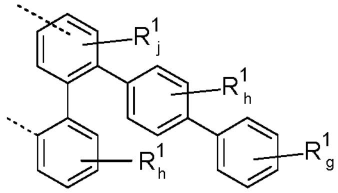
Formel ($L^1$-16)

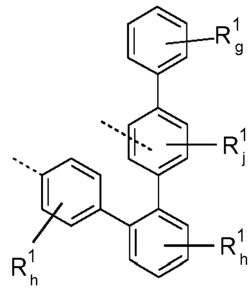
Formel ($L^1$-17)

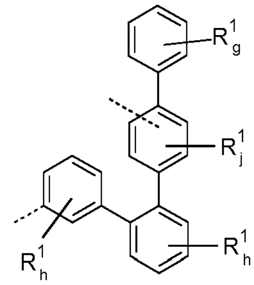
Formel ($L^1$-18)

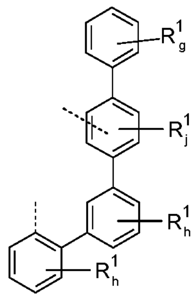
Formel ($L^1$-19)

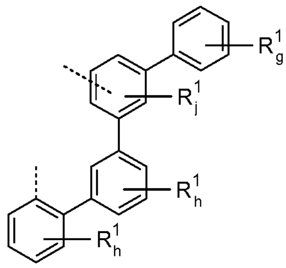
Formel ($L^1$-20)

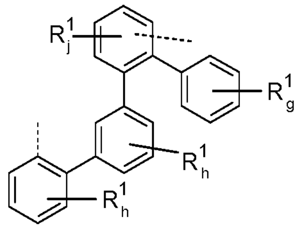
Formel ($L^1$-21)

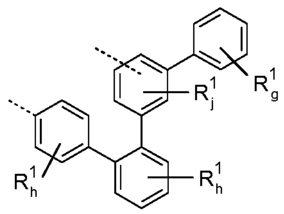
Formel ($L^1$-22)

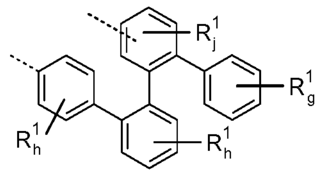
Formel ($L^1$-23)

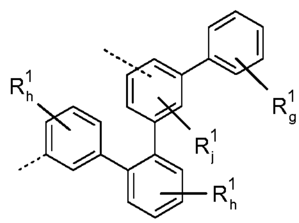
Formel ($L^1$-24)

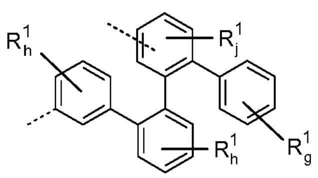
Formel ($L^1$-25)

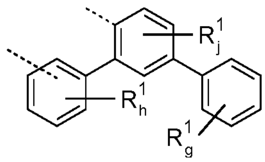
Formel ($L^1$-26)

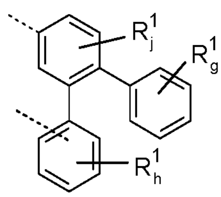
Formel ($L^1$-27)

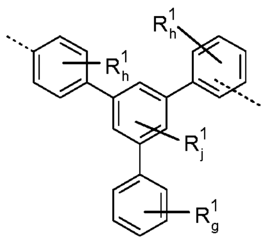
Formel ($L^1$-28)

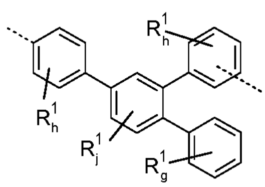
Formel ($L^1$-29)

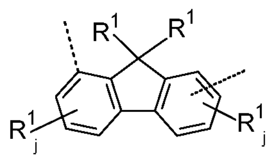
Formel ($L^1$-30)

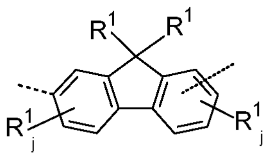
Formel ($L^1$-31)

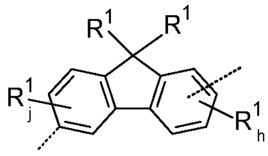
Formel ($L^1$-32)

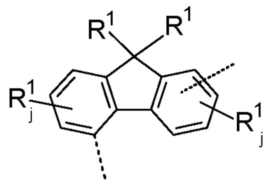
Formel ($L^1$-33)

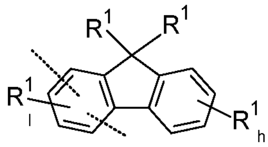
Formel ($L^1$-34)

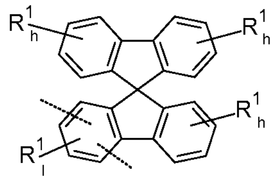
Formel ($L^1$-35)

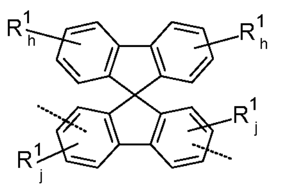
Formel ($L^1$-36)

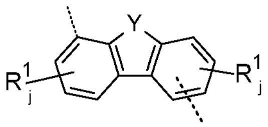
Formel ($L^1$-37)

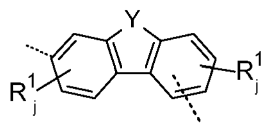
Formel ($L^1$-38)

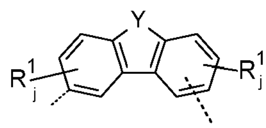
Formel ($L^1$-39)

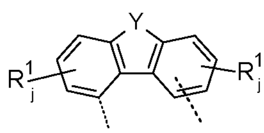
Formel ($L^1$-40)

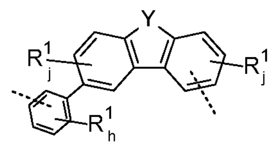
Formel ($L^1$-41)

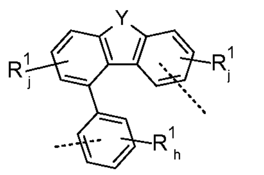
Formel ($L^1$-42)

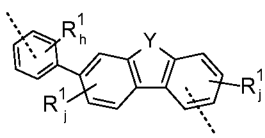
Formel ($L^1$-43)

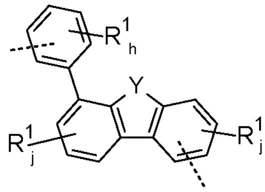
Formel ($L^1$-44)

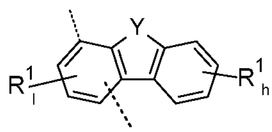
Formel ($L^1$-45)

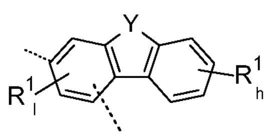
Formel ($L^1$-46)

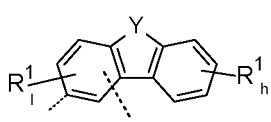
Formel ($L^1$-47)

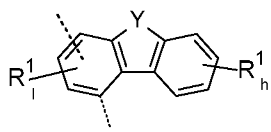
Formel ($L^1$-48)

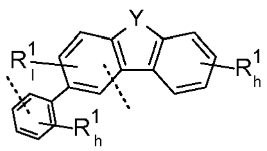

Formel ($L^1$-49)

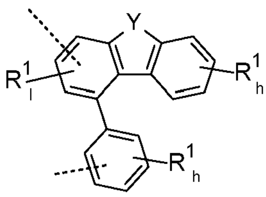

Formel ($L^1$-50)

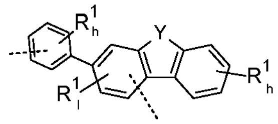

Formel ($L^1$-51)

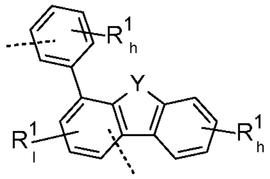

Formel ($L^1$-52)

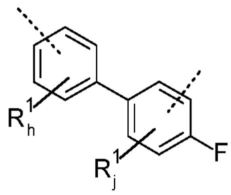

Formel ($L^1$-53)

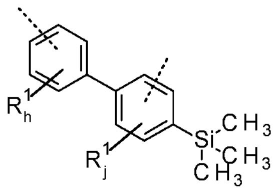

Formel ($L^1$-54)

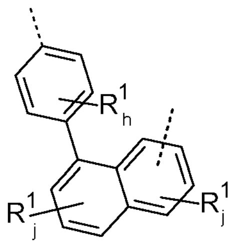

Formel ($L^1$-55)

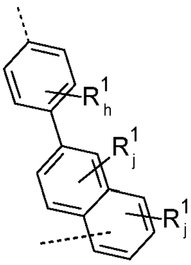

Formel ($L^1$-56)

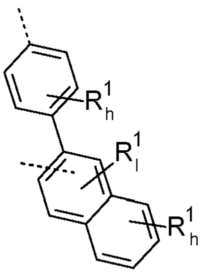

Formel ($L^1$-57)

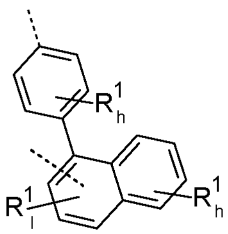

Formel ($L^1$-58)

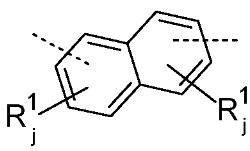

Formel ($L^1$-59)

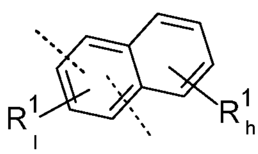

Formel ($L^1$-60)

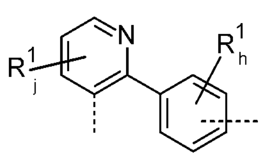

Formel ($L^1$-61)

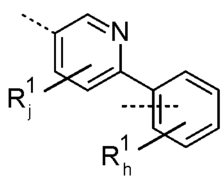

Formel ($L^1$-62)

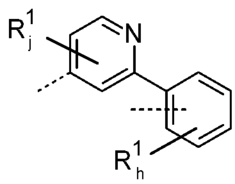

Formel ($L^1$-63)

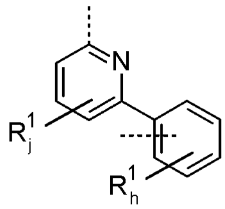
Formel (L1-64)

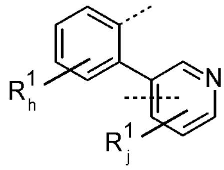
Formel (L1-65)

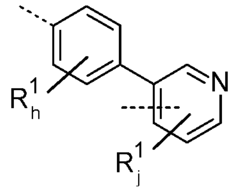
Formel (L1-66)

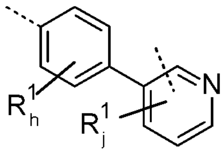
Formel (L1-67)

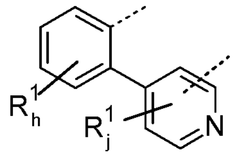
Formel (L1-68)

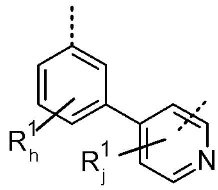
Formel (L1-69)

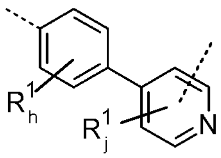
Formel (L1-70)

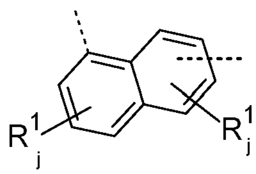
Formel (L1-71)

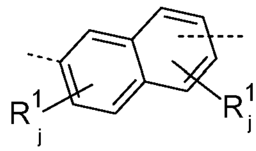
Formel (L1-72)

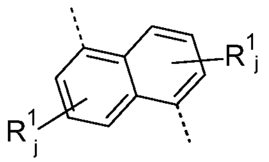
Formel (L1-73)

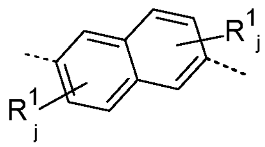
Formel (L1-74)

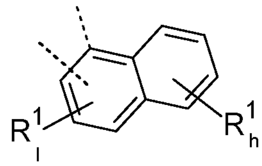
Formel (L1-75)

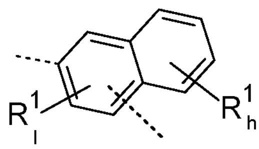
Formel (L1-76)

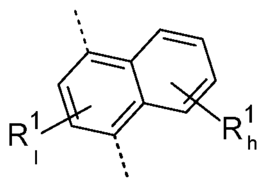
Formel (L1-77)

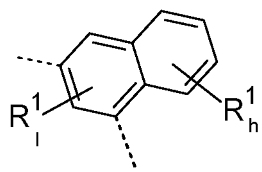
Formel (L1-78)

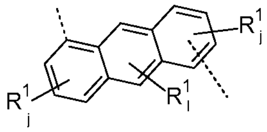
Formel (L1-79)

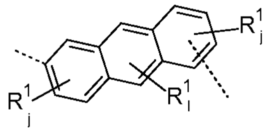
Formel (L1-80)

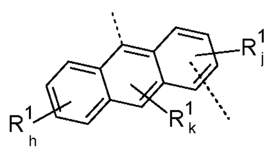
Formel (L1-81)

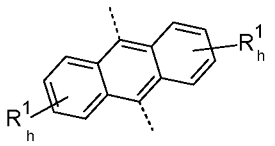

Formel ($L^1$-82)

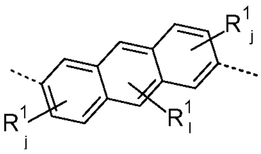

Formel ($L^1$-83)

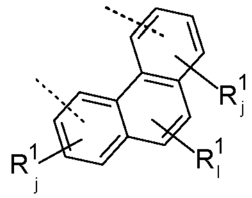

Formel ($L^1$-84)

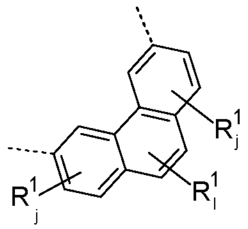

Formel ($L^1$-85)

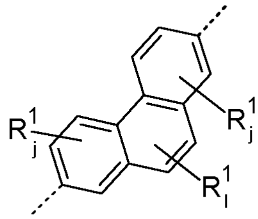

Formel ($L^1$-86)

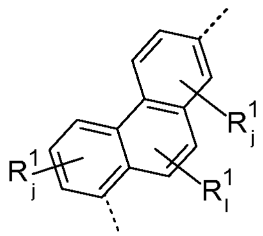

Formel ($L^1$-87)

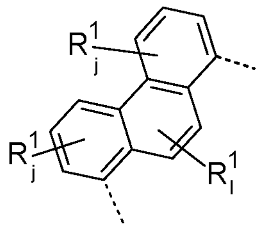

Formel ($L^1$-88)

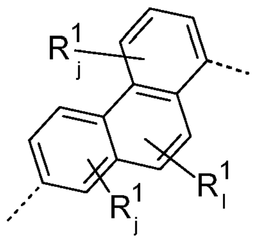

Formel ($L^1$-89)

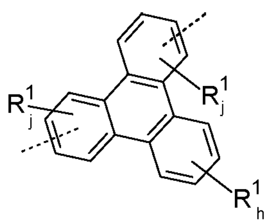

Formel ($L^1$-90)

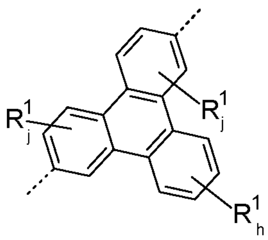

Formel ($L^1$-91)

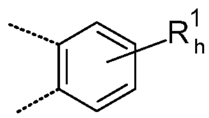

Formel ($L^1$-92)

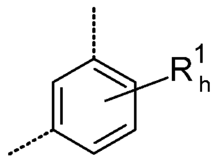

Formel ($L^1$-93)

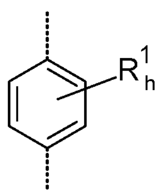

Formel ($L^1$-94)

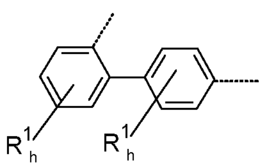

Formel ($L^1$-95)

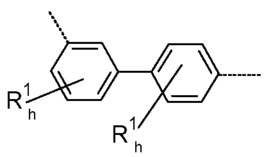

Formel ($L^1$-96)

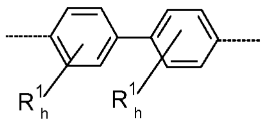

Formel ($L^1$-97)

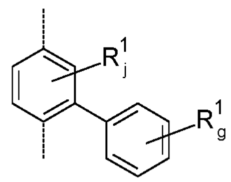

Formel ($L^1$-98)

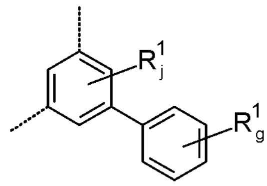

Formel ($L^1$-99)

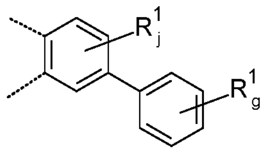

Formel ($L^1$-100)

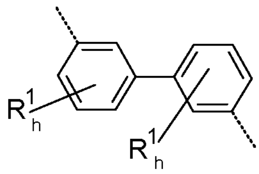

Formel ($L^1$-101)

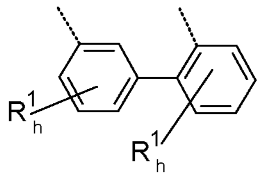

Formel ($L^1$-102)

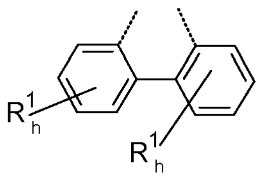

Formel ($L^1$-103)

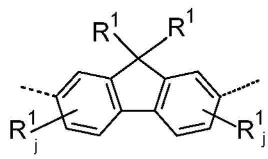

Formel ($L^1$-104)

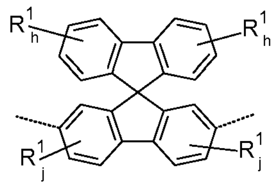

Formel ($L^1$-105)

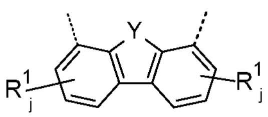

Formel ($L^1$-106)

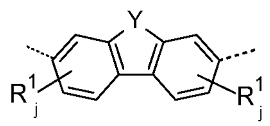

Formel ($L^1$-107)

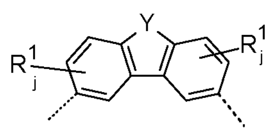

Formel ($L^1$-108)

wobei die gestrichelten Bindungen jeweils die Anbindungspositionen markieren, der Index k 0 oder 1 ist, der Index l 0, 1 oder 2 ist, der Index j bei jedem Auftreten unabhängig 0, 1, 2 oder 3 ist; der Index h bei jedem Auftreten unabhängig 0, 1, 2, 3 oder 4 ist, der Index g 0, 1, 2, 3, 4 oder 5 ist; das Symbol Y O, S oder $NR^1$, vorzugsweise O oder S ist; und das Symbol $R^1$ die zuvor, insbesondere für Formel (I) genannte Bedeutung aufweist.

Vorzugsweise kann vorgesehen sein, dass die Summe der Indices k, l, g, h und j in den Strukturen der Formel ($L^1$-1) bis ($L^1$-108) jeweils höchstens 3, vorzugsweise höchstens 2 und besonders bevorzugt höchstens 1 beträgt.

Bevorzugte erfindungsgemäße Verbindungen mit einer Gruppe der Formeln (H-1) bis (H-26) umfassen eine Gruppe $Ar^2$, die ausgewählt ist aus einer der Formeln ($L^1$-1) bis ($L^1$-78) und/oder ($L^1$-92) bis ($L^1$-108), bevorzugt der Formel ($L^1$-1) bis ($L^1$-54) und/oder ($L^1$-92) bis ($L^1$-108), speziell bevorzugt der Formel ($L^1$-1) bis ($L^1$-29) und/oder ($L^1$-92) bis ($L^1$-103). Mit Vorteil kann die Summe der Indices k, l, g, h und j in den Strukturen der Formeln ($L^1$-1) bis ($L^1$-78) und/oder ($L^1$-92) bis ($L^1$-108), bevorzugt der Formel ($L^1$-1) bis ($L^1$-54) und/oder ($L^1$-92) bis ($L^1$-108), speziell bevorzugt der Formel ($L^1$-1) bis ($L^1$-29) und/oder ($L^1$-92) bis ($L^1$-103) jeweils höchstens 3, vorzugsweise höchstens 2 und besonders bevorzugt höchstens 1 betragen.

Bevorzugte erfindungsgemäße Verbindungen mit einer Gruppe der Formel (QL) umfassen eine Gruppe L', die eine Bindung darstellt oder die ausgewählt ist aus einer der Formeln ($L^1$-1) bis ($L^1$-78) und/oder ($L^1$-92) bis ($L^1$-108), bevorzugt der Formel ($L^1$-1) bis ($L^1$-54) und/oder ($L^1$-92) bis ($L^1$-108), speziell bevorzugt der Formel ($L^1$-1) bis ($L^1$-29) und/oder ($L^1$-92) bis ($L^1$-103). Mit Vorteil kann die Summe der Indices k, l, g, h und j in den Strukturen der Formeln ($L^1$-1) bis ($L^1$-78) und/oder ($L^1$-92) bis ($L^1$-108), bevorzugt der Formel ($L^1$-1) bis ($L^1$-54) und/oder ($L^1$-92) bis ($L^1$-108), speziell bevorzugt der Formel ($L^1$-1) bis ($L^1$-29) und/oder ($L^1$-92) bis ($L^1$-103) jeweils höchstens 3, vorzugsweise höchstens 2 und besonders bevorzugt höchstens 1 betragen.

Bevorzugt bilden die Reste $R^2$ in den Formeln ($L^1$-1) bis ($L^1$-108) mit den Ringatomen der Arylgruppe oder Heteroarylgruppe, an die die Reste $R^2$ gebunden sind, kein kondensiertes aromatisches oder heteroaromatisches Ringsystem, vorzugsweise kein kondensiertes Ringsystem. Dies schließt die Bildung eines kondensierten Ringsystems mit möglichen Substituenten $R^3$ ein, die an die Reste $R^2$ gebunden sein können.

Wenn die erfindungsgemäße Verbindung mit aromatischen oder heteroaromatischen Gruppen $R^1$ bzw. $R^2$ substituiert ist, so ist es bevorzugt, wenn diese keine Aryl- oder Heteroarylgruppen mit mehr als zwei direkt aneinander kondensierten aromatischen Sechsringen aufweisen. Besonders bevorzugt weisen die Substituenten überhaupt keine Aryl- oder Heteroarylgruppen mit direkt aneinander kondensierten Sechsringen auf. Diese Bevorzugung ist mit der geringen Triplettenergie derartiger Strukturen zu begründen. Kondensierte Arylgruppen mit mehr als zwei direkt aneinander kondensierten aromatischen Sechsringen, die dennoch auch erfindungsgemäß geeignet sind, sind Phenanthren und Triphenylen, da auch diese ein hohes Triplettniveau aufweisen.

In einer weiteren bevorzugten Ausführungsform der Erfindung ist $R^2$, beispielsweise bei einer Struktur gemäß Formel (I) sowie bevorzugten Ausführungsformen dieser Struktur oder den Strukturen, bei denen Bezug auf diese Formeln genommen wird, bei jedem Auftreten gleich oder verschieden ausgewählt aus der Gruppe bestehend aus H, D, einem aliphatischen Kohlenwasserstoffrest mit 1 bis 10 C-Atomen, bevorzugt mit 1, 2, 3 oder 4 C-Atomen, oder einem aromatischen oder heteroaromatischen Ringsystem mit 5 bis 30 aromatischen Ringatomen, bevorzugt mit 5 bis 24 aromatischen Ringatome, besonders bevorzugt mit 5 bis 13 aromatischen Ringatomen, das durch ein oder mehrere Alkylgruppen mit jeweils 1 bis 4 Kohlenstoffatomen substituiert sein kann, bevorzugt aber unsubstituiert ist.

Bevorzugt bilden die Reste $R^2$ mit den Ringatomen der Arylgruppe oder Heteroarylgruppe, an die die Reste $R^2$ gebunden sind, kein kondensiertes aromatisches oder heteroaromatisches Ringsystem, vorzugsweise kein kondensiertes Ringsystem. Dies schließt die Bildung eines kondensierten Ringsystems mit möglichen Substituenten $R^3$ ein, die an die Reste $R^2$ gebunden sein können.

In einer weiteren bevorzugten Ausführungsform der Erfindung ist $R^3$, beispielsweise bei einer Struktur gemäß Formel (I) sowie bevorzugten Ausführungsformen dieser Struktur oder den Strukturen, bei denen Bezug auf diese Formeln genommen wird, bei jedem Auftreten gleich oder verschieden ausgewählt aus der Gruppe bestehend aus H, D, F, CN, einem aliphatischen Kohlenwasserstoffrest mit 1 bis 10 C-Atomen, bevorzugt mit 1, 2, 3 oder 4 C-Atomen, oder einem aromatischen oder heteroaromatischen Ringsystem mit 5 bis 30 aromatischen Ringatomen, bevorzugt mit 5 bis 24 aromatischen Ringatome, besonders bevorzugt mit 5 bis 13 aromatischen Ringatomen, das durch ein oder mehrere Alkylgruppen mit jeweils 1 bis 4 Kohlenstoffatomen substituiert sein kann, bevorzugt aber unsubstituiert ist.

Beispiele für geeignete erfindungsgemäße Verbindungen (Formeln 1 bis 144 und 151 bis 153) sowie * weitere Referenzverbindungen (Formeln 145 bis 150), die nicht Teil der Erfindung sind, sind die nachstehend gezeigten Strukturen gemäß den folgenden Formeln 1 bis 153:

| | | |
|---|---|---|
| 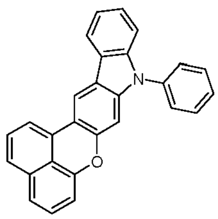 | 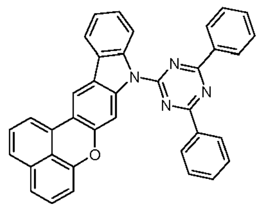 | 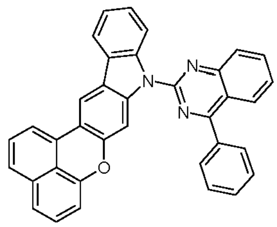 |
| Formel 1 | Formel 2 | Formel 3 |
| 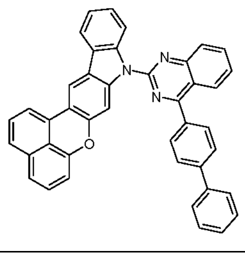 | 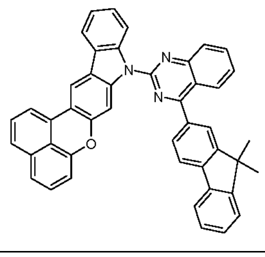 | 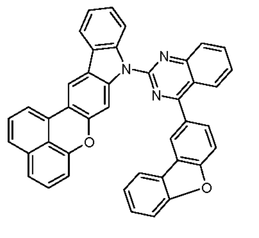 |
| Formel 4 | Formel 5 | Formel 6 |

(fortgesetzt)

| | | |
|---|---|---|
| 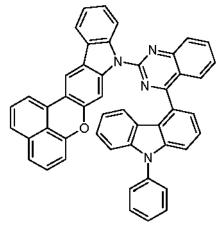 | 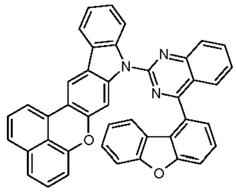 | 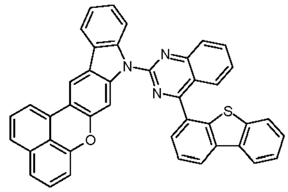 |
| Formel 7 | Formel 8 | Formel 9 |
| 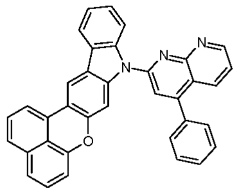 | 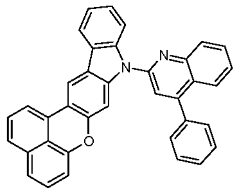 | 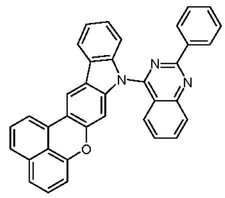 |
| Formel 10 | Formel 11 | Formel 12 |
| 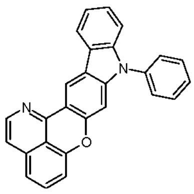 | 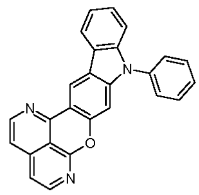 | 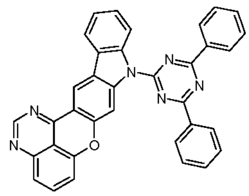 |
| Formel 13 | Formel 14 | Formel 15 |
| 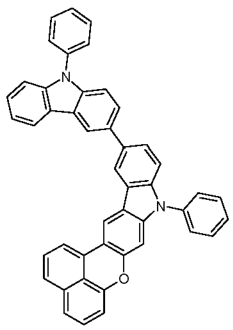 | 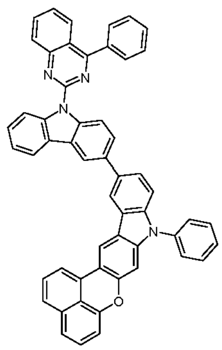 | 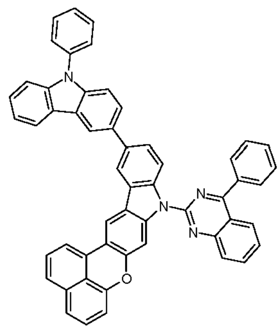 |
| Formel 16 | Formel 17 | Formel 18 |
| 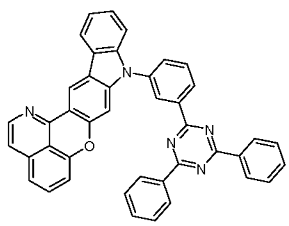 | 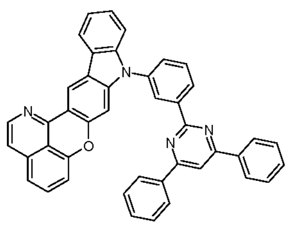 | 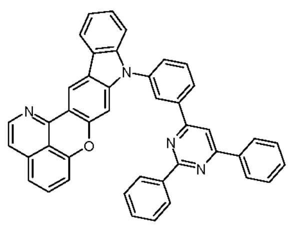 |
| Formel 19 | Formel 20 | Formel 21 |
| 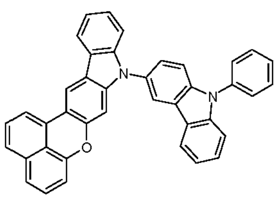 | 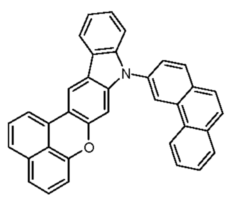 | 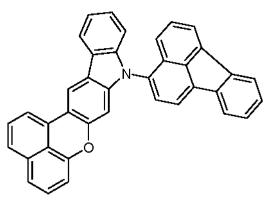 |

(fortgesetzt)

| Formel 22 | Formel 23 | Formel 24 |
|---|---|---|
| 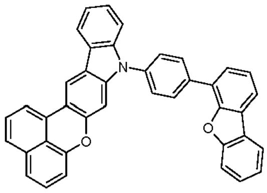 | 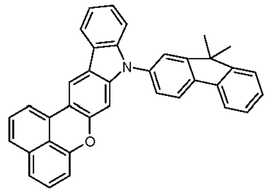 | 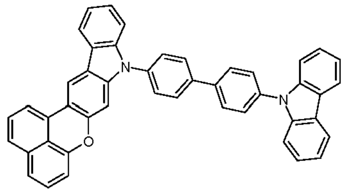 |
| Formel 25 | Formel 26 | Formel 27 |
| 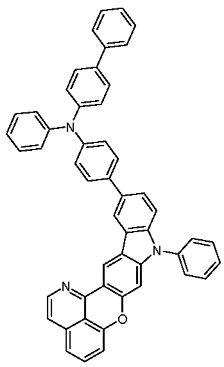 | 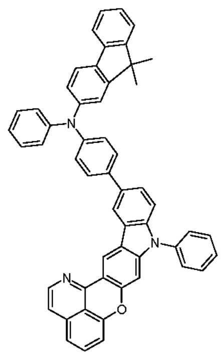 | 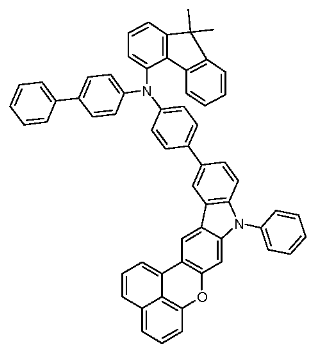 |
| Formel 28 | Formel 29 | Formel 30 |
| 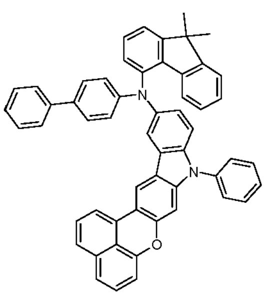 | 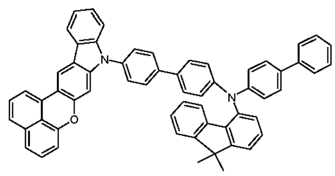 | 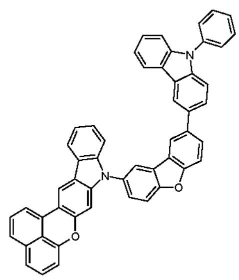 |
| Formel 31 | Formel 32 | Formel 33 |
| 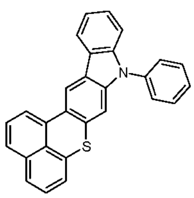 | 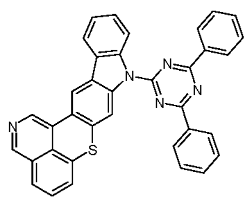 | 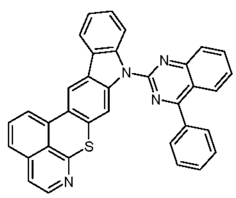 |
| Formel 34 | Formel 35 | Formel 36 |
| 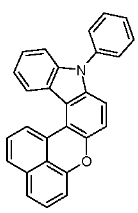 | 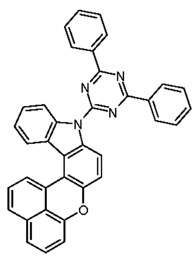 | 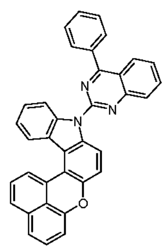 |
| Formel 37 | Formel 38 | Formel 39 |

(fortgesetzt)

| | | |
|---|---|---|
| 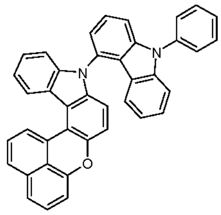 | 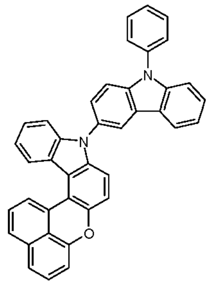 | 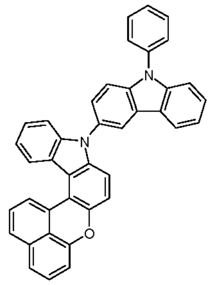 |
| Formel 40 | Formel 41 | Formel 42 |
| 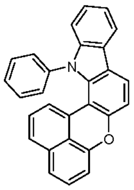 | 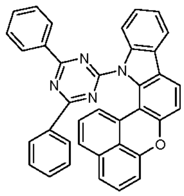 | 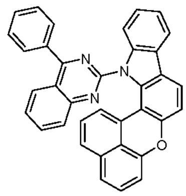 |
| Formel 43 | Formel 44 | Formel 45 |
| 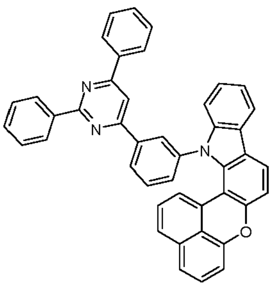 | 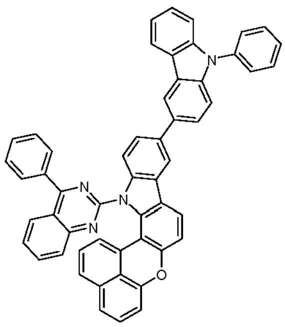 | 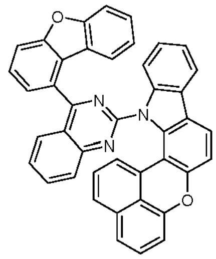 |
| Formel 46 | Formel 47 | Formel 48 |
| 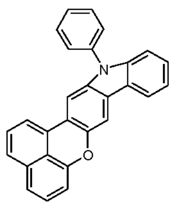 | 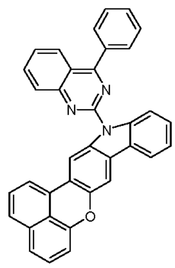 | 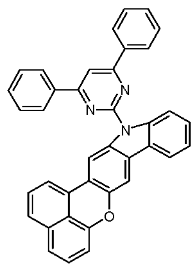 |
| Formel 49 | Formel 50 | Formel 51 |
| 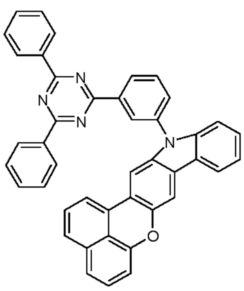 | 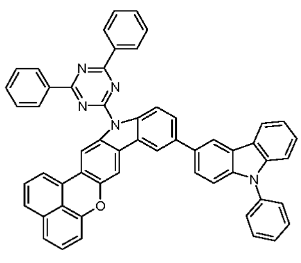 | 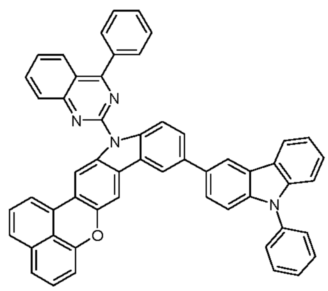 |
| Formel 52 | Formel 53 | Formel 54 |

(fortgesetzt)

| | | |
|---|---|---|
| 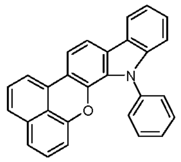 | 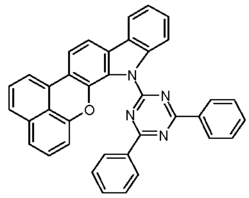 | 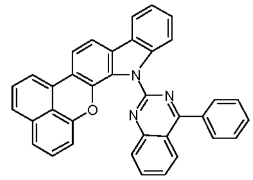 |
| Formel 55 | Formel 56 | Formel 57 |
| 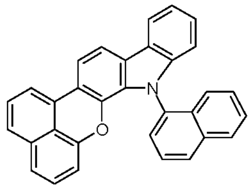 | 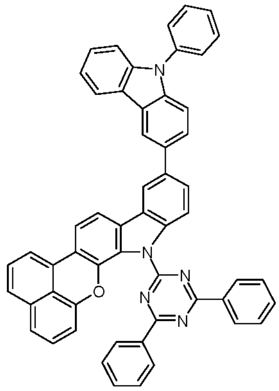 | 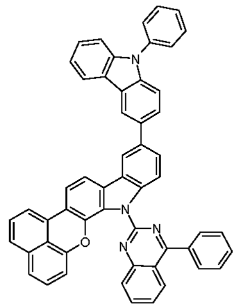 |
| Formel 58 | Formel 59 | Formel 60 |
| 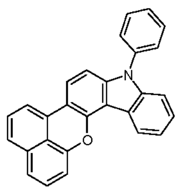 | 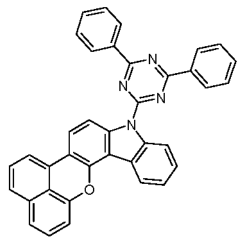 | 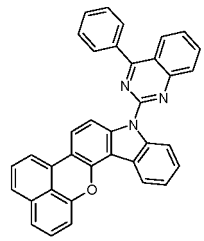 |
| Formel 61 | Formel 62 | Formel 63 |
| 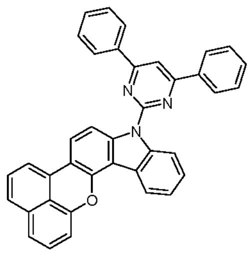 | 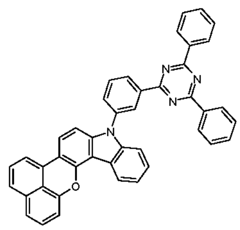 | 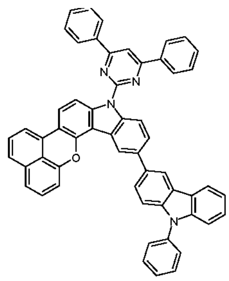 |
| Formel 64 | Formel 65 | Formel 66 |
| 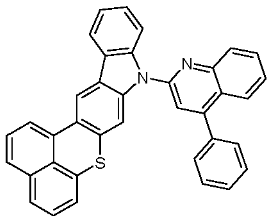 | 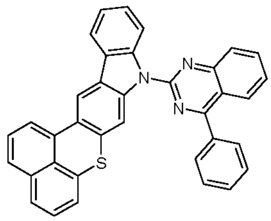 | 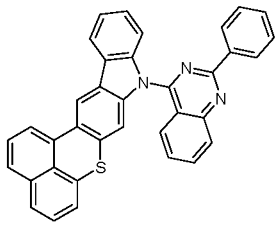 |
| Formel 67 | Formel 68 | Formel 69 |

(fortgesetzt)

| | | |
|---|---|---|
| 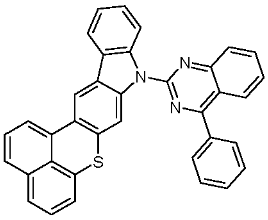 | 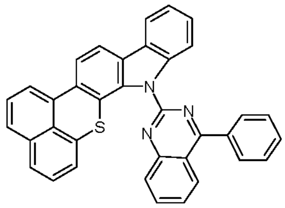 | 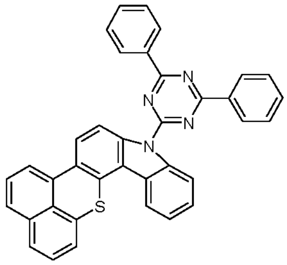 |
| Formel 70 | Formel 71 | Formel 72 |
| 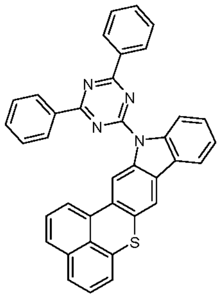 | 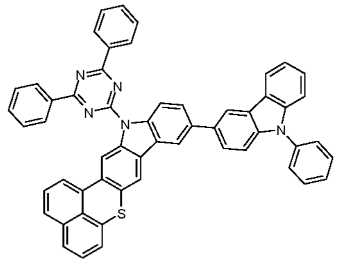 | 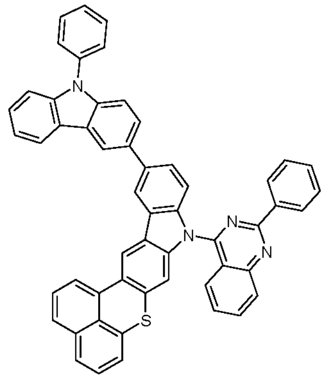 |
| Formel 73 | Formel 74 | Formel 75 |
| 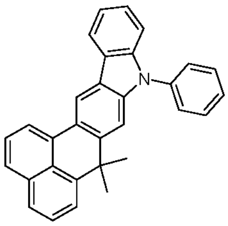 | 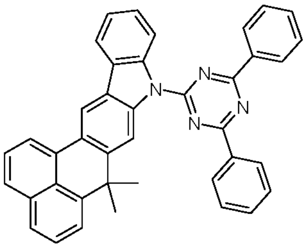 | 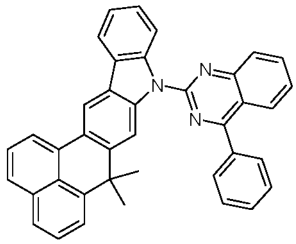 |
| Formel 76 | Formel 77 | Formel 78 |
| 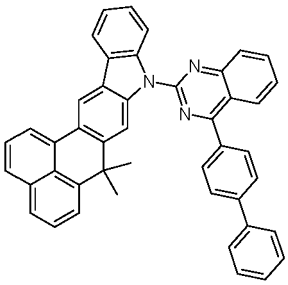 | 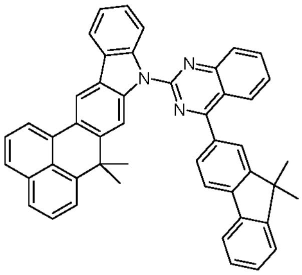 | 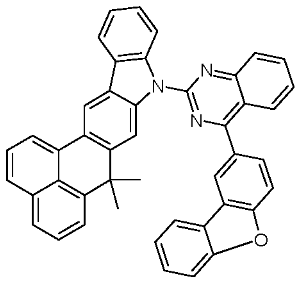 |
| Formel 79 | Formel 80 | Formel 81 |

(fortgesetzt)

| | | |
|---|---|---|
| 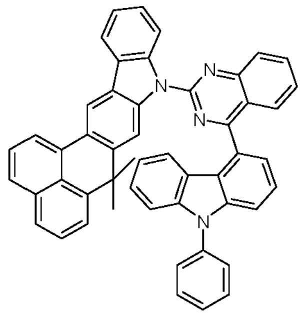 | 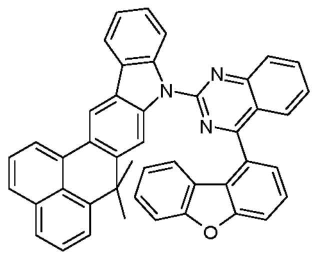 | 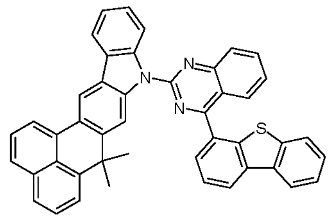 |
| Formel 82 | Formel 83 | Formel 84 |
| 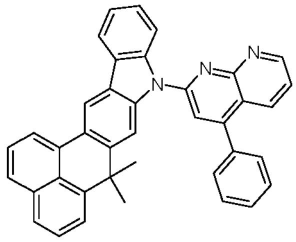 | 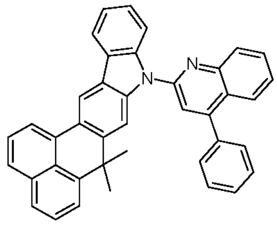 | 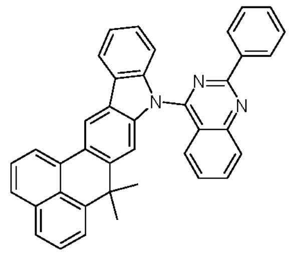 |
| Formel 85 | Formel 86 | Formel 87 |
| 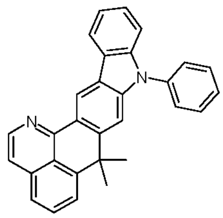 | 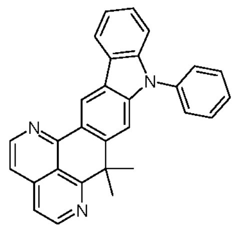 | 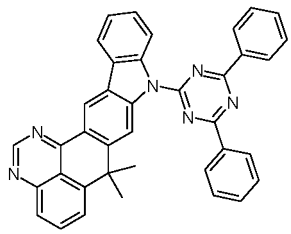 |
| Formel 88 | Formel 89 | Formel 90 |
| 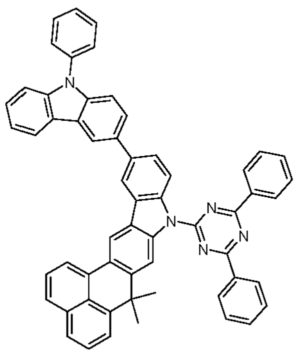 | 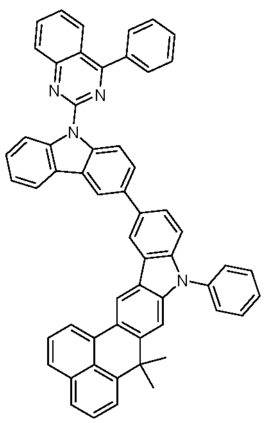 | 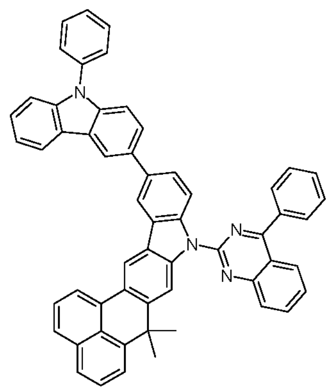 |
| Formel 91 | Formel 92 | Formel 93 |

(fortgesetzt)

| | | |
|---|---|---|
| 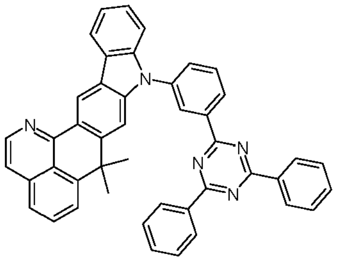 | 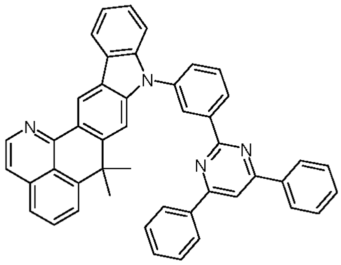 | 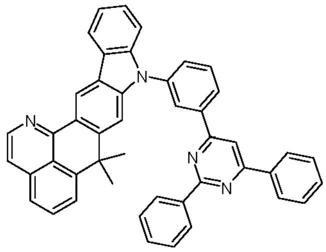 |
| Formel 94 | Formel 95 | Formel 96 |
| 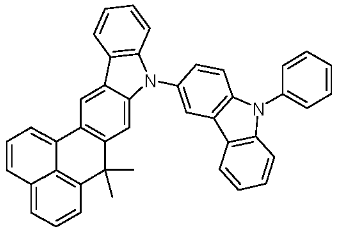 | 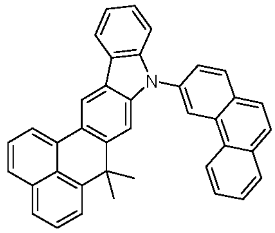 | 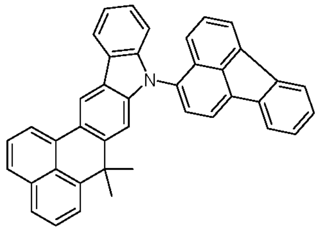 |
| Formel 97 | Formel 98 | Formel 99 |
| 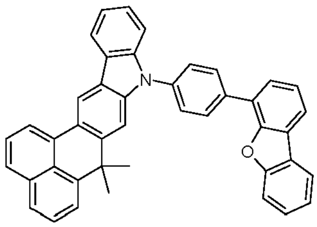 | 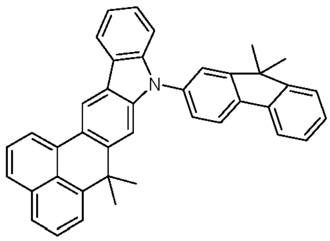 | 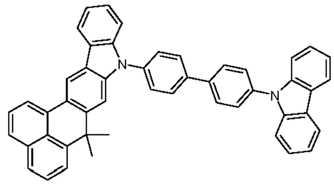 |
| Formel 100 | Formel 101 | Formel 102 |
| 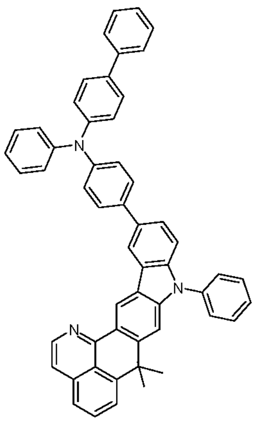 | 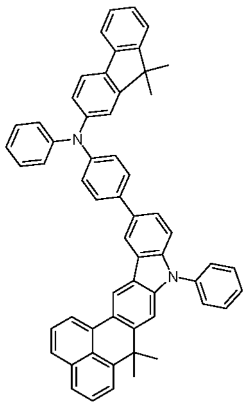 | 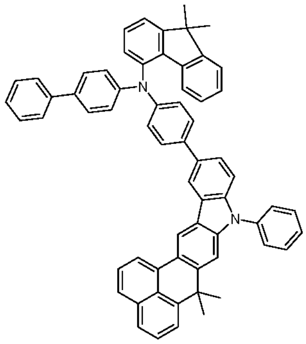 |
| Formel 103 | Formel 104 | Formel 105 |

(fortgesetzt)

| | | |
|---|---|---|
| 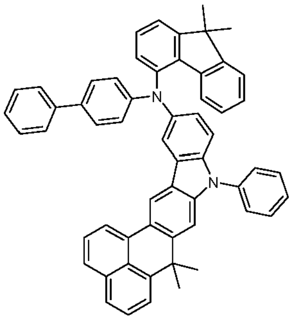 | 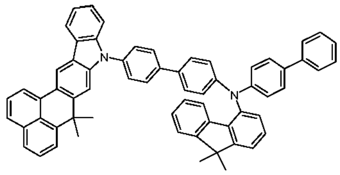 | 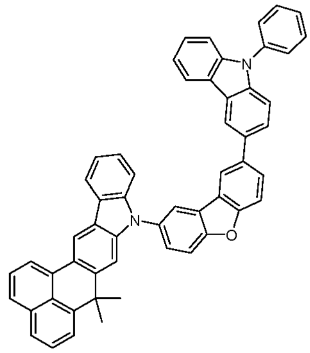 |
| Formel 106 | Formel 107 | Formel 108 |
| 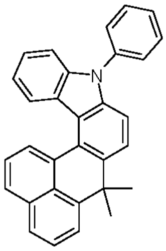 | 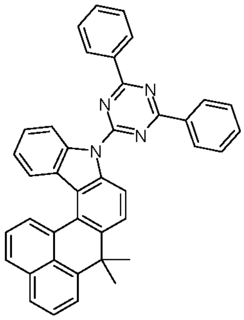 | 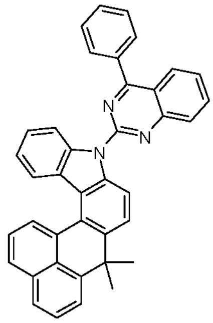 |
| Formel 109 | Formel 110 | Formel 111 |
| 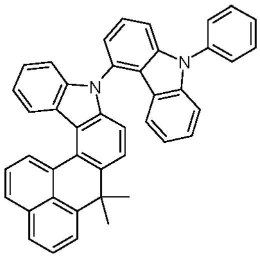 | 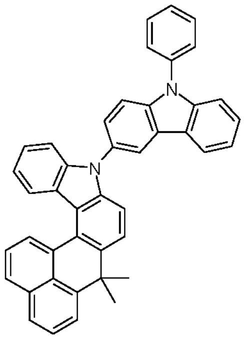 | 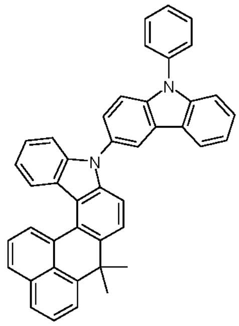 |
| Formel 112 | Formel 113 | Formel 114 |
| 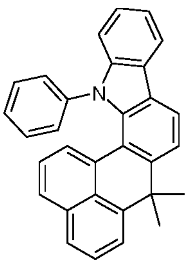 | 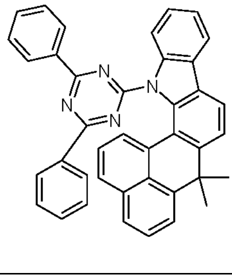 | 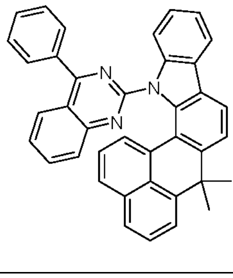 |
| Formel 115 | Formel 116 | Formel 117 |

(fortgesetzt)

| | | |
|---|---|---|
| 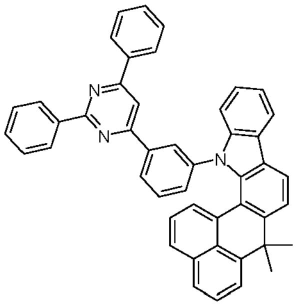 | 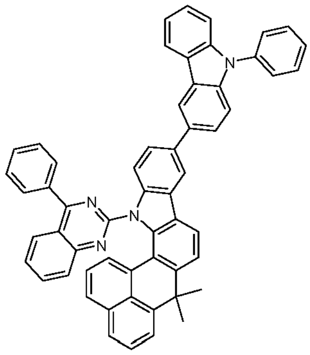 | 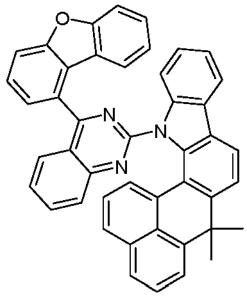 |
| Formel 118 | Formel 119 | Formel 120 |
| 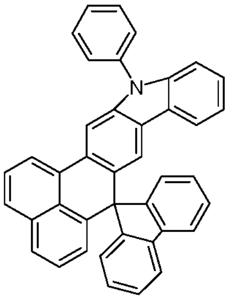 | 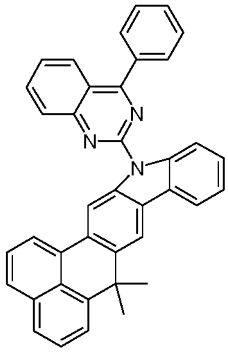 | 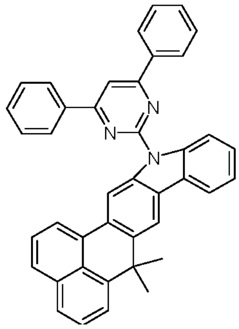 |
| Formel 121 | Formel 122 | Formel 123 |
| 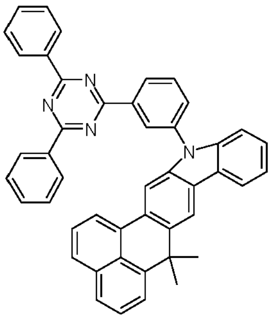 | 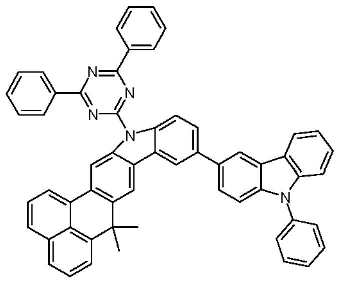 | 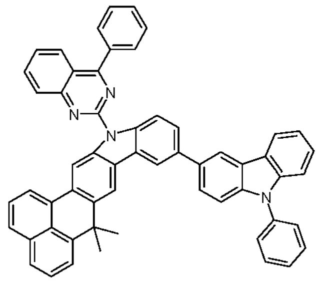 |
| Formel 124 | Formel 125 | Formel 126 |
| 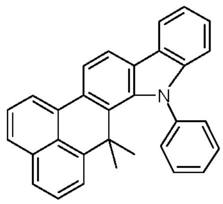 | 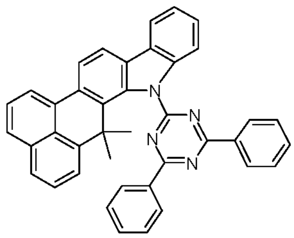 | 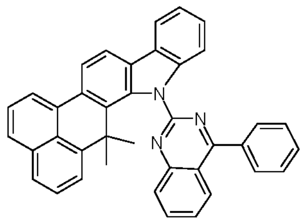 |
| Formel 127 | Formel 128 | Formel 129 |

(fortgesetzt)

| | | |
|---|---|---|
| 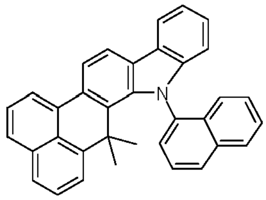 | 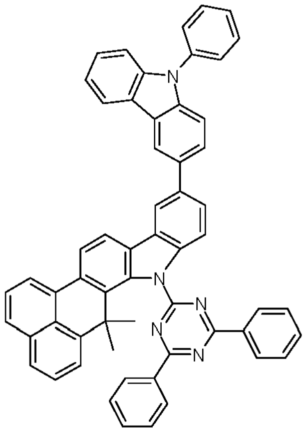 | 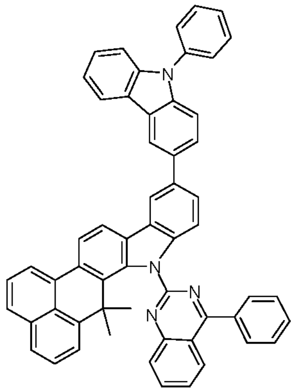 |
| Formel 130 | Formel 131 | Formel 132 |
| 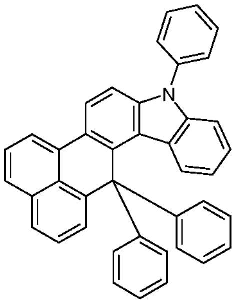 | 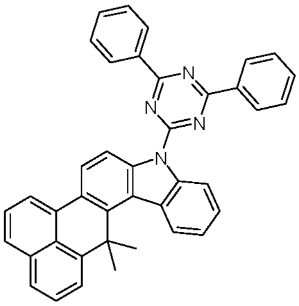 | 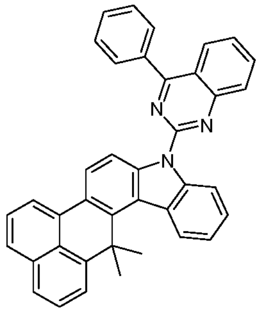 |
| Formel 133 | Formel 134 | Formel 135 |
| 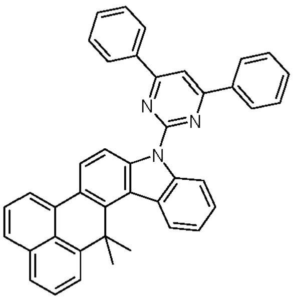 | 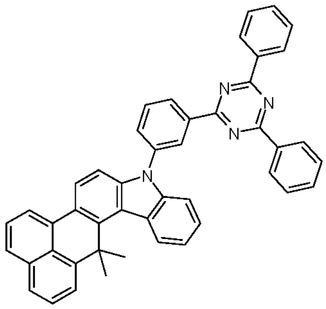 | 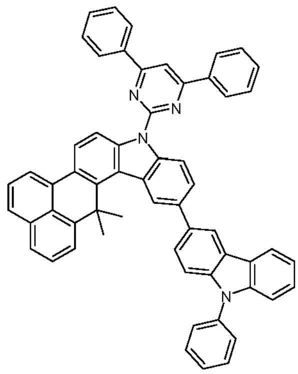 |
| Formel 136 | Formel 137 | Formel 138 |
| 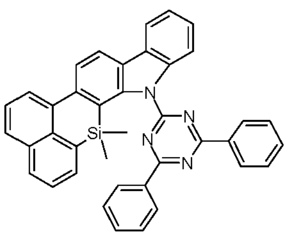 | 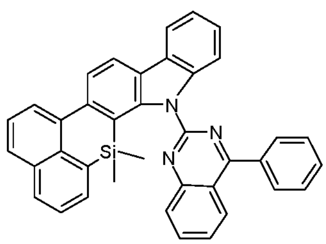 | 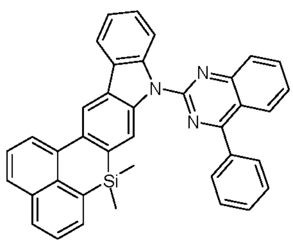 |
| Formel 139 | Formel 140 | Formel 141 |

(fortgesetzt)

| | | |
|---|---|---|
| 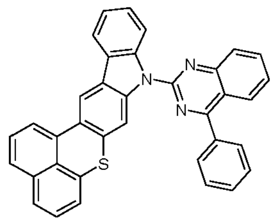 | 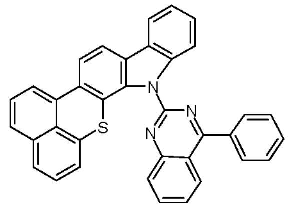 | 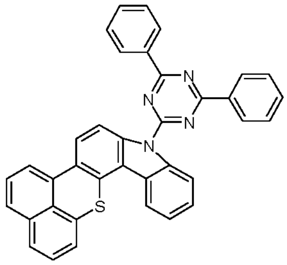 |
| Formel 142 | Formel 143 | Formel 144 |
| 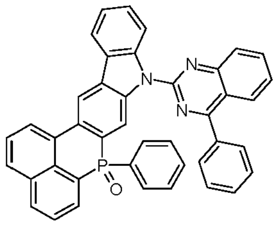 | 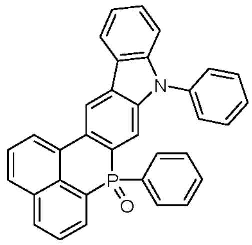 | 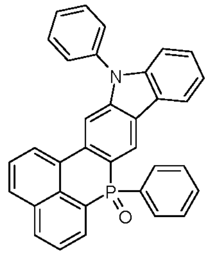 |
| Formel 145 * | Formel 146 * | Formel 147 * |
| 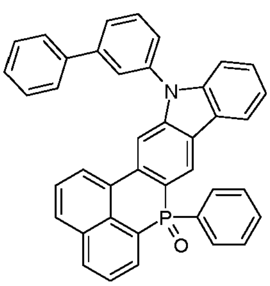 | 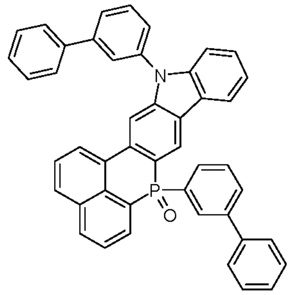 | 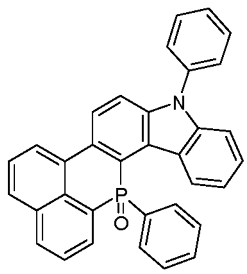 |
| Formel 148 * | Formel 149 * | Formel 150 * |
| 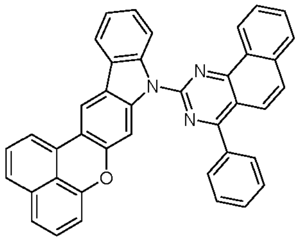 | 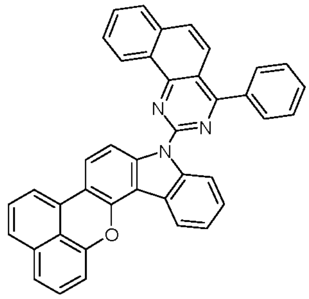 | 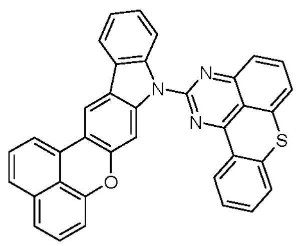 |
| Formel 151 | Formel 152 | Formel 153 |

Bevorzugte Ausführungsformen von erfindungsgemäßen Verbindungen werden in den Beispielen näher ausgeführt, wobei diese Verbindungen allein oder in Kombination mit weiteren für alle erfindungsgemäßen Verwendungszwecke eingesetzt werden können.

Unter der Voraussetzung, dass die in Anspruch 1 genannten Bedingungen eingehalten werden, sind die oben genannten bevorzugten Ausführungsformen beliebig miteinander kombinierbar. In einer besonders bevorzugten Ausführungsform der Erfindung gelten die oben genannten bevorzugten Ausführungsformen gleichzeitig.

Die erfindungsgemäßen Verbindungen sind prinzipiell durch verschiedene Verfahren darstellbar. Es haben sich jedoch die im Folgenden beschriebenen Verfahren als besonders geeignet herausgestellt.

Daher ist ein weiterer Gegenstand der vorliegenden Erfindung ein Verfahren zur Herstellung der Verbindungen, umfassend Strukturen gemäß Formel (I), bei dem in einer Kupplungsreaktion eine Verbindung, umfassend mindestens

eine stickstoffhaltige heterocyclische Gruppe, mit einer Verbindung, umfassend mindestens eine aromatische oder heteroaromatische Gruppe, verbunden wird.

Geeignete Verbindungen mit einer Carbazolgruppe können vielfach kommerziell erhalten werden, wobei die in den Beispielen dargelegten Ausgangsverbindungen durch bekannte Verfahren erhältlich sind, so dass hierauf verwiesen wird.

Diese Verbindungen können durch bekannte Kupplungsreaktionen mit weiteren Arylverbindungen umgesetzt werden, wobei die notwendigen Bedingungen hierfür dem Fachmann bekannt sind und ausführliche Angaben in den Beispielen den Fachmann zur Durchführung dieser Umsetzungen unterstützen.

Besonders geeignete und bevorzugte Kupplungsreaktionen, die alle zu C-C-Verknüpfungen und/oder C-N-Verknüpfungen führen, sind solche gemäß BUCHWALD, SUZUKI, YAMAMOTO, STILLE, HECK, NEGISHI, SONOGASHIRA und HIYAMA. Diese Reaktionen sind weithin bekannt, wobei die Beispiele dem Fachmann weitere Hinweise bereitstellen.

Im allen folgenden Syntheseschemata sind die Verbindungen zur Vereinfachung der Strukturen mit einer geringen Anzahl an Substituenten gezeigt gezeigt. Dies schließt das Vorhandensein von beliebigen weiteren Substituenten in den Verfahren nicht aus.

Eine Umsetzung ergibt sich beispielhaft gemäß folgenden Schemata, ohne dass hierdurch eine Beschränkung erfolgen soll. Die Teilschritte der einzelnen Schemata können hierbei beliebig kombiniert werden.

Schema 1

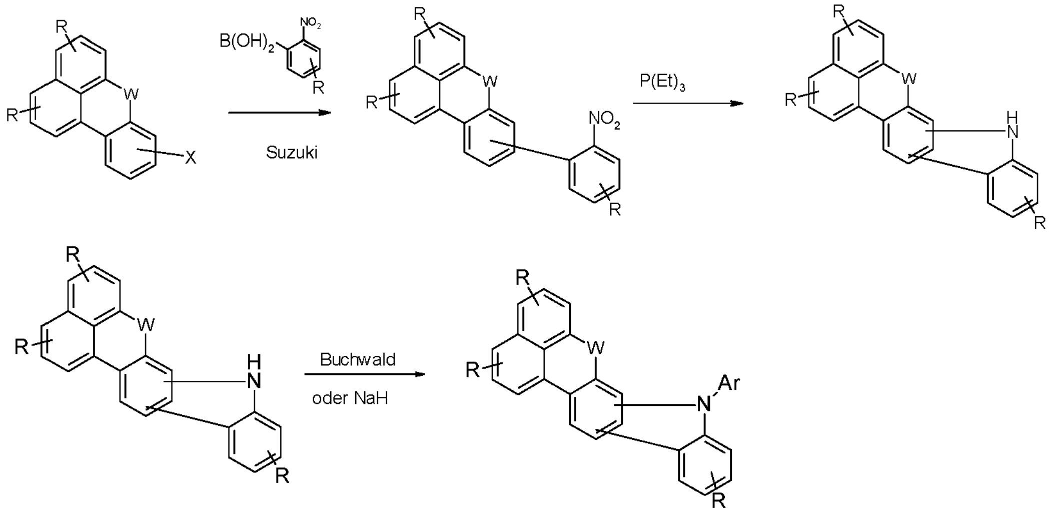


Schema 2

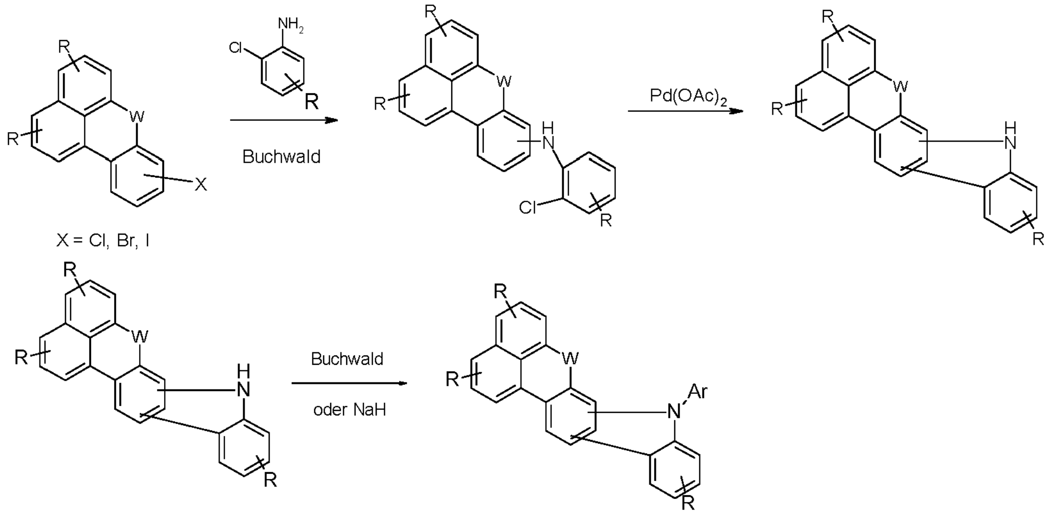


Die Bedeutung der in den Schemata 1 und 2 verwendeten Symbole entspricht im Wesentlichen denen, die für Formel (I) definiert wurden, wobei aus Gründen der Übersichtlichkeit auf eine Nummerierung verzichtet wurde.

Die gezeigten Verfahren zur Synthese der erfindungsgemäßen Verbindungen sind exemplarisch zu verstehen. Der Fachmann kann alternative Synthesewege im Rahmen seines allgemeinen Fachwissens entwickeln.

Die Grundlagen der zuvor dargelegten Herstellungsverfahren sind im Prinzip aus der Literatur für ähnliche Verbindungen bekannt und können vom Fachmann leicht zur Herstellung der erfindungsgemäßen Verbindungen angepasst werden. Weitere Informationen können den Beispielen entnommen werden.

Durch diese Verfahren, gegebenenfalls gefolgt von Aufreinigung, wie z. B. Umkristallisation oder Sublimation, lassen sich die erfindungsgemäßen Verbindungen, umfassend Strukturen gemäß Formel (I) in hoher Reinheit, bevorzugt mehr als 99 % (bestimmt mittels $^1$H-NMR und/oder HPLC) erhalten.

Die erfindungsgemäßen Verbindungen können auch geeignete Substituenten aufweisen, beispielsweise durch längere Alkylgruppen (ca. 4 bis 20 C-Atome), insbesondere verzweigte Alkylgruppen, oder gegebenenfalls substituierte Arylgruppen, beispielsweise Xylyl-, Mesityl- oder verzweigte Terphenyl- oder Quaterphenylgruppen, die eine Löslichkeit in gängigen organischen Lösemitteln bewirken, so dass die Verbindungen beispielsweise in Toluol oder Xylol bei Raumtemperatur in ausreichender Konzentration löslich sind, um die Verbindungen aus Lösung verarbeiten zu können. Diese löslichen Verbindungen eignen sich besonders gut für die Verarbeitung aus Lösung, beispielsweise durch Druckverfahren. Weiterhin ist festzuhalten, dass die erfindungsgemäßen Verbindungen, umfassend mindestens eine Struktur der Formel (I) bereits eine gesteigerte Löslichkeit in diesen Lösungsmitteln besitzen.

Die erfindungsgemäßen Verbindungen können auch mit einem Polymer gemischt werden. Ebenso ist es möglich, diese Verbindungen kovalent in ein Polymer einzubauen. Dies ist insbesondere möglich mit Verbindungen, welche mit reaktiven Abgangsgruppen, wie Brom, Iod, Chlor, Boronsäure oder Boronsäureester, oder mit reaktiven, polymerisierbaren Gruppen, wie Olefinen oder Oxetanen, substituiert sind. Diese können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere enthaltend eine oder mehrere der oben aufgeführten Strukturen der Formel (I) oder erfindungsgemäße Verbindungen, wobei ein oder mehrere Bindungen der erfindungsgemäßen Verbindungen oder der Strukturen der Formel (I) zum Polymer, Oligomer oder Dendrimer vorhanden sind. Je nach Verknüpfung der Strukturen der Formel (I) bzw. der Verbindungen bilden diese daher eine Seitenkette des Oligomers oder Polymers oder sind in der Hauptkette verknüpft. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Copolymere, wobei die Einheiten gemäß Formel (I) bzw. die zuvor und nachfolgend ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/022026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten.

Von besonderem Interesse sind des Weiteren erfindungsgemäße Verbindungen, die sich durch eine hohe Glasübergangstemperatur auszeichnen. In diesem Zusammenhang sind insbesondere erfindungsgemäße Verbindungen umfassend Strukturen der allgemeinen Formel (I) bzw. die zuvor und nachfolgend ausgeführten bevorzugten Ausführungsformen bevorzugt, die eine Glasübergangstemperatur von mindestens 70 °C, besonders bevorzugt von mindestens 110 °C, ganz besonders bevorzugt von mindestens 125 °C und insbesondere bevorzugt von mindestens 150 °C aufweisen, bestimmt nach DIN 51005 (Version 2005-08).

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol,

Cyclohexanon, Cyclohexylbenzol, Decalin, Dodecylbenzol, Ethylbenzoat, Indan, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, Hexamethylindan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthyl-isovalerat, Cyclohexylhexanoat oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung, beispielsweise ein fluoreszierender Dotand, ein phosphoreszierender Dotand oder eine Verbindung, die TADF (thermally activated delayed fluorescence) zeigt, insbesondere ein phosphoreszierender Dotand, und/oder ein weiteres Matrixmaterial. Diese weitere Verbindung kann auch polymer sein.

Nochmals ein weiterer Gegenstand der vorliegenden Erfindung ist daher eine Zusammensetzung enthaltend eine erfindungsgemäße Verbindung und wenigstens ein weiteres organisch funktionelles Material. Funktionelle Materialen sind generell die organischen oder anorganischen Materialien, welche zwischen Anode und Kathode eingebracht sind. Vorzugsweise ist das organisch funktionelle Material ausgewählt aus der Gruppe bestehend aus fluoreszierenden Emittern, phosphoreszierenden Emittern, Emittern, die TADF (thermally activated delayed fluorescence) zeigen, Host-Materialien, Elektronentransportmaterialien, Elektroneninjektionsmaterialien, Lochleitermaterialien, Lochinjektionsmaterialien, Elektronenblockiermaterialien, Lochblockiermaterialien, Wide-Band-Gap-Materialien und n-Dotanden.

Die vorliegenden Erfindung betrifft daher auch eine Zusammensetzung enthaltend wenigstens eine Verbindung umfassend Strukuren gemäß Formel (I) bzw. die zuvor und nachfolgend ausgeführten bevorzugten Ausführungsformen sowie wenigstens ein weiteres Matrixmaterial. Gemäß einem besonderen Aspekt der vorliegenden Erfindung weist das weitere Matrixmaterial lochtransportierende Eigenschaften auf.

Ein weiterer Gegenstand der vorliegenden Erfindung stellt eine Zusammensetzung dar, enthaltend wenigstens eine Verbindung, umfassend mindestens eine Struktur gemäß Formel (I) bzw. die zuvor und nachfolgend ausgeführten bevorzugten Ausführungsformen sowie wenigstens ein Wide-Band-Gap-Material, wobei unter Wide-Band-Gap-Material ein Material im Sinne der Offenbarung von US 7,294,849 verstanden wird. Diese Systeme zeigen besondere vorteilhafte Leistungsdaten in elektrolumineszierenden Vorrichtungen.

Vorzugsweise kann die zusätzliche Verbindung eine Bandlücke (band gap) von 2,5 eV oder mehr, bevorzugt 3,0 eV oder mehr, ganz bevorzugt von 3,5 eV oder mehr aufweisen. Die Bandlücke kann unter anderem durch die Energieniveaus des highest occupied molecular orbital (HOMO) und des lowest unoccupied molecular orbital (LUMO) berechnet werden. Molekülorbitale, insbesondere auch das highest occupied molecular orbital (HOMO) und das lowest unoccupied molecular orbital (LUMO), deren Energieniveaus sowie die Energie des niedrigsten Triplettzustands $T_1$ bzw. des niedrigsten angeregten Singulettzustands $S_1$ der Materialien werden über quantenchemische Rechnungen bestimmt. Zur Berechnung organischer Substanzen ohne Metalle wird zuerst eine Geometrieoptimierung mit der Methode "Ground State/Semi-empirical/Default Spin/AM1/Charge 0/Spin Singlet" durchgeführt. Im Anschluss erfolgt auf Grundlage der optimierten Geometrie eine Energierechnung. Hierbei wird die Methode "TD-SCF/DFT/Default Spin/B3PW91" mit dem Basissatz "6-31 G(d)" verwendet (Charge 0, Spin Singlet). Für metallhaltige Verbindungen wird die Geometrie über die Methode "Ground State/ Hartree-Fock/Default Spin/LanL2MB/Charge 0/Spin Singlet" optimiert. Die Energierechnung erfolgt analog zu der oben beschriebenen Methode für die organischen Substanzen mit dem Unterschied, dass für das Metallatom der Basissatz "LanL2DZ" und für die Liganden der Basissatz "6-31 G(d)" verwendet wird. Aus der Energierechnung erhält man das HOMO-Energieniveau HEh bzw. LUMO-Energieniveau LEh in Hartree-Einheiten. Daraus werden die anhand von Cyclovoltammetriemessungen kalibrierten HOMO- und LUMO-Energieniveaus in Elektronenvolt wie folgt bestimmt:

$$HOMO(eV) = ((HEh*27.212)-0.9899)/1.1206$$

$$LUMO(eV) = ((LEh*27.212)-2.0041)/1.385$$

Diese Werte sind im Sinne dieser Anmeldung als HOMO- bzw. LUMO-Energieniveaus der Materialien anzusehen.

Der niedrigste Triplettzustand $T_1$ ist definiert als die Energie des Triplettzustands mit der niedrigsten Energie, der sich aus der beschriebenen quantenchemischen Rechnung ergibt.

Der niedrigste angeregte Singulettzustand $S_1$ ist definiert als die Energie des angeregten Singulettzustands mit der niedrigsten Energie, der sich aus der beschriebenen quantenchemischen Rechnung ergibt.

Die hierin beschriebene Methode ist unabhängig von dem verwendeten Softwarepaket und liefert immer dieselben Ergebnisse. Beispiele oft benutzter Programme für diesen Zweck sind "Gaussian09W" (Gaussian Inc.) und Q-Chem 4.1 (Q-Chem, Inc.).

Die vorliegende Erfindung betrifft auch eine Zusammensetzung umfassend wenigstens eine Verbindung umfassend Strukuren gemäß Formel (I) bzw. die zuvor und nachfolgend ausgeführten bevorzugten Ausführungsformen sowie wenigstens einen phosphoreszierende Emitter, wobei unter dem Begriff phosphoreszierende Emitter auch phosphoreszierende Dotanden verstanden werden.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Bevorzugte phosphoreszierende Dotanden zur Verwendung in Matrix-Systemen, vorzugsweise Mixed-Matrix-Systemen sind die im Folgenden angebenen bevorzugten phosphoreszierenden Dotanden.

Vom Begriff phosphoreszierende Dotanden sind typischerweise Verbindungen umfasst, bei denen die Lichtemission durch einen spin-verbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden Erfindung werden alle lumineszierenden Verbindungen, die die oben genannten Metalle enthalten, als phosphoreszierende Verbindungen angesehen.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439 und den noch nicht offen gelegten Anmeldungen EP 16179378.1 und EP 16186313.9 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Explizite Beispiele für phosphoreszierende Dotanden sind in der folgenden Tabelle aufgeführt.

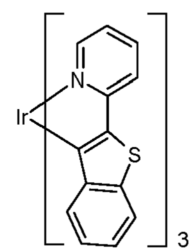
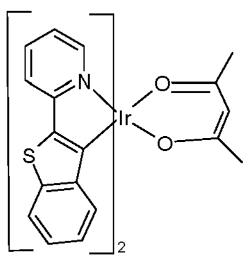
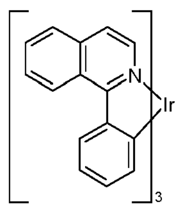
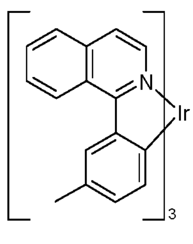
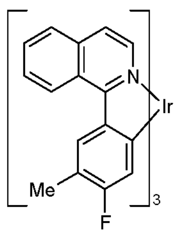
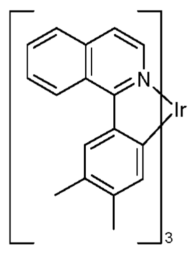

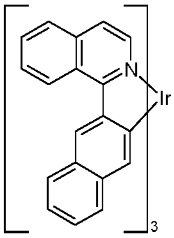
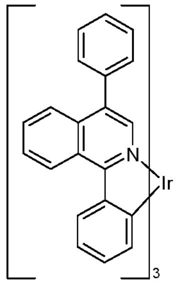
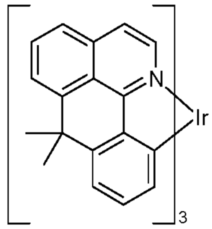

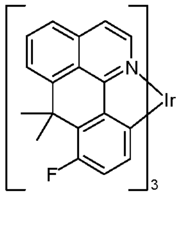
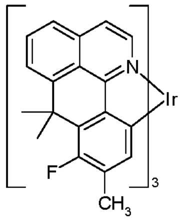
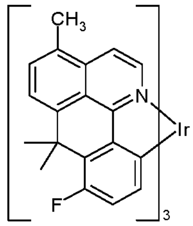

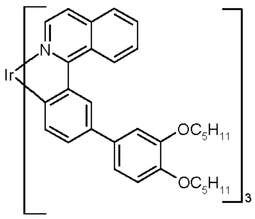
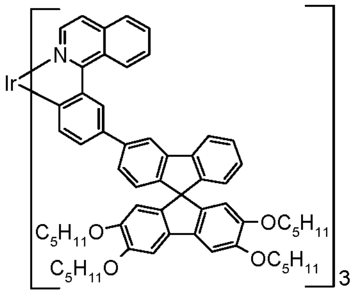
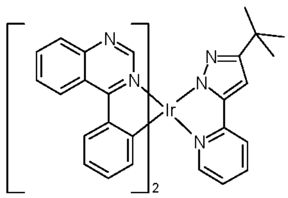

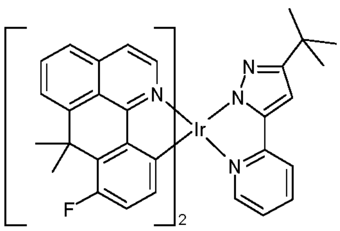
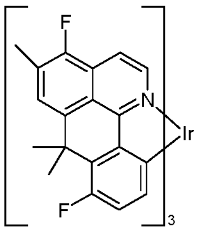
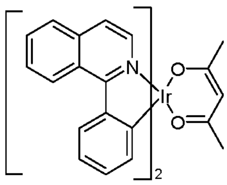

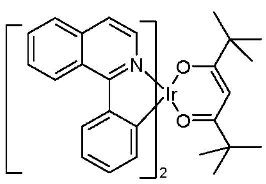
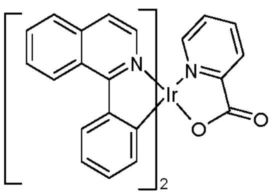
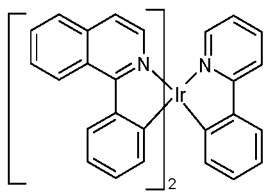

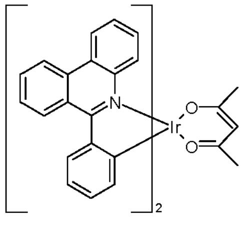
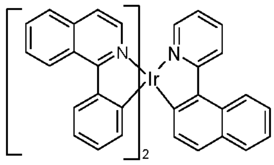
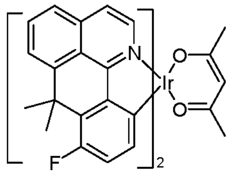

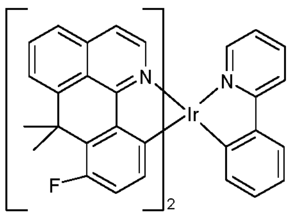
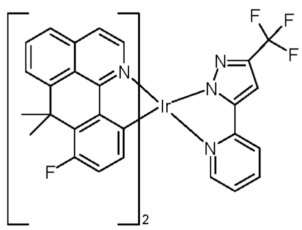
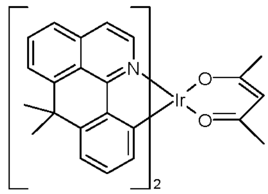

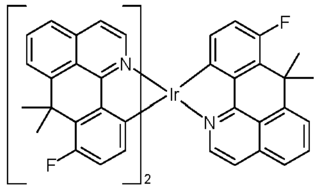
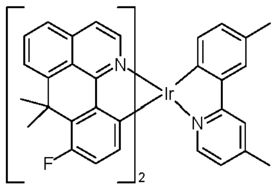
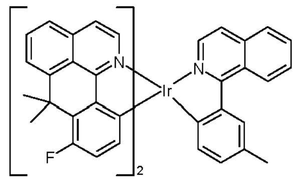

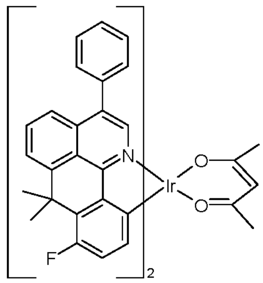
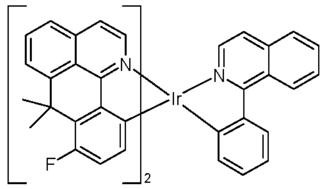
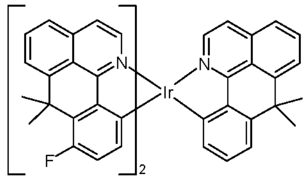

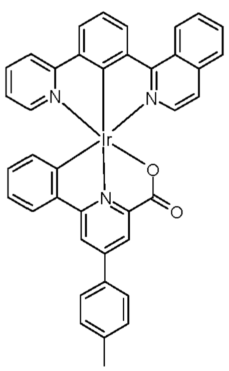
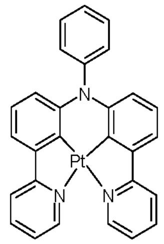
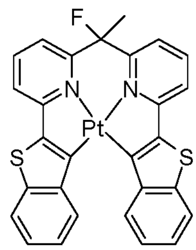

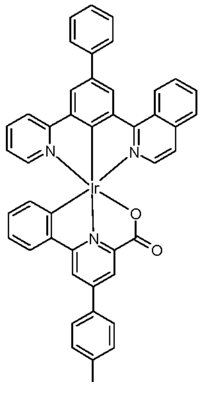
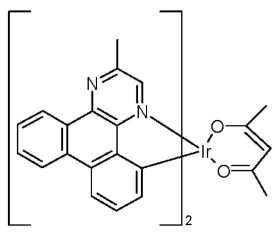
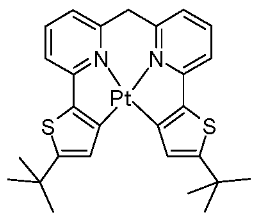

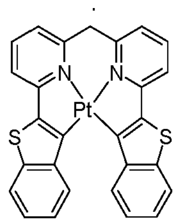

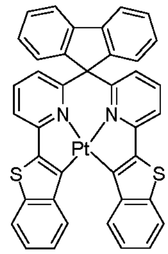

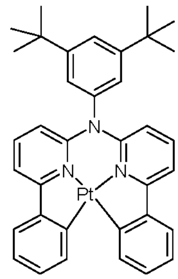

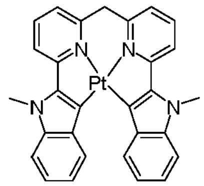

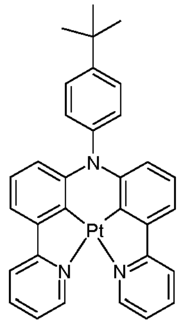

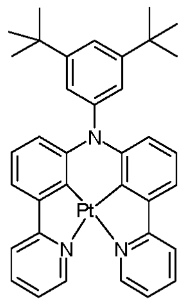

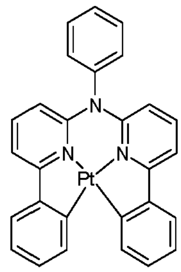

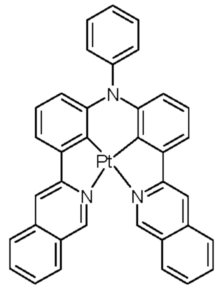

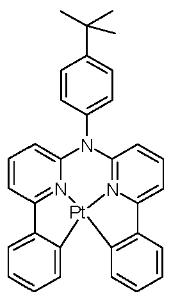

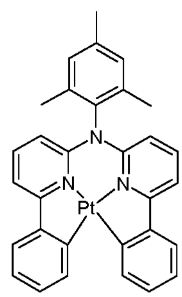

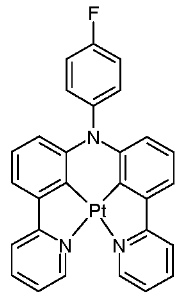

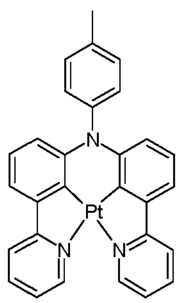

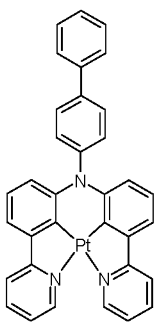

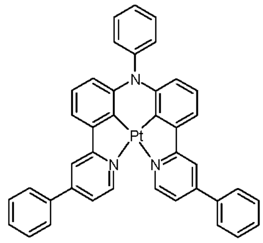

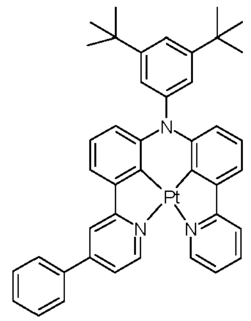
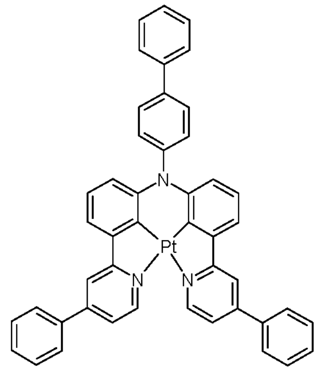
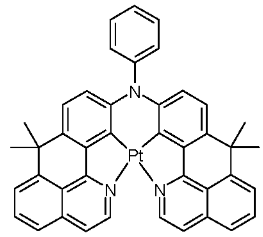

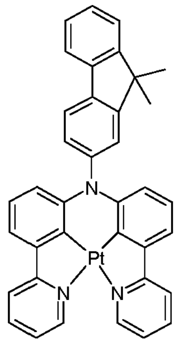
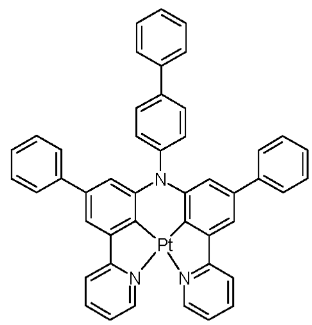
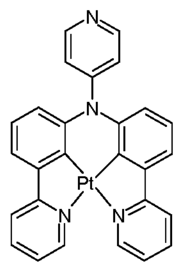

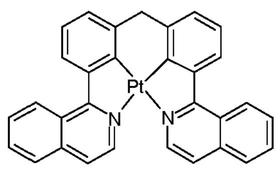
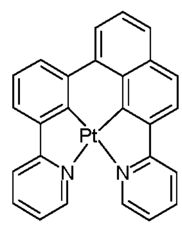
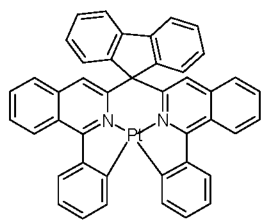

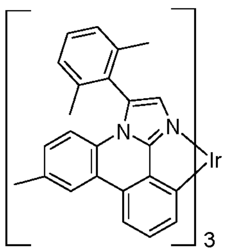
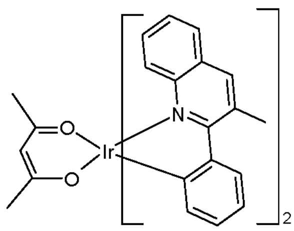
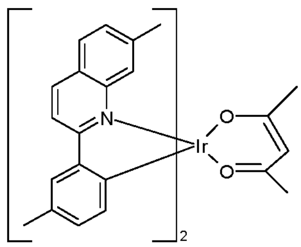

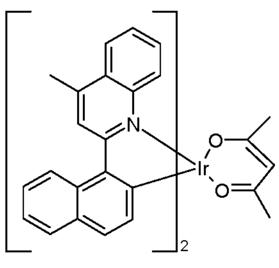
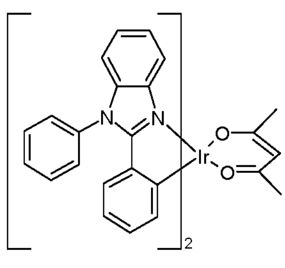
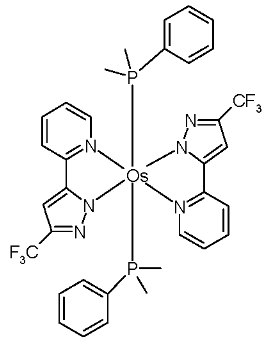

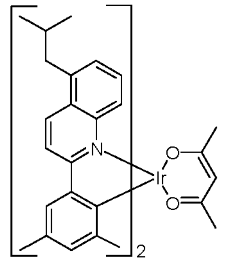
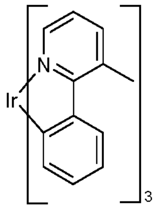
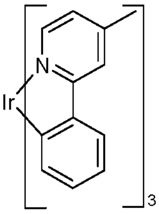

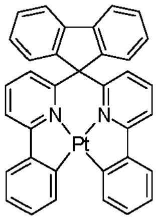
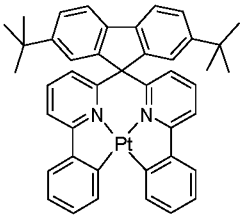
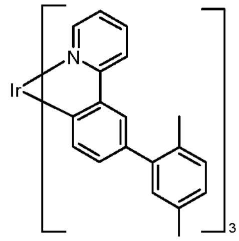

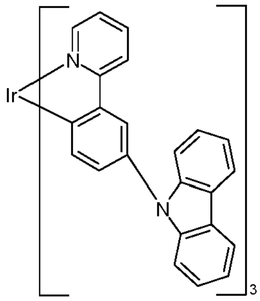
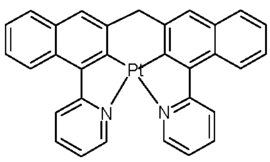
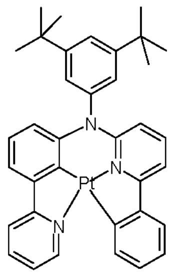

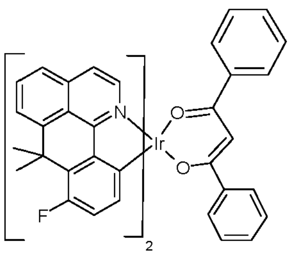
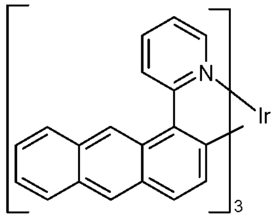
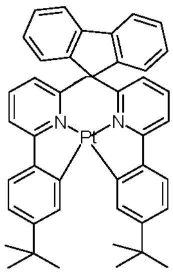

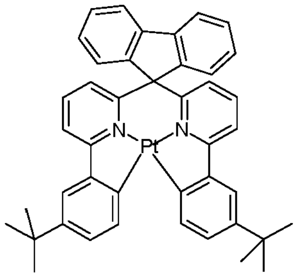
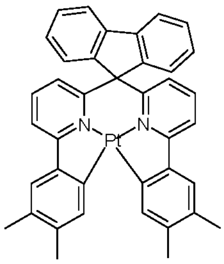
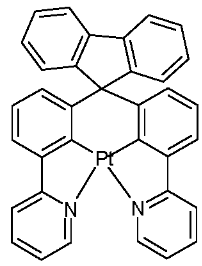

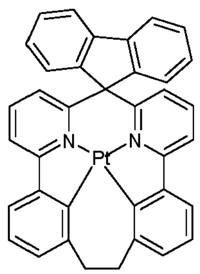
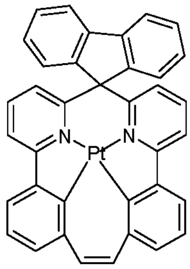
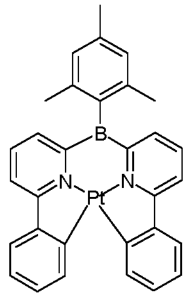

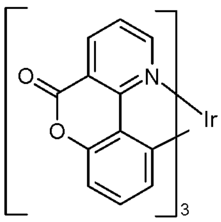
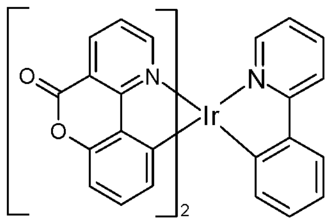
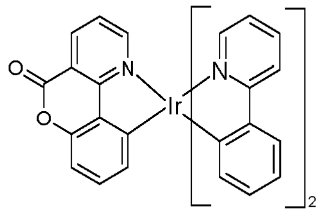

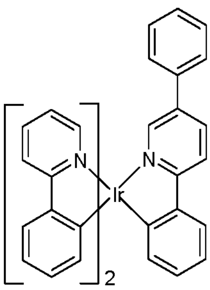
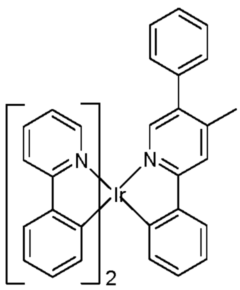
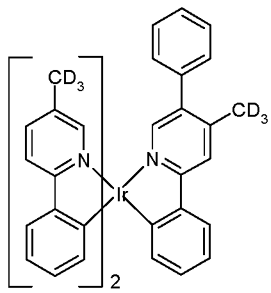

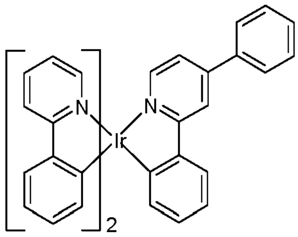
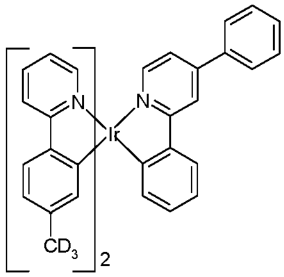
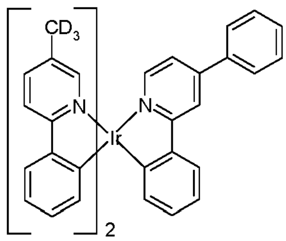

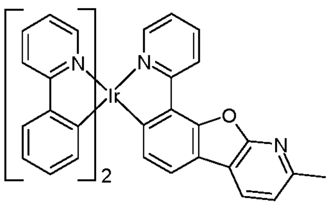
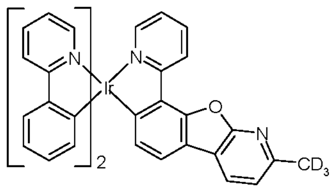
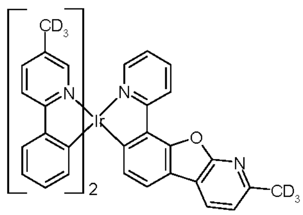

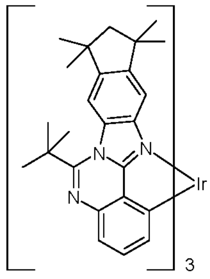
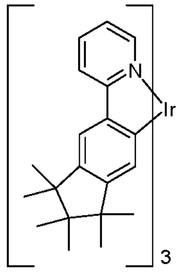
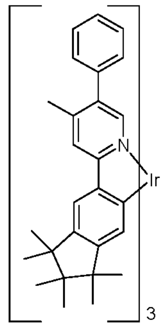

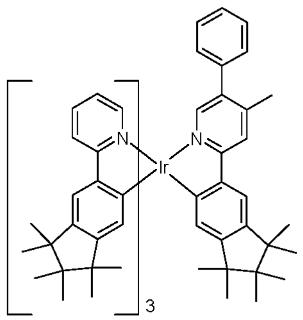
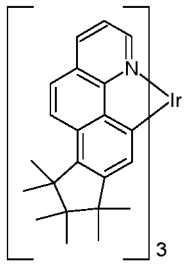
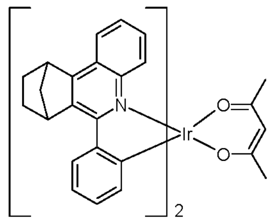

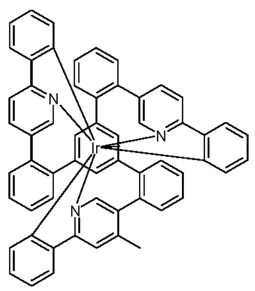
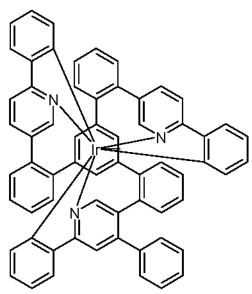
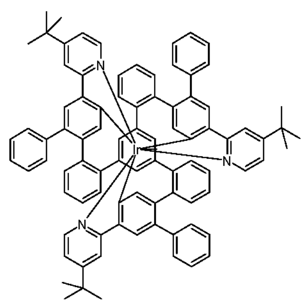

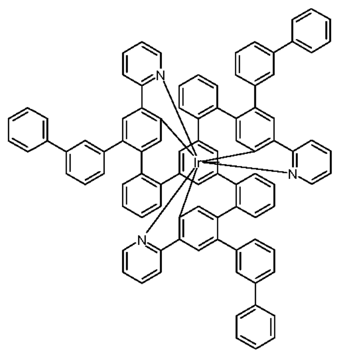
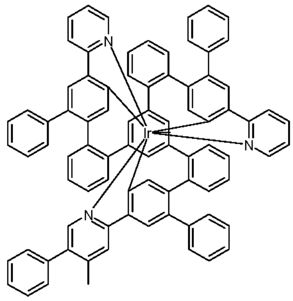
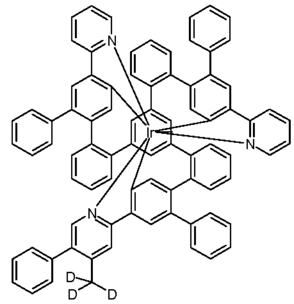

Die oben beschriebenen Verbindung, umfassend Strukturen der Formel (I) bzw. die oben aufgeführten bevorzugten Ausführungsformen, können in einer elektronischen Vorrichtung bevorzugt als aktive Komponente verwendet werden. Unter einer elektronischen Vorrichtung wird eine Vorrichtung verstanden, welche Anode, Kathode und mindestens eine zwischen Anode und Kathode liegende Schicht enthält, wobei diese Schicht mindestens eine organische bzw. metallorganische Verbindung enthält. Die erfindungsgemäße elektronische Vorrichtung enthält also Anode, Kathode und mindestens eine dazwischen liegende Schicht, welche mindestens eine Verbindung, umfassend Strukturen der Formel (I), enthält. Dabei sind bevorzugte elektronische Vorrichtungen ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), organischen elektrischen Sensoren, lichtemittierenden elektrochemischen Zellen (LECs), organischen Laserdioden (O-Laser) und "organic plasmon emitting devices" (D. M. Koller et al., Nature Photonics 2008, 1-4), bevorzugt organischen Elektrolumineszenzvorrichtungen (OLEDs, PLEDs), insbesondere phosphoreszierenden OLEDs,enthaltend in mindestens einer Schicht mindestens eine Verbindung, umfassend Strukturen der Formel (I). Besonders bevorzugt sind organische Elektrolumineszenzvorrichtungen. Aktive Komponenten sind generell die organischen oder anorganischen Materialien, welche zwischen Anode und Kathode eingebracht sind, beispielsweise Ladungsinjektions-, Ladungstransport- oder Ladungsblockiermaterialien, insbesondere aber Emissionsmaterialien und Matrixmaterialien.

Eine bevorzugte Ausführungsform der Erfindung sind organische Elektrolumineszenzvorrichtungen. Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten, Ladungserzeugungsschichten und/oder organische oder anorganische p/n-Übergänge. Dabei ist es möglich, dass eine oder mehrere Lochtransportschichten p-dotiert sind, beispielsweise mit Metalloxiden, wie MoOs oder WOs oder mit (per)fluorierten elektronenarmen Aromaten, und/oder dass eine oder mehrere Elektronentransportschichten n-dotiert sind. Ebenso können zwischen zwei emittierende Schichten Interlayers eingebracht sein, welche beispielsweise eine Exzitonenblockierende Funktion aufweisen und/oder die Ladungsbalance in der Elektrolumineszenzvorrichtung steuern. Es sei aber darauf hingewiesen, dass nicht notwendigerweise jede dieser Schichten vorhanden sein muss.

Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B. WO 2005/011013) bzw. Systeme, welche mehr als drei emittierende Schichten aufweisen. Weiterhin bevorzugt sind auch Tandem-OLEDs. Es kann sich auch um ein Hybrid-System handeln, wobei eine oder mehrere Schichten fluoreszieren und eine oder mehrere andere Schichten phosphoreszieren.

In einer bevorzugten Ausführungsform der Erfindung enthält die organische Elektrolumineszenzvorrichtung die efindungsgemäße Verbindung umfassend Strukturen gemäß Formel (I) bzw. die oben aufgeführten bevorzugten Ausführungsformen als Matrixmaterial, vorzugsweise als elektronenleitendes Matrixmaterial in einer oder mehreren emittierenden Schichten, bevorzugt in Kombination mit einem weiteren Matrixmaterial, vorzugsweise einem lochleitenden Matrixmaterial. In einer weiteren bevorzugten Ausführungsform der Erfindung ist das weitere Matrixmaterial eine elektronentransportierende Verbindung. In nochmals einer weiteren bevorzugten Ausführungsform ist das weitere Matrixmaterial eine Verbindung mit großem Bandabstand, das nicht oder nicht in wesentlichem Umfang am Loch- und Elektronentransport in der Schicht beteiligt ist. Eine emittierende Schicht umfasst mindestens eine emittierende Verbindung.

Geeignete Matrixmaterialien, welche in Kombination mit den Verbindungen gemäß Formel (I) bzw. gemäß den bevorzugten Ausführungsformen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, insbesondere Monoamine, z. B. gemäß WO 2014/015935, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109 und WO 2011/000455, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte CarbazolDerivate, z. B. gemäß US 2009/0136779, WO 2010/050778, WO 2011/042107, WO 2011/088877 oder WO 2012/143080, Triphenylenderivate, z. B. gemäß WO 2012/048781, Lactame, z. B. gemäß WO 2011/116865, WO 2011/137951 oder WO 2013/064206, 4-SpirocarbazolDerivate, z. B. gemäß WO 2014/094963 oder WO 2015/192939, oder Dibenzofuran-Derivate, z. B. gemäß WO 2015/169412, WO 2016/015810, WO 2016/023608, WO 2017/148564 oder WO 2017/148565. Ebenso kann ein weiterer phosphoreszierender Emitter, welcher kürzerwellig als der eigentliche Emitter emittiert, als Co-Host in der Mischung vorhanden sein.

Bevorzugte Co-Host-Materialien sind Triarylaminderivate, insbesondere Monoamine, Indenocarbazolderivate, 4-Spirocarbazolderivate, Lactame und Carbazolderivate.

Bevorzugte Triarylaminderivate, die als Co-Host-Materialien zusammen mit den erfindungsgemäßen Verbindungen eingesetzt werden, sind ausgewählt aus den Verbindungen der folgenden Formel (TA-1),

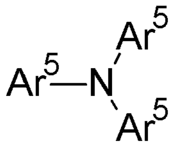

Formel (TA-1)

wobei $Ar^5$ gleich oder verschieden bei jedem Auftreten ein aromatisches oder heteroaromatisches Ringsystem mit 6 bis 40 C-Atomen, das jeweils mit einem oder mehreren Resten $R^2$ substituiert sein kann, darstellt, wobei optional zwei oder mehr benachbarte Substituenten $R^2$ ein mono- oder polycyclisches, aliphatisches Ringsystem bilden können, das mit einem oder mehreren Resten $R^3$ substituiert sein kann, wobei das Symbol $R^2$ die zuvor, insbesondere für Formel (I) genannte Bedeutung aufweist. Vorzugsweise stellt $Ar^5$ gleich oder verschieden bei jedem Auftreten eine Aryl- oder Heteroarylgruppe mit 5 bis 24, vorzugsweise 5 bis 12 aromatischen Ringatomen dar, die jeweils mit einem oder mehreren Resten $R^2$ substituiert sein kann, vorzugsweise jedoch unsubstituiert ist.

Beispiele für geeignete Gruppen $Ar^5$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind.

Bevorzugt sind die Gruppen $Ar^5$ gleich oder verschieden bei jedem Auftreten ausgewählt aus den oben genannten Gruppen $R^1$-1 bis $R^1$-92, besonders bevorzugt $R^1$-1 bis $R^1$-54.

In einer bevorzugten Ausführungsform der Verbindungen der Formel (TA-1) ist mindestens eine Gruppe $Ar^5$ ausgewählt aus einer Biphenylgruppe, wobei es sich um eine ortho-, meta- oder para-Biphenylgruppe handeln kann. In einer weiteren bevorzugten Ausführungsform der Verbindungen der Formel (TA-1) ist mindestens eine Gruppe $Ar^5$ ausgewählt aus einer Fluorengruppe oder Spirobifluorengruppe, wobei diese Gruppen jeweils in 1-, 2-, 3- oder 4-Position an das Stickstoffatom gebunden sein können. In nochmals einer weiteren bevorzugten Ausführungsform der Verbindungen der Formel (TA-1) ist mindestens eine Gruppe $Ar^5$ ausgewählt aus einer Phenylen- oder Biphenylgruppe, wobei es sich um eine ortho-, meta- oder para-verknüpfte Gruppe handelt, die mit einer Dibenzofurangruppe, einer Dibenzothiophengruppe oder einer Carbazolgruppe, insbesondere einer Dibenzofurangruppe, substituiert ist, wobei die Dibenzofuran- bzw. Dibenzothiophengruppe über die 1-, 2-, 3- oder 4-Position mit der Phenylen- bzw. Biphenylgruppe verknüpft ist und wobei die Carbazolgruppe über die 1-, 2-, 3- oder 4-Position oder über das Stickstoffatom mit der Phenylen- bzw. Biphenylgruppe verknüpft ist.

In einer besonders bevorzugten Ausführungsform der Verbindungen der Formel (TA-1) ist eine Gruppe $Ar^5$ ausgewählt aus einer Fluoren- oder Spirobifluorengruppe, insbesondere einer 4-Fluoren- bzw. 4-Spirobifluorengruppe, und eine Gruppe $Ar^5$ ist ausgewählt aus einer Biphenylgruppe, insbesondere einer para-Biphenylgruppe, oder einer Fluorengruppe, insbesondere einer 2-Fluorengruppe, und die dritte Gruppe $Ar^5$ ist ausgewählt aus einer para-Phenylengruppe oder einer para-Biphenylgruppe, die mit einer Dibenzofurangruppe, insbesondere einer 4-Dibenzofurangruppe, oder einer Carbazolgruppe, insbesondere einer N-Carbazolgruppe oder einer 3-Carbazolgruppe, substituiert ist.

Bevorzugte Indenocarbazolderivate, die als Co-Host-Materialien zusammen mit den erfindungsgemäßen Verbindungen eingesetzt werden, sind ausgewählt aus den Verbindungen der folgenden Formel (TA-2),

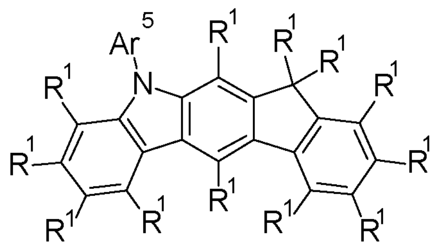

Formel (TA-2)

wobei $Ar^5$ und $R^1$ die oben insbesondere für Formeln (I) und/oder (TA-1) aufgeführten Bedeutungen aufweisen. Dabei sind bevorzugte Ausführungsformen der Gruppe $Ar^5$ die oben aufgeführten Strukturen $R^1$-1 bis $R^1$-92, besonders bevorzugt $R^1$-1 bis $R^1$-54.

Eine bevorzugte Ausführungsform der Verbindungen der Formel (TA-2) sind die Verbindungen der folgenden Formel (TA-2a),

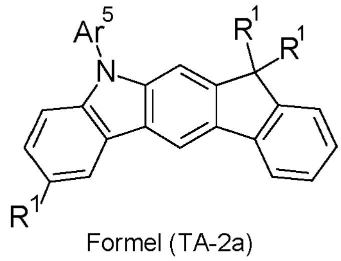

Formel (TA-2a)

wobei $Ar^5$ und $R^1$ die oben, insbesondere für Formeln (I) und/oder (TA-1) aufgeführten Bedeutungen aufweisen. Dabei stehen die beiden Gruppen $R^1$, die an das Indenokohlenstoffatom gebunden sind, bevorzugt gleich oder verschieden für eine Alkylgruppe mit 1 bis 4 C-Atomen, insbesondere für Methylgruppen, oder für ein aromatisches Ringsystem mit 6 bis 12 C-Atomen, insbesondere für Phenylgruppen. Besonders bevorzugt stehen die beiden Gruppen $R^1$, die an das Indenokohlenstoffatom gebunden sind, für Methylgruppen. Weiterhin bevorzugt steht der Substituent $R^1$, der in Formel (TA-2a) an den Indenocarbazolgrundkörper gebunden ist, für H oder für eine Carbazolgruppe, die über die 1-, 2-, 3- oder 4-Position oder über das N-Atom an den Indenocarbazolgrundkörper gebunden sein kann, insbesondere über die 3-Position.

Bevorzugte 4-Spirocarbazolderivate, die als Co-Host-Materialien zusammen mit den erfindungsgemäßen Verbindungen eingesetzt werden, sind ausgewählt aus den Verbindungen der folgenden Formel (TA-3),

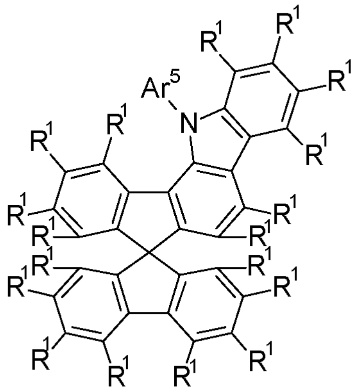

Formel (TA-3)

wobei $Ar^5$ und $R^1$ die oben, insbesondere für Formeln (I) und/oder (TA-1), aufgeführten Bedeutungen aufweisen. Dabei sind bevorzugte Ausführungsformen der Gruppe $Ar^5$ die oben aufgeführten Strukturen $R^1$-1 bis $R^1$-92, besonders bevorzugt $R^1$-1 bis $R^1$-54.

Eine bevorzugte Ausführungsform der Verbindungen der Formel (TA-3) sind die Verbindungen der folgenden Formel (TA-3a),

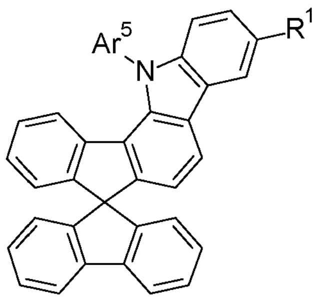

Formel (TA-3a)

wobei $Ar^5$ und $R^1$ die oben, insbesondere für Formeln (I) und/oder (TA-1), aufgeführten Bedeutungen Bedeutungen aufweisen. Dabei sind bevorzugte Ausführungsformen der Gruppe $Ar^5$ die oben aufgeführten Strukturen $R^1$-1 bis $R^1$-

92, besonders bevorzugt $R^1$-1 bis $R^1$-54.

Bevorzugte Lactame, die als Co-Host-Materialien zusammen mit den erfindungsgemäßen Verbindungen eingesetzt werden, sind ausgewählt aus den Verbindungen der folgenden Formel (LAC-1),

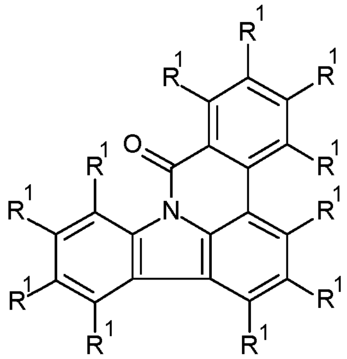

Formel (LAC-1)

wobei $R^1$ die oben, insbesondere für Formeln (I) aufgeführte Bedeutung aufweist.

Eine bevorzugte Ausführungsform der Verbindungen der Formel (LAC-1) sind die Verbindungen der folgenden Formel (LAC-1a),

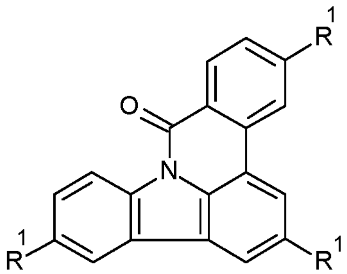

Formel (LAC-1a)

wobei $R^1$ die oben, insbesondere für Formel (I) genannte Bedeutung aufweist. Dabei steht $R^1$ bevorzugt gleich oder verschieden bei jedem Auftreten für H oder ein aromatisches oder heteroaromatisches Ringsystem mit 5 bis 40 aromatischen Ringatomen, das mit einem oder mehreren Resten $R^2$ substituiert sein kann, wobei $R^2$ die zuvor, insbesondere für Formel (I) genannte Bedeutung aufweisen kann. Ganz besonders bevorzugt sind die Substituenten $R^1$ ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nichtaromatischen Resten $R^2$ substituiert sein kann, bevorzugt aber unsubstituiert ist. Beispiele für geeignete Substituenten $R^1$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3-oder 4-Dibenzothienyl und 1-, 2-, 3- oder 4-Carbazolyl, die jeweils durch einen oder mehrere Reste $R^2$ substituiert sein können, bevorzugt aber unsubstituiert sind. Dabei sind geeignete Strukturen $R^1$ die gleichen Strukturen, wie sie zuvor für R-1 bis R-79 abgebildet sind, besonders bevorzugt $R^1$-1 bis $R^1$-51.

Es kann auch bevorzugt sein, mehrere verschiedene Matrixmaterialien als Mischung einzusetzen, insbesondere mindestens ein elektronenleitendes Matrixmaterial und mindestens ein lochleitendes Matrixmaterial. Ebenso bevorzugt ist die Verwendung einer Mischung aus einem ladungstransportierenden Matrixmaterial und einem elektrisch inerten Matrixmaterial, welches nicht bzw. nicht in wesentlichem Maße am Ladungstransport beteiligt ist, wie z. B. in WO 2010/108579 beschrieben.

Weiterhin bevorzugt ist es, eine Mischung aus zwei oder mehr Triplett-Emittern zusammen mit einer Matrix einzusetzen. Dabei dient der Triplett-Emitter mit dem kürzerwelligen Emissionsspektrum als Co-Matrix für den Triplett-Emitter mit dem längerwelligen Emissionsspektrum.

Besonders bevorzugt kann eine erfindungsgemäße Verbindung umfassend Strukuren gemäß Formel (I) in einer bevorzugten Ausführungsform als Matrixmaterial in einer Emissionsschicht einer organischen elektronischen Vorrichtung, insbesondere in einer organischen elektrolumineszierenden Vorrichtung, beispielsweise in einer OLED oder OLEC, eingesetzt werden. Dabei ist das Matrixmaterial enthaltend Verbindung umfassend Strukuren gemäß Formel (I) bzw. die zuvor und nachfolgend ausgeführten bevorzugten Ausführungsformen in der elektronischen Vorrichtung in Kombination mit einem oder mehreren Dotanden, vorzugsweise phosphoreszierenden Dotanden, vorhanden.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindung umfassend Strukuren gemäß Formel (I) bzw. die zuvor und nachfolgend ausgeführten bevorzugten Ausführungsformen als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt.

Genauere Angaben zu Mixed-Matrix-Systemen sind unter anderem in der Anmeldung WO 2010/108579 enthalten.

Ferner ist eine elektronische Vorrichtung, vorzugsweise eine organische Elektrolumineszenzvorrichtung Gegenstand der vorliegenden Erfindung, die eine oder mehrere erfindungsgemäße Verbindungen und/oder mindestens ein erfindungsgemäßes Oligomer, Polymer oder Dendrimer in einer oder mehreren elektronenleitenden Schichten umfasst, als elektronenleitende Verbindung.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Mg/Ag, Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $Cs_2CO_3$, etc.). Ebenso kommen hierfür organische Alkalimetallkomplexe in Frage, z. B. Liq (Lithiumchinolinat). Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/$NiO_x$, Al/$PtO_x$) bevorzugt sein. Für einige Anwendungen muss mindestens eine der Elektroden transparent oder teiltransparent sein, um entweder die Bestrahlung des organischen Materials (O-SC) oder die Auskopplung von Licht (OLED/PLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere, z. B. PEDOT, PANI oder Derivate dieser Polymere. Bevorzugt ist weiterhin, wenn auf die Anode ein p-dotiertes Lochtransportmaterial als Lochinjektionsschicht aufgebracht wird, wobei sich als p-Dotanden Metalloxide, beispielsweise MoOs oder WOs, oder (per)fluorierte elektronenarme Aromaten eignen. Weitere geeignete p-Dotanden sind HAT-CN (Hexacyano-hexaazatriphenylen) oder die Verbindung NPD9 von Novaled. Eine solche Schicht vereinfacht die Lochinjektion in Materialien mit einem tiefen HOMO, also einem betragsmäßig großen HOMO.

In den weiteren Schichten können generell alle Materialien verwendet werden, wie sie gemäß dem Stand der Technik für die Schichten verwendet werden, und der Fachmann kann ohne erfinderisches Zutun jedes dieser Materialien in einer elektronischen Vorrichtung mit den erfindungsgemäßen Materialien kombinieren.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Weiterhin bevorzugt ist eine elektronischen Vorrichtung, insbesondere eine organische Elektrolumineszenzvorrichtung, welche dadurch gekennzeichnet ist, dass eine oder mehrere Schichten mit einem Sublimationsverfahren beschichtet werden. Dabei werden die Materialien in Vakuum-Sublimationsanlagen bei einem Anfangsdruck von üblicherweise kleiner $10^{-5}$ mbar, bevorzugt kleiner $10^{-6}$ mbar aufgedampft. Es ist auch möglich, dass der Anfangsdruck noch geringer oder noch höher ist, beispielsweise kleiner $10^{-7}$ mbar.

Bevorzugt ist ebenfalls eine elektronischen Vorrichtung, insbesondere eine organische Elektrolumineszenzvorrichtung, welche dadurch gekennzeichnet ist, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronischen Vorrichtung, insbesondere eine organische Elektrolumineszenzvorrichtung, welche dadurch gekennzeichnet ist, dass eine oder mehrere Schichten aus Lösung, wie z. B. durch Spincoating, oder mit einem beliebigen Druckverfahren, wie z. B. Siebdruck, Flexodruck, Offsetdruck oder Nozzle-Printing, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, welche beispielsweise durch geeignete Substitution erhalten werden.

Die elektronischen Vorrichtung, insbesondere die organische Elektrolumineszenzvorrichtung kann auch als Hybridsystem hergestellt werden, indem eine oder mehrere Schichten aus Lösung aufgebracht werden und eine oder mehrere andere Schichten aufgedampft werden. So ist es beispielsweise möglich, eine emittierende Schicht enthaltend eine erfindungsgemäße Verbindung umfassend Strukturen gemäß Formel (I) und ein Matrixmaterial aus Lösung aufzubringen und darauf eine Lochblockierschicht und/oder eine Elektronentransportschicht im Vakuum aufzudampfen.

Diese Verfahren sind dem Fachmann generell bekannt und können von ihm ohne Probleme auf elektronischen Vorrichtungen, insbesondere organische Elektrolumineszenzvorrichtungen enthaltend erfindungsgemäße Verbindungen umfassend Strukturen gemäß Formel (I) bzw. die oben aufgeführten bevorzugten Ausführungsformen angewandt werden.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:

1. Elektronische Vorrichtungen, insbesondere organische Elektrolumineszenzvorrichtungen enthaltend Verbindungen, Oligomere, Polymere oder Dendrimere mit Strukturen gemäß Formel (I) bzw. die zuvor und nachfolgend ausgeführten bevorzugten Ausführungsformen, insbesondere als elektronenleitende Materialien und/oder Lochleitermaterialien oder als Matrixmaterialien, weisen eine sehr gute Lebensdauer auf.

2. Elektronische Vorrichtungen, insbesondere organische Elektrolumineszenzvorrichtungen enthaltend Verbindungen, Oligomere, Polymere oder Dendrimere mit Strukturen gemäß Formel (I) bzw. die zuvor und nachfolgend ausgeführten bevorzugten Ausführungsformen insbesondere als Elektronentransport-Materialien, Lochleitermaterialien und/oder als Hostmaterialien weisen eine hervorragende Effizienz auf. Insbesondere ist die Effizienz deutlich höher gegenüber analogen Verbindungen, die keine Struktureinheit gemäß Formel (I) enthalten. Hierbei bewirken die erfindungsgemäßen Verbindungen, Oligomere, Polymere oder Dendrimere mit Strukturen gemäß Formel (I) bzw. die zuvor und nachfolgend ausgeführten bevorzugten Ausführungsformen eine geringe Betriebsspannung bei Verwendung in elektronischen Vorrichtungen. Hierbei bewirken diese Verbindungen insbesondere einen geringen Roll-off, d.h. einen geringen Abfall der Leistungseffizienz der Vorrichtung bei hohen Leuchtdichten.

3. Elektronische Vorrichtungen, insbesondere organische Elektrolumineszenzvorrichtungen enthaltend Verbindungen, Oligomere, Polymere oder Dendrimere mit Strukturen gemäß Formel (I) bzw. die zuvor und nachfolgend ausgeführten bevorzugten Ausführungsformen als Elektronentransportmaterialien, Lochleitermaterialien und/oder als Hostmaterialien weisen eine hervorragende Farbreinheit auf.

4. Die erfindungsgemäßen Verbindungen, Oligomere, Polymere oder Dendrimere mit Strukturen gemäß Formel (I) bzw. die zuvor und nachfolgend ausgeführten bevorzugten Ausführungsformen zeigen eine sehr hohe thermische und photochemische Stabilität und führen zu Verbindungen mit einer sehr hohen Lebensdauer.

5. Mit Verbindungen, Oligomeren, Polymeren oder Dendrimeren mit Strukturen gemäß Formel (I) bzw. die zuvor

und nachfolgend ausgeführten bevorzugten Ausführungsformen kann in elektronischen Vorrichtungen, insbesondere organische Elektrolumineszenzvorrichtungen die Bildung von optischen Verlustkanäle vermieden werden. Hierdurch zeichnen sich diese Vorrichtungen durch eine hohe PL- und damit hohe EL-Effizienz von Emittern bzw. eine ausgezeichnete Energieübertragung der Matrices auf Dotanden aus.

6. Verbindungen, Oligomere, Polymere oder Dendrimere mit Strukturen gemäß Formel (I) bzw. die zuvor und nachfolgend ausgeführten bevorzugten Ausführungsformen weisen eine ausgezeichnete Glasfilmbildung auf.

7. Verbindungen, Oligomere, Polymere oder Dendrimere mit Strukturen gemäß Formel (I) bzw. die zuvor und nachfolgend ausgeführten bevorzugten Ausführungsformen bilden aus Lösungen sehr gute Filme.

Diese oben genannten Vorteile gehen nicht mit einer Verschlechterung der weiteren elektronischen Eigenschaften einher.

Die erfindungsgemäßen Verbindungen und Mischungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der erfindungsgemäßen Verbindungen oder Mischungen in einer elektronischen Vorrichtung, insbesondere in einer organischen Elektrolumineszenzvorrichtung.

Ein nochmals weiterer Gegenstand der vorliegenden Erfindung ist die Verwendung einer erfindungsgemäßen Verbindung und/oder eines erfindungsgemäßen Oligomers, Polymers oder Dendrimers in einer elektronischen Vorrichtung als als Hostmaterial, Lochleitermaterial, Elektroneninjektionsmaterial und/oder Elektronentransportmaterial, vorzugsweise als Hostmaterial und/oder Elektronentransportmaterial.

Nochmals ein weiterer Gegenstand der vorliegenden Erfindung ist eine elektronische Vorrichtung enthaltend mindestens eine der oben ausgeführten erfindungsgemäßen Verbindungen oder Mischungen. Dabei gelten die oben für die Verbindung ausgeführten Bevorzugungen auch für die elektronischen Vorrichtungen. Besonders bevorzugt ist elektronische Vorrichtung ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), organischen elektrischen Sensoren, lichtemittierenden elektrochemischen Zellen (LECs), organischen Laserdioden (O-Laser) und Organic Plasmon Emitting Devices, bevorzugt organischen Elektrolumineszenzvorrichtungen (OLEDs, PLEDs), insbesondere phosphoreszierenden OLEDs.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (I) bzw. gemäß den bevorzugten Ausführungsformen einsetzen.

Die erfindungsgemäßen Verbindungen weisen bei Verwendung in organischen Elektrolumineszenzvorrichtungen generell sehr gute Eigenschaften auf. Insbesondere ist bei Verwendung der erfindungsgemäßen Verbindungen in organischen Elektrolumineszenzvorrichtungen die Lebensdauer wesentlich besser im Vergleich zu ähnlichen Verbindungen gemäß dem Stand der Technik. Dabei sind die weiteren Eigenschaften der organischen Elektrolumineszenzvorrichtung, insbesondere die Effizienz und die Spannung, ebenfalls besser oder zumindest vergleichbar.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen. Der Fachmann kann aus den Schilderungen ohne erfinderisches Zutun weitere erfindungsgemäße elektronische Vorrichtungen herstellen und somit die Erfindung im gesamten beanspruchten Bereich ausführen.

## Beispiele

Die nachfolgenden Synthesen werden, sofern nicht anders angegeben, unter einer Schutzgasatmosphäre in getrockneten Lösungsmitteln durchgeführt. Die Edukte können von ALDRICH bezogen werden. Die Nummern bei den literaturbekannten Edukten, die teilweise in eckigen Klammern angegeben sind, sind die entsprechenden CAS-Nummern.

### Synthesebeispiele

#### a) Benzo[kl]xanthen-9-yl-(2-chlorphenyl)-amin

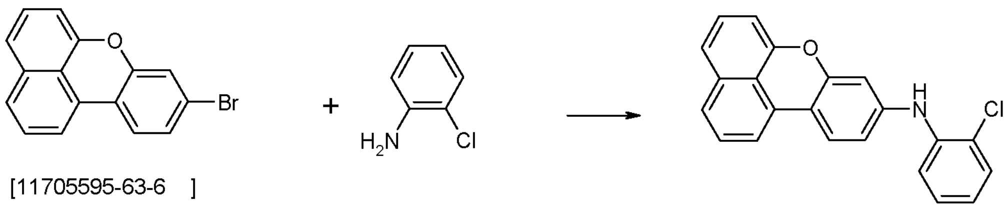

[11705595-63-6 ]

40 g (137 mmol) 9-Brom-benzo[kl]xanthen, 17.9 g (140 mmol) 2-Chloranilin, 68.2 g (710 mmol) Natrium-tert-butylat, 613 mg (3 mmol) Palladium-(II)acetat und 3.03 g (5 mmol) dppf werden in 1.3 L Toluol gelöst und 5 h unter Rückfluss gerührt. Das Reaktionsgemisch wird auf Raumtemperatur abgekühlt, mit Toluol erweitert und über Celite filtriert. Das Filtrat wird im Vakuum eingeengt und der Rückstand aus Toluol/Heptan kristallisiert. Das Produkt wird als farbloser Feststoff isoliert. Ausbeute: 39 g (109 mmol); 83% der Theorie.

Analog können folgende Verbindungen hergestellt werden:

| | Edukt 1 | Edukt 2 | Produkt | Ausbeute |
|---|---|---|---|---|
| 1a | 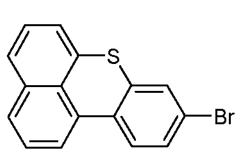 [1705595-60-3 | 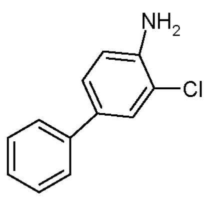 7285-66-7 | 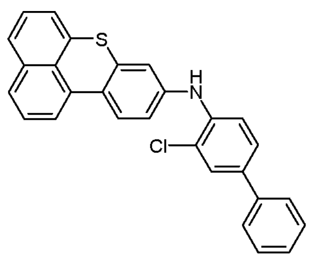 | 81% |

(fortgesetzt)

| | Edukt 1 | Edukt 2 | Produkt | Ausbeute |
|---|---|---|---|---|
| 2a | 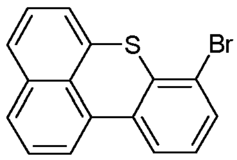 [1705595-57-8 ] | 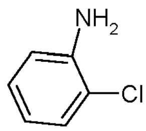 | 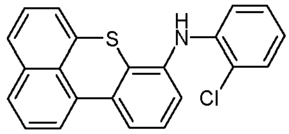 | 75% |
| 3a | 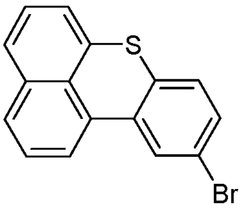 [11705595-56-7 ] | 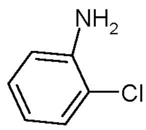 | 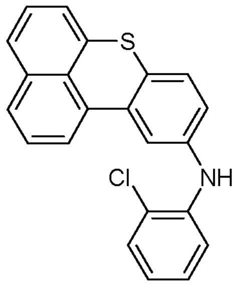 | 76% |
| 4a | 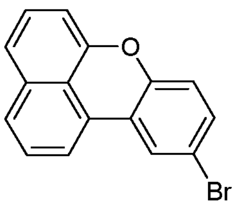 [1705595-93-2 ] | 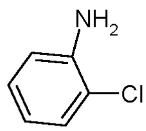 | 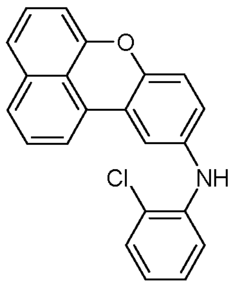 | 72% |
| 5a | 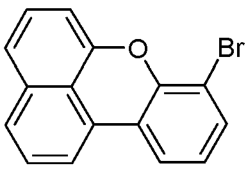 [1705595-92-1 ] | 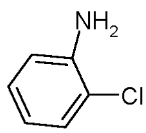 | 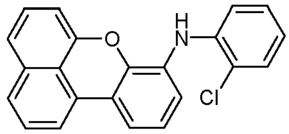 | 79% |
| 6a | 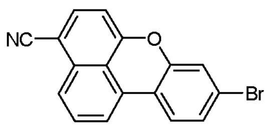 [1809903-63-6 ] | 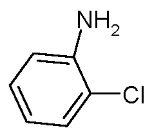 | 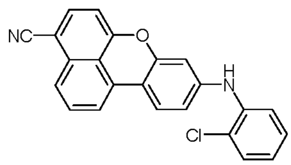 | 71% |
| 7a | 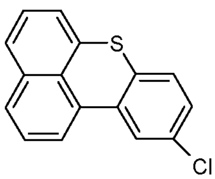 [ 1803291-30-6 ] | 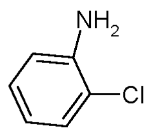 | 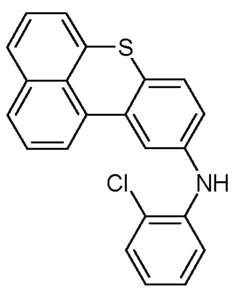 | 73% |

(fortgesetzt)

| | Edukt 1 | Edukt 2 | Produkt | Ausbeute |
|---|---|---|---|---|
| 8a | 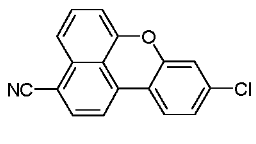 [ 1643433-77-5 ] | 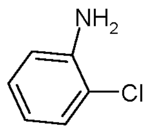 | 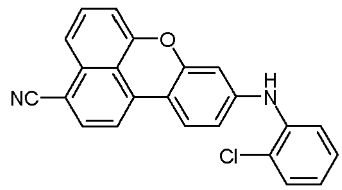 | 77% |
| 9a | 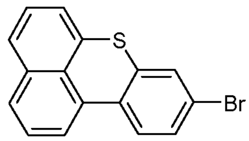 [1705595-60-3 | 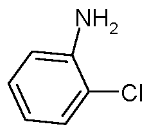 | 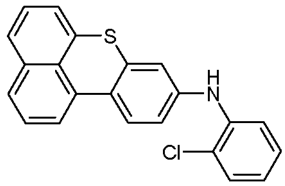 | 70% |
| 10a | 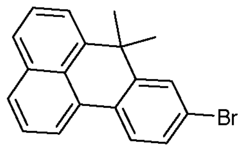 [1198396-54-1 ] | 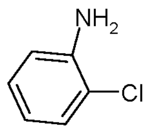 | 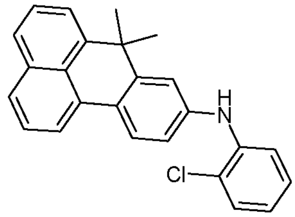 | 70% |
| 11a | 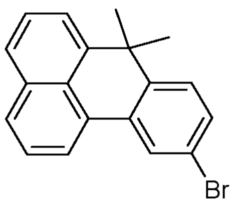 [1705595-69-2 ] | 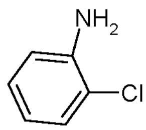 | 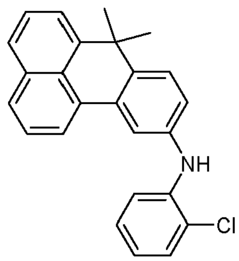 | 71% |
| 12a | 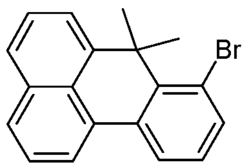 [1705595-68-1 ] | 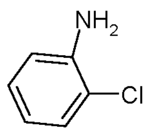 | 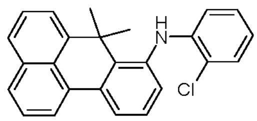 | 76% |
| 13a | 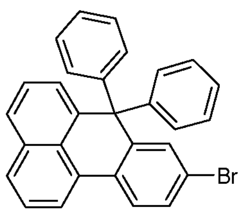 [ 1705595-95-4 ] | 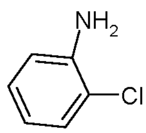 | 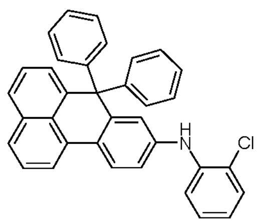 | 70% |

(fortgesetzt)

| | Edukt 1 | Edukt 2 | Produkt | Ausbeute |
|---|---|---|---|---|
| 14a | 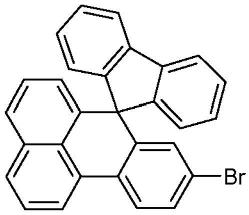 [ 1705595-96-5 ] | 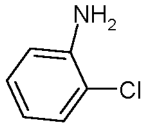 | 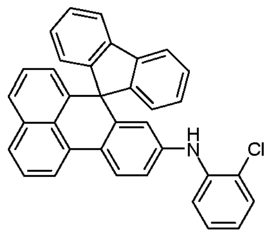 | 69% |
| 15a | 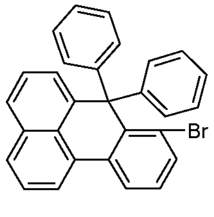 [ 1705595-94-3 ] | 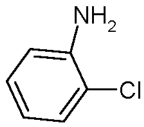 | 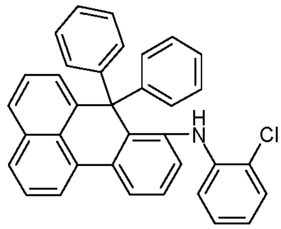 | 71% |
| 16a | 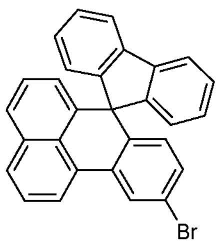 [1705595-71-6 ] | 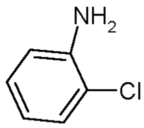 | 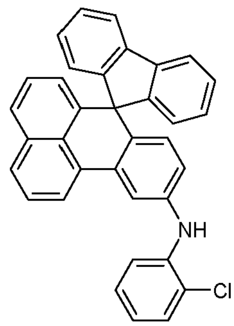 | 67% |
| 17a | 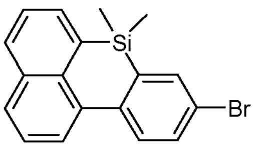 [1612224-62-0 ] | 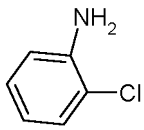 | 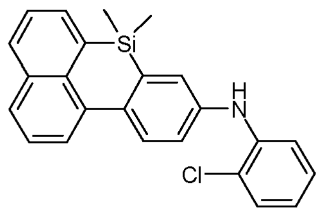 | 71% |

**b) Cyclisierung**

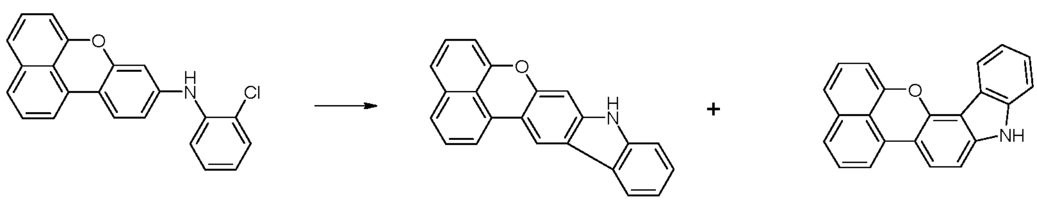

A

B

35 g (102 mmol) Benzo[kl]xanthen-9-yl-(2-chlorphenyl)-amin, 56 g (409 mmol) Kaliumcarbonat, 4.5 g (12 mmol) Tricyclohexylphosphintetrafluoroborat und 1.38 g (6 mmol) Palladium(II)acetat werden in 500 mL Dimethylacetamid suspendiert und 6 h unter Rückfluss gerührt. Nach Erkalten wird die Reaktionmischung mit 300 ml Wasser und 400 mL

Ethylacetat versetzt. Man rührt 30 min. nach, trennt die organische Phase ab, filtriert diese über ein kurzes Celite-Bett und entfernt dann das Lösemittel im Vakuum. Das Rohprodukt wird mit Toluol heiß extrahiert und aus Toluol umkristallisiert. Ausbeute: 28 g (229 mmol) der Mischung A+B; 90% der Theorie; Reinheit: 98.0% nach HPLC. Nach Umkristallisation aus Ethylacetat/Toluol (1:2) erhält man 62% A und 23% B.

Analog können folgende Verbindungen hergestellt werden:

| | Edukt 1 | Produkt | Produkt | Ausbeute |
|---|---|---|---|---|
| 1b | 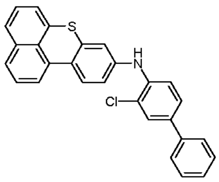 | 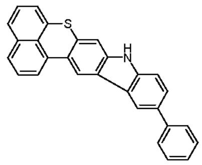 | 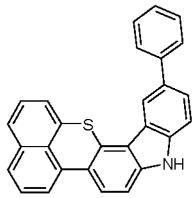 | 60% / 19% |
| 2b | 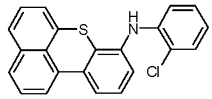 | 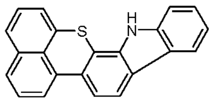 | | 72% |
| 3b | 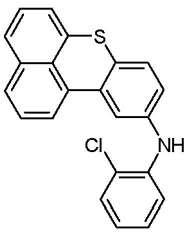 | 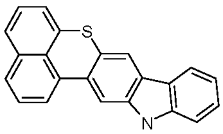 | 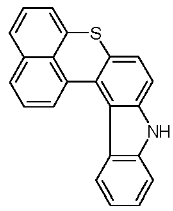 | 54% / 22% |
| 4b | 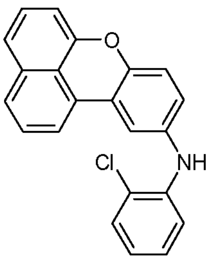 | 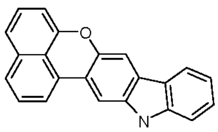 | 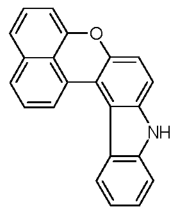 | 59% / 18% |
| 5b | 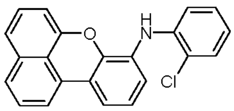 | 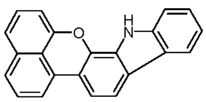 | | 67% |
| 6b | 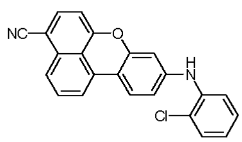 | 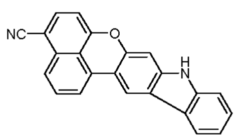 | 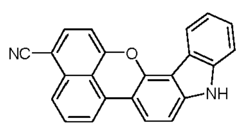 | 56% / 20% |
| 7b | 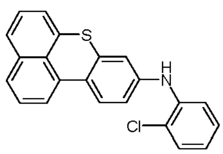 | 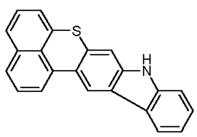 | 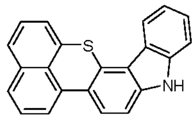 | 53% / 19% |
| 8b | 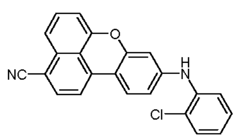 | 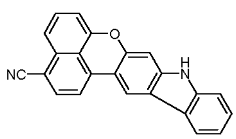 | 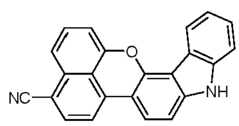 | 50% / 21% |

(fortgesetzt)

| | Edukt 1 | Produkt | Produkt | Ausbeute |
|---|---|---|---|---|
| 9b | 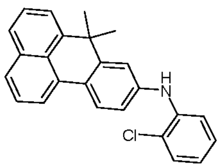 | 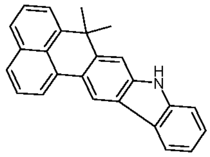 | 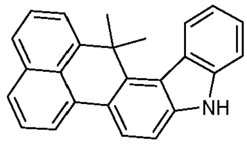 | 59% / 11 % |

| | | | | |
|---|---|---|---|---|
| 10b | 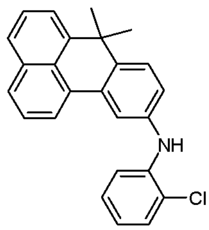 | 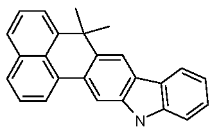 | 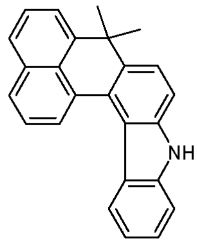 | 50% / 20% |
| 11b | 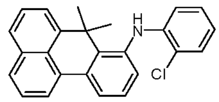 | 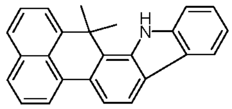 | | 77% |
| 12b | 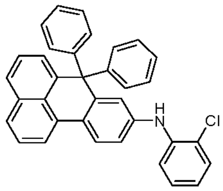 | 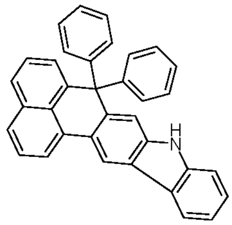 | 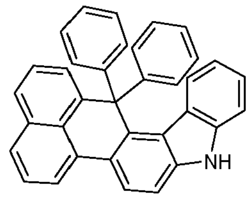 | 61% / 15% |
| 13b | 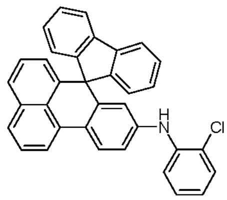 | 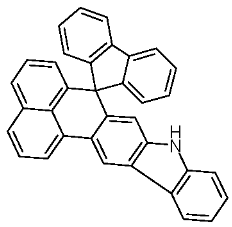 | 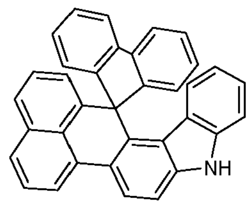 | 59% / 16% |
| 14b | 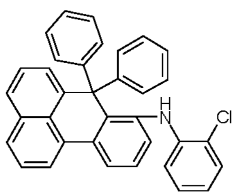 | 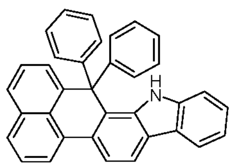 | | 72% |
| 15b | 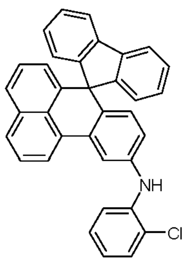 | 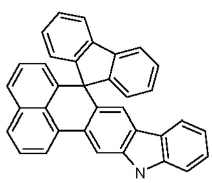 | 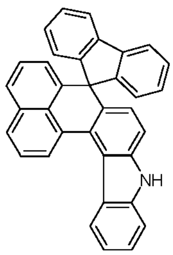 | 55% / 22% |

(fortgesetzt)

| | | | | |
|---|---|---|---|---|
| 16b | 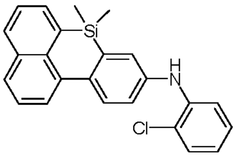 | 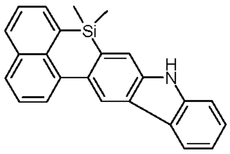 | 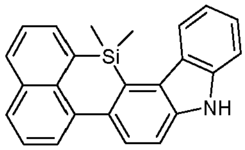 | 41% / 24% |

**c) Buchwald**

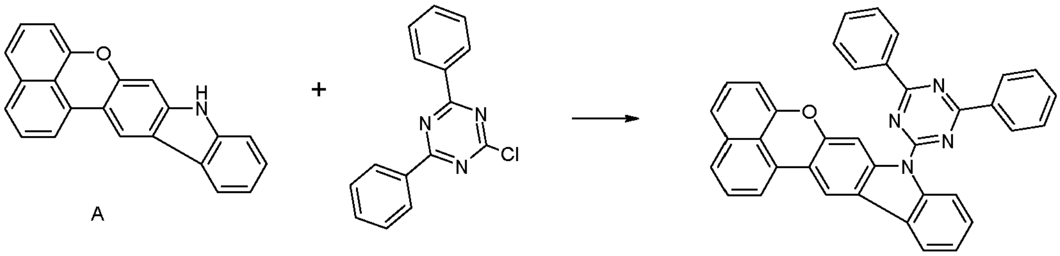
A

4.3 g NaH (107 mmol), 60%ig in Mineralöl, werden in 300 mL Dimethylformamid unter Schutzatmosphäre gelöst. 32 g (107 mmol) Carbazol-Derivat (A) werden in 250 mL DMF gelöst und zu der Reaktionsmischung zugetropft. Nach 1 h bei Raumtemperatur wird eine Lösung von 2-Chlor-4,6-diphenyl-[1,3,5]triazin (34.5 g, 0.122 mol) in 200 mL THF zugetropft. Das Reaktionsgemisch wird 12 h bei Raumtemperatur gerührt und dann auf Eis gegossen. Der dabei ausgefallene Feststoff wird nach Erwärmen auf Raumtemperatur filtriert und mit Ethanol und Heptan gewaschen. Der Rückstand wird mit Toluol heiß extrahiert, aus Toluol/n-Heptan umkristallisiert und abschließend im Hochvakuum sublimiert. Die Reinheit beträgt 99.9%. Die Ausbeute beträgt 38 g (70 mmol); 66% der Theorie.

Analog können folgende Verbindungen hergestellt werden:

| | Edukt1 | Edukt 2 | Produkt | Ausbeute |
|---|---|---|---|---|
| 1c | 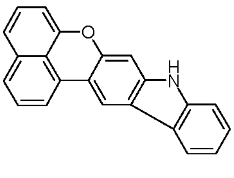 | 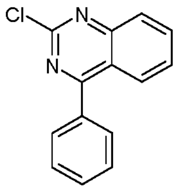 [29874-83-7] | 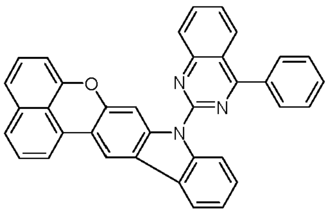 | 66% |
| 2c | 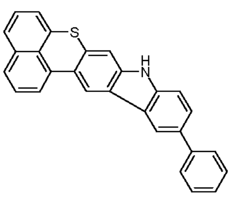 | 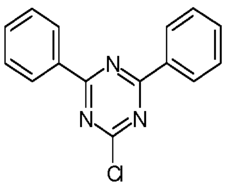 3842-55-5 | 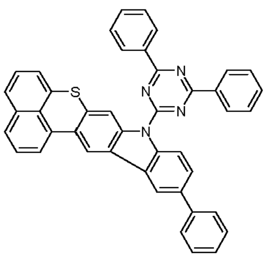 | 62% |

(fortgesetzt)

| | Edukt1 | Edukt 2 | Produkt | Ausbeute |
|---|---|---|---|---|
| 3c | 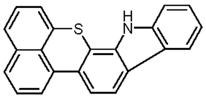 | 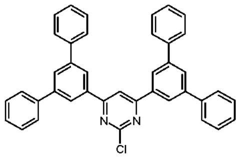 1384480-21-0 | 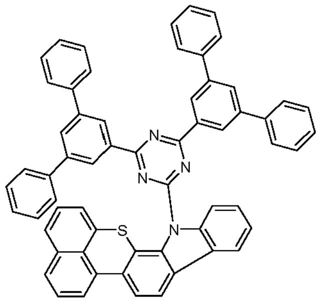 | 64% |
| 4c | 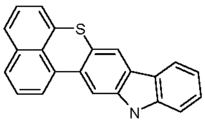 | 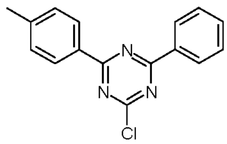 92853-85-5 | 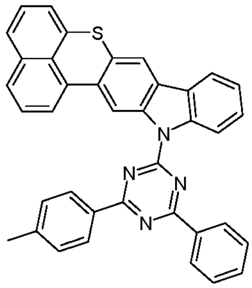 | 57% |
| 5c | 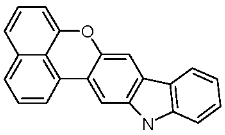 | 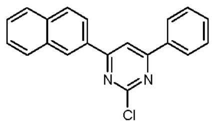 1260393-65-4 | 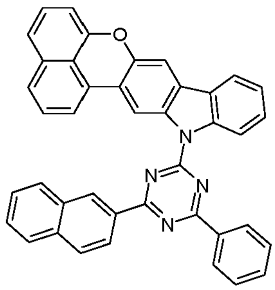 | 61% |
| 6c | 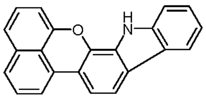 | 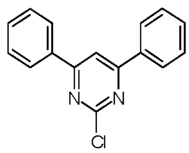 2915-16-4 | 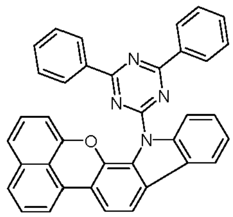 | 63% |
| 7c | 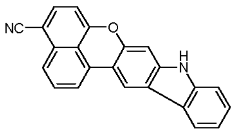 | 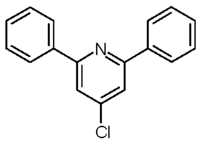 133785-60-1 | 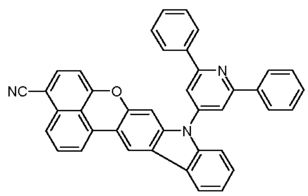 | 60% |
| 8c | 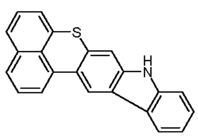 | 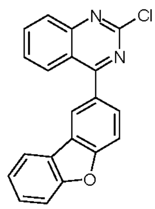 [1616499-38-7] | 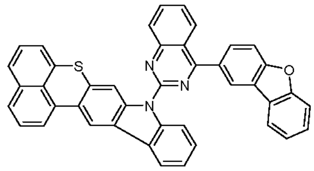 | 68% |

(fortgesetzt)

| | Edukt1 | Edukt 2 | Produkt | Ausbeute |
|---|---|---|---|---|
| 9c | 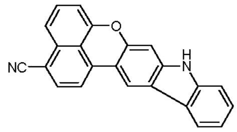 | 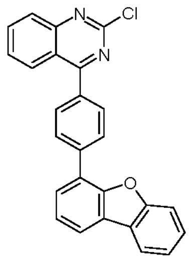 [1403252-58-3] | 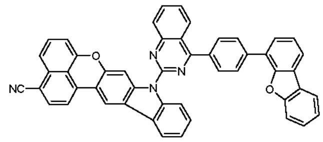 | 65% |
| 10c | 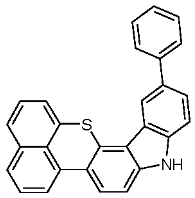 | 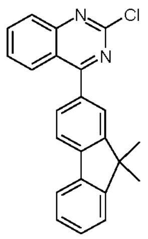 [1373265-66-7] | 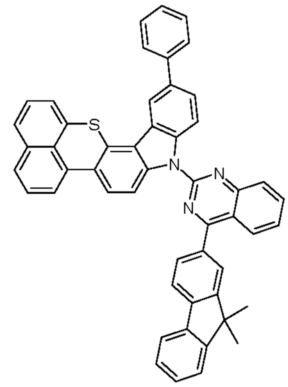 | 69% |
| 11c | 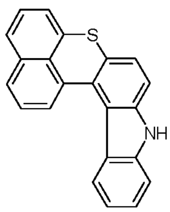 | 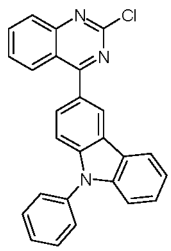 [1373317-91-9] | 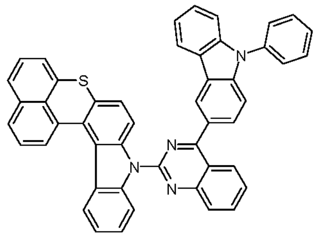 | 67% |
| 12c | 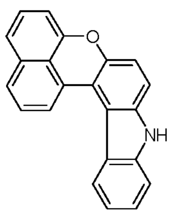 | 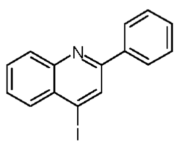 [643017-61-2] | 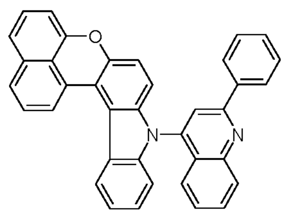 | 64% |
| 13c | 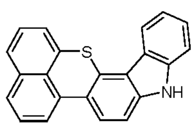 | 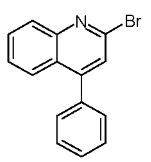 [857206-12-3] | 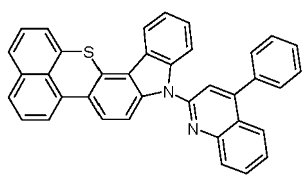 | 63% |

(fortgesetzt)

| | Edukt1 | Edukt 2 | Produkt | Ausbeute |
|---|---|---|---|---|
| 14c | 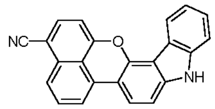 | 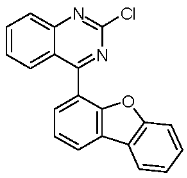 [14003252-55-0] | 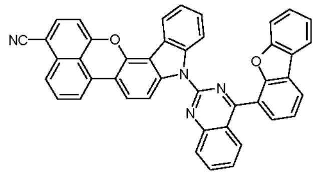 | 68% |
| 15c | 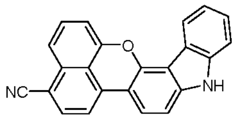 | 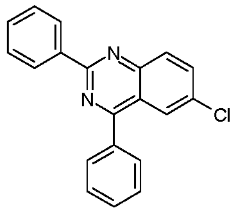 [30169-34-7] | 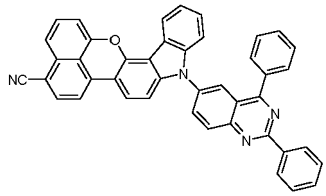 | 65% |
| 16c | 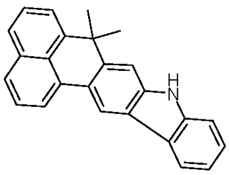 | 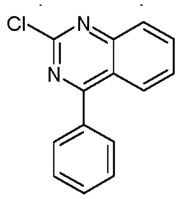 [29874-83-7] | 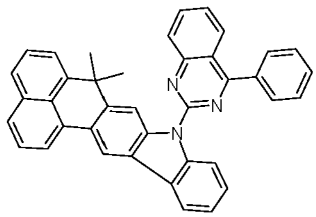 | 62% |
| 17c | 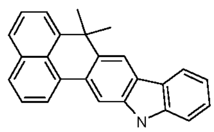 | 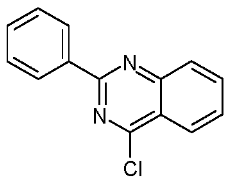 [6484-25-9] | 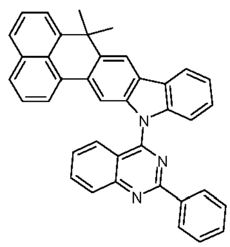 | 65% |
| 18c | 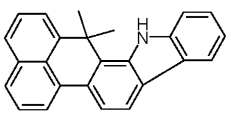 | 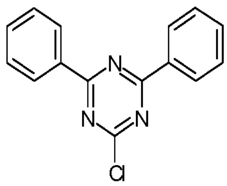 3842-55-5 | 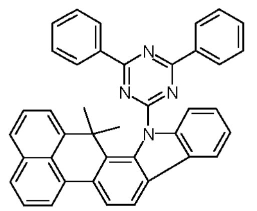 | 71% |
| 19c | 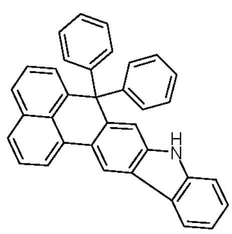 | 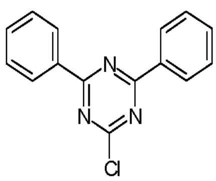 3842-55-5 | 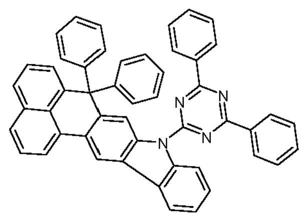 | 69% |

(fortgesetzt)

| | Edukt1 | Edukt 2 | Produkt | Ausbeute |
|---|---|---|---|---|
| 20c | 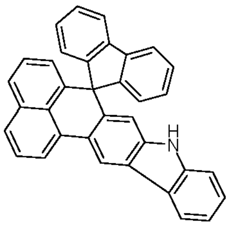 | 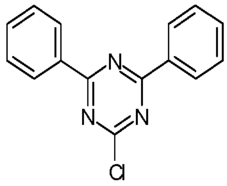 3842-55-5 | 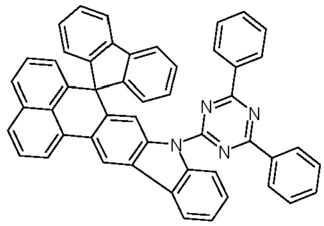 | 73% |
| 21c | 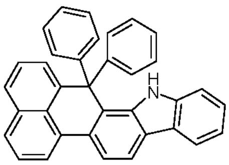 | 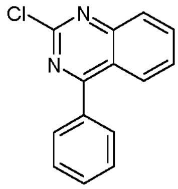 [29874-83-7] | 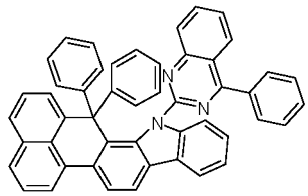 | 70% |
| 22c | 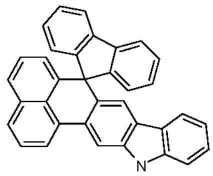 | 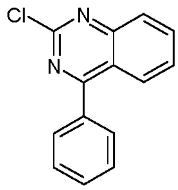 [29874-83-7] | 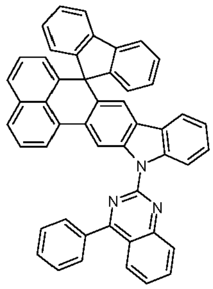 | 63% |
| 23c | 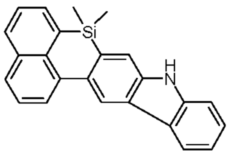 | 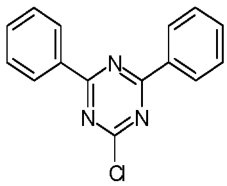 3842-55-5 | 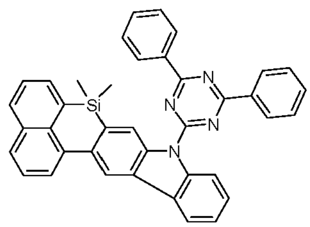 | 60% |
| 24c | 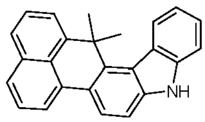 | 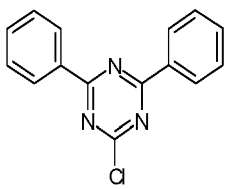 3842-55-5 | 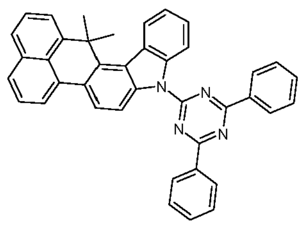 | 71% |
| 25c | 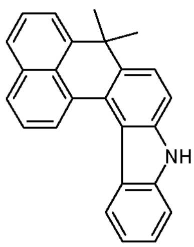 | 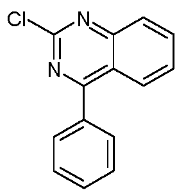 [29874-83-7] | 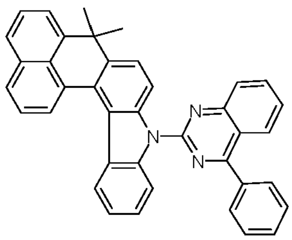 | 7% |

(fortgesetzt)

| | Edukt1 | Edukt 2 | Produkt | Ausbeute |
|---|---|---|---|---|
| 26c | 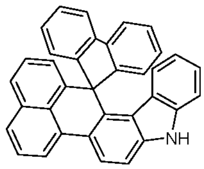 | 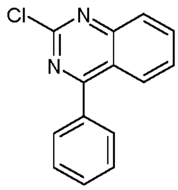 [29874-83-7] | 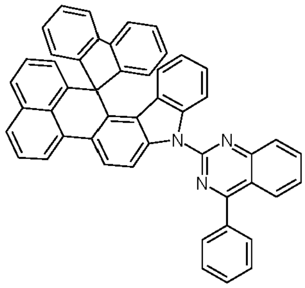 | 69% |
| 27v | 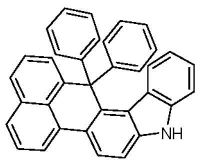 | 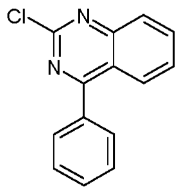 [29874-83-7] | 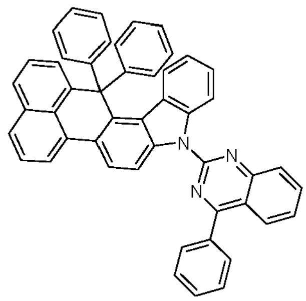 | 63% |
| 28c | 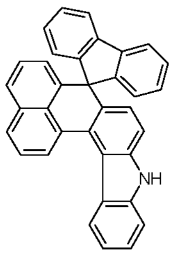 | 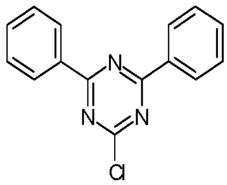 3842-55-5 | 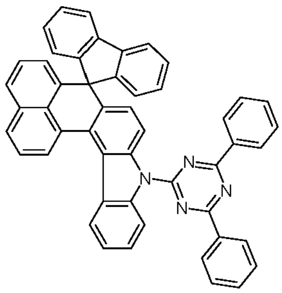 | 64% |
| 29c | 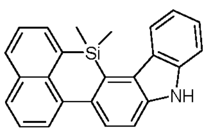 | 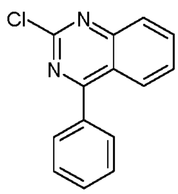 [29874-83-7] | 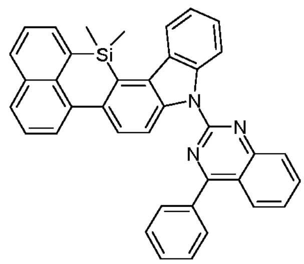 | 70% |
| 30c | 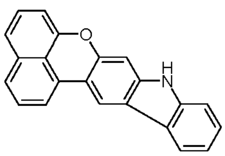 | 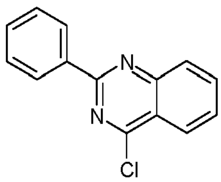 [6484-25-9] | 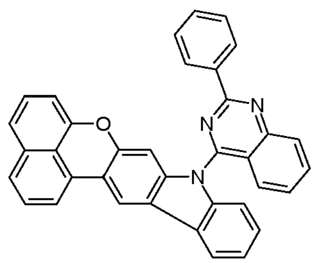 | 76% |

(fortgesetzt)

| | Edukt1 | Edukt 2 | Produkt | Ausbeute |
|---|---|---|---|---|
| 31c | 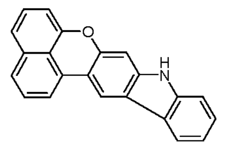 | 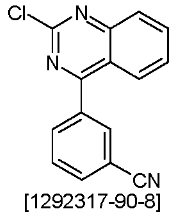 [1292317-90-8] | 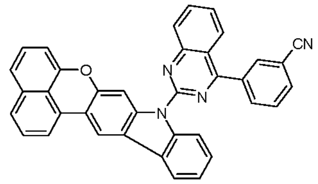 | 65% |
| 32c | 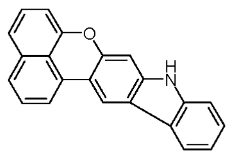 | 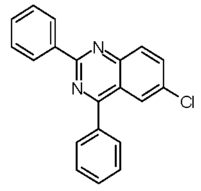 [30169-34-7] | 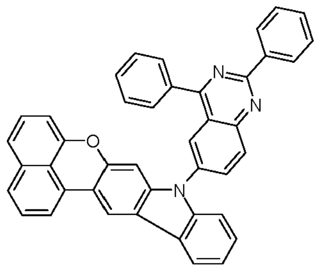 | 69% |
| 33c | 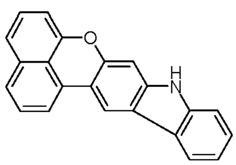 | 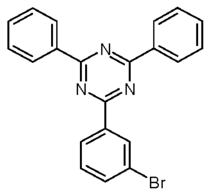 864377-31-1 | 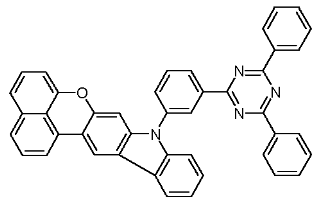 | 72% |
| 34c | 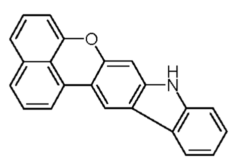 | 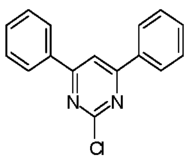 2915-16-4 | 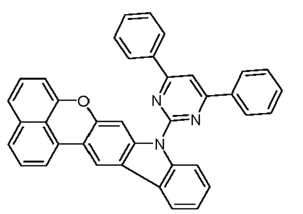 | 74% |

**Herstellung der OLEDs**

In den folgenden Beispielen E1 bis E19 werden die Daten verschiedener OLEDs vorgestellt.

**Vorbehandlung für die Beispiele E1-E19:** Glasplättchen, die mit strukturiertem ITO (Indium Zinn Oxid) der Dicke 50 nm beschichtet sind, werden vor der Beschichtung mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 2 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC5:IC3:TEG2 (55%:35%:10%) bedeutet hierbei, dass das Material IC5 in einem Volumenanteil von 55%, IC3 in einem Anteil von 35% und TEG2 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/$m^2$ bestimmt und daraus die CIE 1931 x und y Farbkoordinaten berechnet.

**Verwendung von erfindungsgemäßen Mischungen in der Emissionsschicht phosphoreszenter OLEDs**

Die erfindungsgemäßen Materialien können in der Emissionsschicht in phosphoreszierenden roten OLEDs eingesetzt werden. Das erfindungsgemäße Material EG1 ist in Beispiel E1 als Matrixmaterial in Kombination mit dem phosphoreszenten Emitter TEG5 eingesetzt. Bei einer Leuchtdichte von 1000 cd/$m^2$ hat die OLED Farbkoordinaten von CIEx=0.67 und CIEy=0.33. Auch in den Beispielen E2 bis E13 emittiert die OLED Licht mit den Farbkoordinaten CIEx=0.67 und CIEy=0.33. Dies zeigt, dass sich die erfindungsgemäßen Verbindungen EG1-EG13 zum Einsatz als Matrixmaterial in OLEDs eignen.

**Verwendung von erfindungsgemäßen Mischungen in der Elektronentransportschicht phosphoreszenter OLEDs**

Die erfindungsgemäßen Materialien können auch in der Elektronentransportschicht in OLEDs eingesetzt werden. In den Beispielen E14 bis E19 werden die erfindungsgemäßen Materialien EG14 bis EG19 in der Elektronentransportschicht eingesetzt. In den Beispielen E14 bis E19 emittiert die OLED Licht mit den Farbkoordinaten CIEx=0.67 und CIEy=0.33. Dies zeigt, dass sich die erfindungsgemäßen Verbindungen EG14 bis EG19 zum Einsatz als Elektronentransportmaterial in OLEDs eignen.

Tabelle 1: Aufbau der OLEDs

| Bsp | HIL Dicke | HTL Dicke | EBL Dicke | EML Dicke | HBL Dicke | ETL Dicke | EIL Dicke |
|---|---|---|---|---|---|---|---|
| E1 | HATCN 5nm | SpMA1 125nm | SpMA3 10nm | EG1:TER5 (95%:5%) 40nm | | ST2:LiQ (50%:50%) 35nm | |
| E2 | HATCN 5nm | SpMA1 125nm | SpMA3 10nm | EG2:TER5 (95%:5%) 40nm | | ST2:LiQ (50%:50%) 35nm | |
| E3 | HATCN 5nm | SpMA1 125nm | SpMA3 10nm | EG3:TER5 (95%:5%) 40nm | | ST2:LiQ (50%:50%) 35nm | |
| E4 | HATCN 5nm | SpMA1 125nm | SpMA3 10nm | EG4:TER5 (95%:5%) 40nm | | ST2:LiQ (50%:50%) 35nm | |
| E5 | HATCN 5nm | SpMA1 125nm | SpMA3 10nm | EG5:TER5 (95%:5%) 40nm | | ST2:LiQ (50%:50%) 35nm | |
| E6 | HATCN 5nm | SpMA1 125nm | SpMA3 10nm | EG6:TER5 (95%:5%) 40nm | | ST2:LiQ (50%:50%) 35nm | |
| E7 | HATCN 5nm | SpMA1 125nm | SpMA3 10nm | EG7:TER5 (95%:5%) 40nm | | ST2:LiQ (50%:50%) 35nm | |
| E8 | HATCN 5nm | SpMA1 125nm | SpMA3 10nm | EG8:TER5 (95%:5%) 40nm | | ST2:LiQ (50%:50%) 35nm | |
| E9 | HATCN 5nm | SpMA1 125nm | SpMA3 10nm | EG9:TER5 (95%:5%) 40nm | | ST2:LiQ (50%:50%) 35nm | |

(fortgesetzt)

| Bsp | HIL Dicke | HTL Dicke | EBL Dicke | EML Dicke | HBL Dicke | ETL Dicke | EIL Dicke |
|---|---|---|---|---|---|---|---|
| E10 | HATCN 5nm | SpMA1 125nm | SpMA3 10nm | EG10:TER5 (95%:5%) 40nm | | ST2:LiQ (50%:50%) 35nm | |
| E11 | HATCN 5nm | SpMA1 125nm | SpMA3 10nm | EG11:TER5 (95%:5%) 40nm | | ST2:LiQ (50%:50%) 35nm | |
| E12 | HATCN 5nm | SpMA1 125nm | SpMA3 10nm | EG12:TER5 (95%:5%) 40nm | | ST2:LiQ (50%:50%) 35nm | |
| E13 | HATCN 5nm | SpMA1 125nm | SpMA3 10nm | EG1:TER5 (95%:5%) 40nm | ST2 5nm | ST2:EG13 (50%:50%) 30nm | LiQ 3nm |
| E14 | HATCN 5nm | SpMA1 125nm | SpMA3 10nm | EG1:TER5 (95%:5%) 40nm | ST2 5nm | ST2:EG14 (50%:50%) 30nm | LiQ 3nm |
| E15 | HATCN 5nm | SpMA1 125nm | SpMA3 10nm | EG1:TER5 (95%:5%) 40nm | ST2 5nm | ST2:EG15 (50%:50%) 30nm | LiQ 3nm |
| E16 | HATCN 5nm | SpMA1 125nm | SpMA3 10nm | EG1:TER5 (95%:5%) 40nm | ST2 5nm | ST2:EG16 (50%:50%) 30nm | LiQ 3nm |
| E17 | HATCN 5nm | SpMA1 125nm | SpMA3 10nm | EG1:TER5 (95%:5%) 40nm | ST2 5nm | ST2:EG17 (50%:50%) 30nm | LiQ 3nm |
| E18 | HATCN 5nm | SpMA1 125nm | SpMA3 10nm | EG1:TER5 (95%:5%) 40nm | ST2 5nm | ST2:EG18 (50%:50%) 30nm | LiQ 3nm |
| E19 | HATCN 5nm | SpMA1 125nm | SpMA3 10nm | EG1:TER5 (95%:5%) 40nm | ST2 5nm | ST2:EG19 (50%:50%) 30nm | LiQ 3nm |

Tabelle 2: Strukturformeln der Materialien für die OLEDs

| | |
|---|---|
| 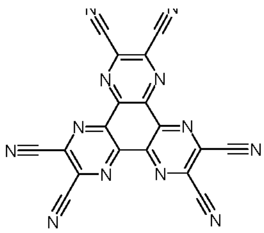 | 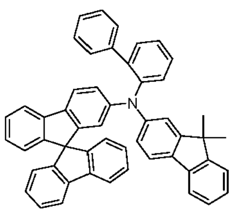 |
| HATCN | SpMA1 |

(fortgesetzt)

| | |
|---|---|
| 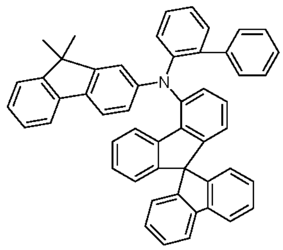 | 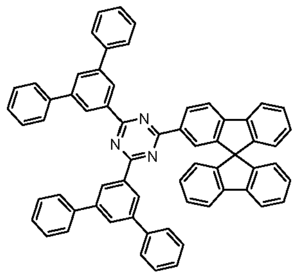 |
| SpMA3 | ST2 |
| 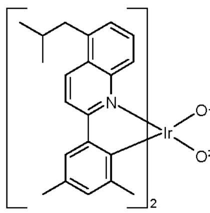 | 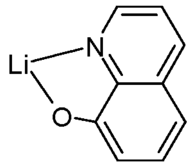 |
| TER5 | LiQ |
| 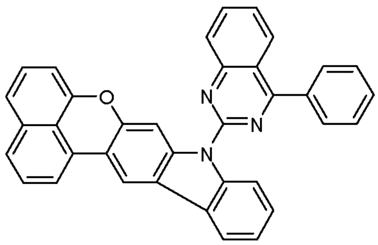 | 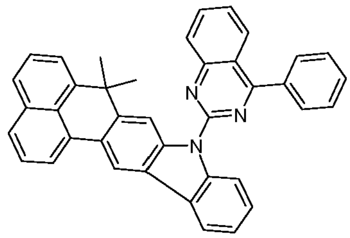 |
| EG1 | EG2 |
| 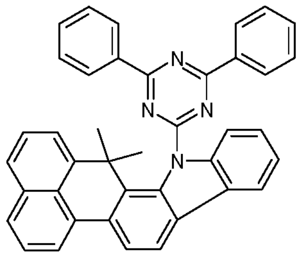 | 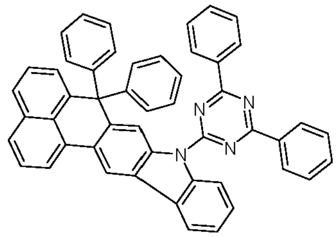 |
| EG3 | EG4 |
| 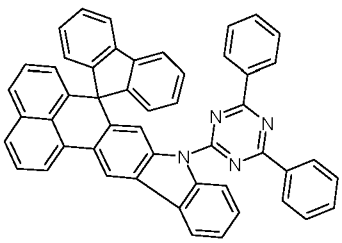 | 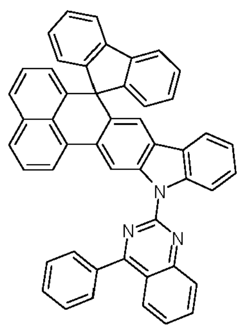 |
| EG5 | EG6 |

(fortgesetzt)

| | |
|---|---|
| 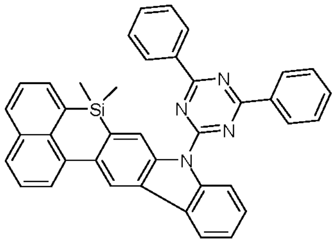 | 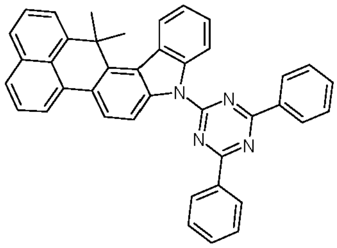 |
| EG7 | EG8 |
| 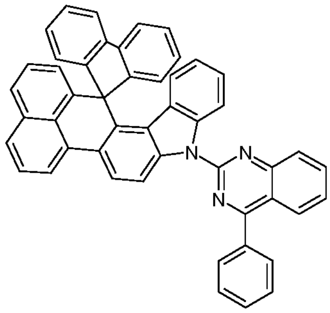 | 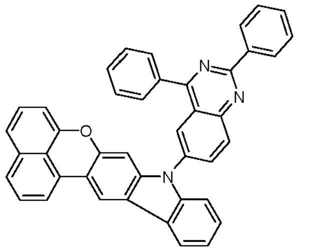 |
| EG9 | EG10 |
| 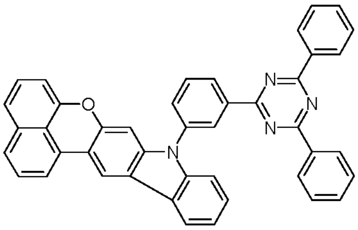 | 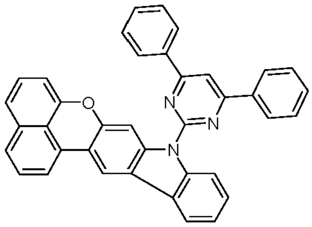 |
| EG11 | EG12 |
| 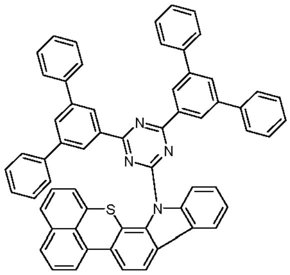 | 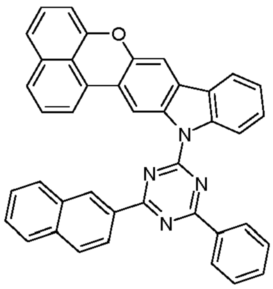 |
| EG13 | EG14 |
| 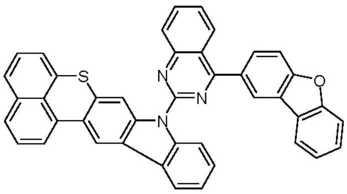 | 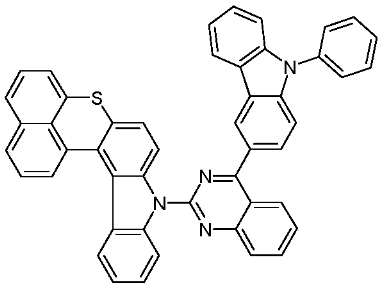 |
| EG15 | EG16 |

(fortgesetzt)

| | |
|---|---|
| 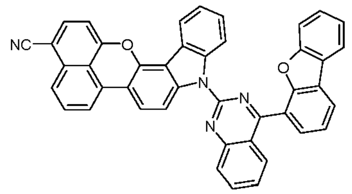 | 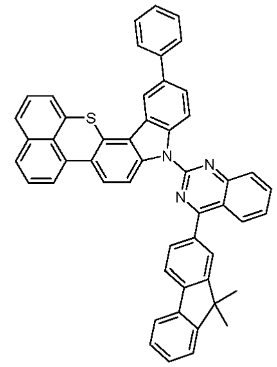 |
| EG17 | EG18 |
| 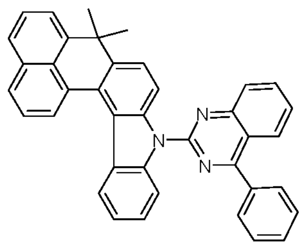 | |
| EG19 | |

**Patentansprüche**

**1.** Verbindung, umfassend mindestens eine Struktur der Formel (I),

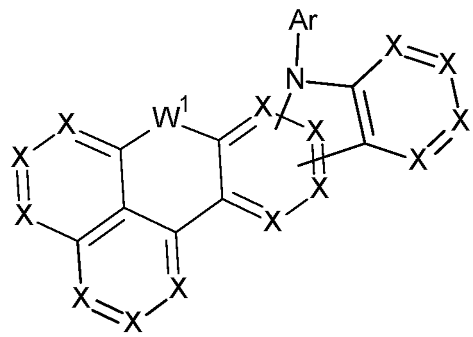

Formel (I)

wobei für die verwendeten Symbole gilt:

X ist bei jedem Auftreten gleich oder verschieden N oder $CR^1$, vorzugsweise $CR^1$, oder C, falls an X die Indologruppe gebunden ist;

$W^1$ ist O, S, $C(R^1)_2$, oder $Si(R^1)_2$;

$R^1$ ist bei jedem Auftreten gleich oder verschieden H, D, F, Cl, Br, I, CN, $NO_2$, $N(Ar^1)_2$, $N(R^2)_2$, $C(=O)Ar^1$, $C(=O)R^2$, $P(=O)(Ar^1)_2$, $P(Ar^1)_2$, $B(Ar^1)_2$, $B(OR^2)_2$, $Si(Ar^1)_3$, $Si(R^2)_3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Alkenylgruppe mit 2 bis 40 C-Atomen, worunter auch Cyclopentenyl, Cyclohexenyl, Cycloheptenyl, Cyclooctenyl und Cyclooctadienyl fallen, die jeweils mit einem oder mehreren Resten $R^2$ substituiert sein kann, wobei eine oder mehrere nicht benachbarte $CH_2$-Gruppen durch $-R^2C=CR^2-$, -C=C-, $Si(R^2)_2$, $Ge(R^2)_2$, $Sn(R^2)_2$, C=O, C=S, C=Se, $C=NR^2$, -C(=O)O-, $-C(=O)NR^2-$, $NR^2$, $P(=O)(R^2)$, -O-, -S-, SO

oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^2$ substituiert sein kann, oder eine Aryloxy- oder Heteroaryloxygruppe mit 5 bis 40 aromatischen Ringatomen, die durch einen oder mehrere Reste $R^2$ substituiert sein kann, oder eine Aralkyl- oder Heteroaralkylgruppe mit 5 bis 40 aromatischen Ringatomen, die mit einem oder mehreren Resten $R^2$ substituiert sein kann, oder eine Kombination dieser Systeme;;

Ar ist bei jedem Auftreten gleich oder verschieden ein aromatisches oder heteroaromatisches Ringsystem mit 5 bis 60 aromatischen Ringatomen, das mit einem oder mehreren Resten $R^1$ substituiert sein kann;

$Ar^1$ ist bei jedem Auftreten gleich oder verschieden ein aromatisches oder heteroaromatisches Ringsystem mit 5 bis 30 aromatischen Ringatomen, das mit einem oder mehreren Resten $R^2$ substituiert sein kann, dabei können zwei Reste $Ar^1$, welche an dasselbe Si-Atom, N-Atom, P-Atom oder B-Atom binden, auch durch eine Einfachbindung oder eine Brücke, ausgewählt aus $B(R^2)$, $C(R^2)_2$, $Si(R^2)_2$, C=O, $C=NR^2$, $C=C(R^2)_2$, O, S, S=O, $SO_2$, $N(R^2)$, $P(R^2)$ und $P(=O)R^2$, miteinander verbrückt sein;

$R^2$ ist bei jedem Auftreten gleich oder verschieden H, D, F, Cl, Br, I, CN, $B(OR^3)_2$, $NO_2$, $C(=O)R^3$, $CR^3=C(R^3)_2$, $C(=O)OR^3$, $C(=O)N(R^3)_2$, $Si(R^3)_3$, $P(R^3)_2$, $B(R^3)_2$, $N(R^3)_2$, $NO_2$, $P(=O)(R^3)_2$, $OSO_2R^3$, $OR^3$, $S(=O)R^3$, $S(=O)_2R^3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $-R^3C=CR^3-$, -C=C-, $Si(R^3)_2$, $Ge(R^3)_2$, $Sn(R^3)_2$, C=O, C=S, $C=NR^3$, -C(=O)O-, $-C(=O)NR^3-$, $NR^3$, $P(=O)(R^3)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^3$ substituiert sein kann, oder eine Aryloxy- oder Heteroaryloxy-gruppe mit 5 bis 40 aromatischen Ringatomen, die durch einen oder mehrere Reste $R^3$ substituiert sein kann, oder eine Kombination dieser Systeme; dabei können zwei oder mehrere, vorzugsweise benachbarte Substituenten $R^2$ auch miteinander ein mono- oder polycyclisches, aliphatisches, heteroaliphatisches, aromatisches oder heteroaromatisches Ringsystem bilden;

$R^3$ ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen, worunter auch Cyclopropyl und Cyclobutyl fallen, substituiert sein kann, dabei können zwei oder mehrere, vorzugsweise benachbarte Substituenten $R^3$ auch miteinander ein mono- oder polycyclisches, aliphatisches, heteroaliphatisches, aromatisches oder heteroaromatisches Ringsystem bilden.

**2.** Verbindung gemäß Anspruch 1, umfassend mindestens eine Struktur der Formel (IIa), (IIb), (IIc), (IId), (IIe) oder (IIf),

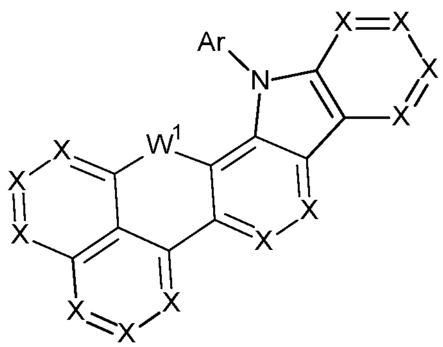

Formel (IIa)

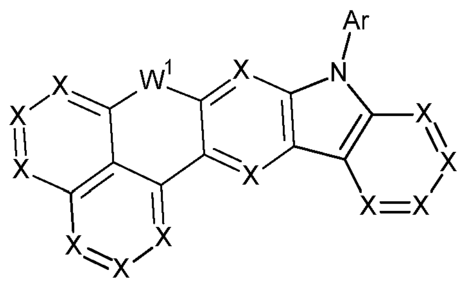

Formel (IIb)

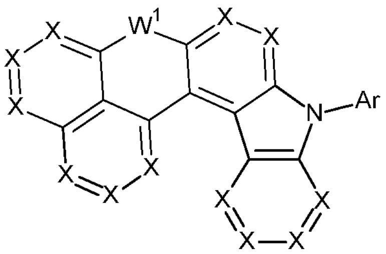

Formel (IIc)

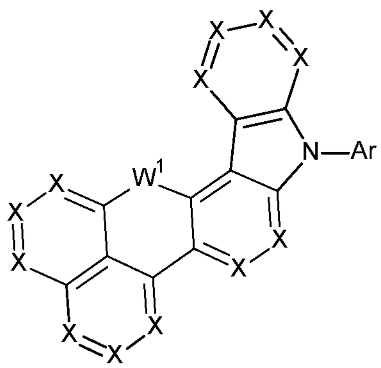

Formel (IId)

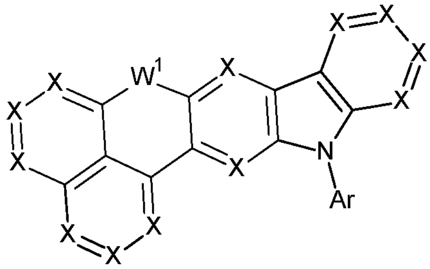

Formel (IIe)

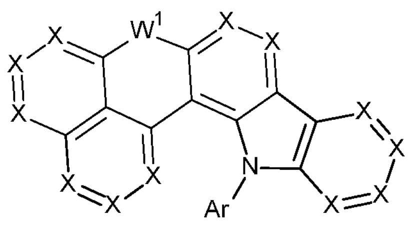

Formel (IIf)

wobei die verwendeten Symbole Ar, $W^1$ und X die in Anspruch 1 genannte Bedeutung aufweisen.

3. Verbindung gemäß Anspruch 1 oder 2, umfassend mindestens eine Struktur der Formel (IIIa), (IIIb), (IIIc), (IIId), (IIIe) oder (IIIf),

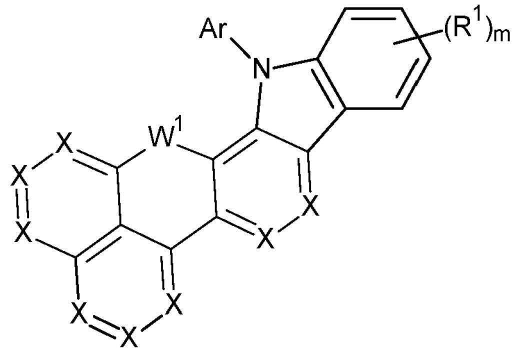

Formel (IIIa)

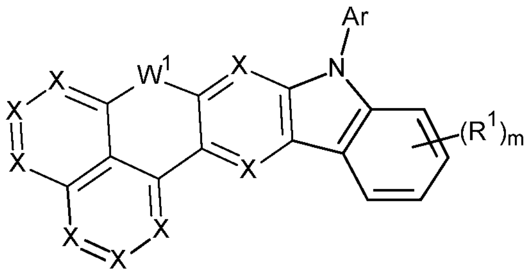

Formel (IIIb)

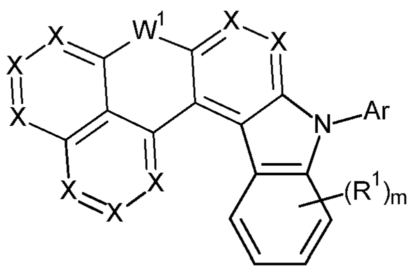

Formel (IIIc)

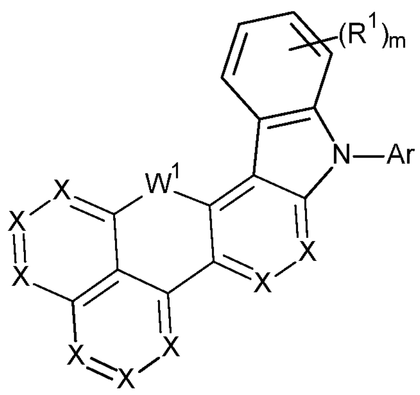

Formel (IIId)

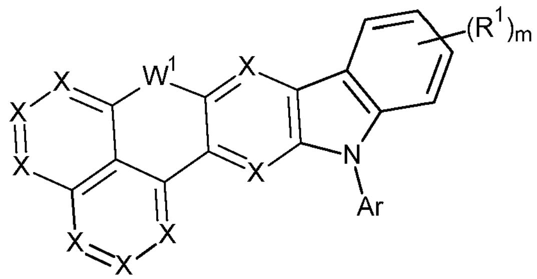

Formel (IIIe)

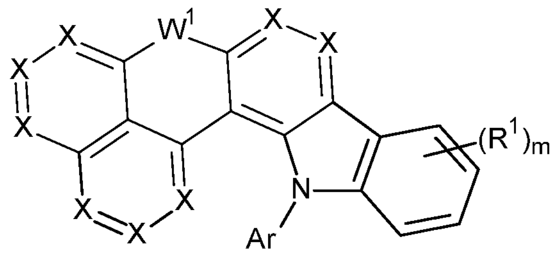

Formel (IIIf)

wobei die Symbole $R^1$, Ar, $W^1$ und X die in Anspruch 1 genannte Bedeutung aufweisen und m 0, 1, 2, 3 oder 4 ist.

**4.** Verbindung gemäß mindestens einem der vorhergehenden Ansprüche, umfassend mindestens eine Struktur der Formel (IVa), (IVb), (IVc), (IVd), (IVe) oder (IVf),

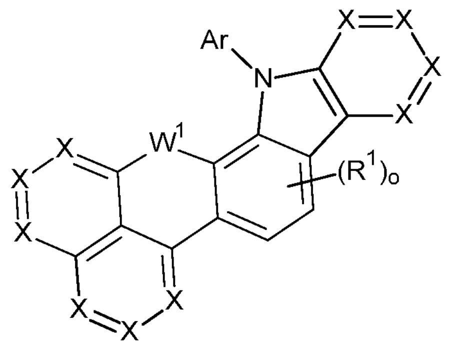

Formel (IVa)

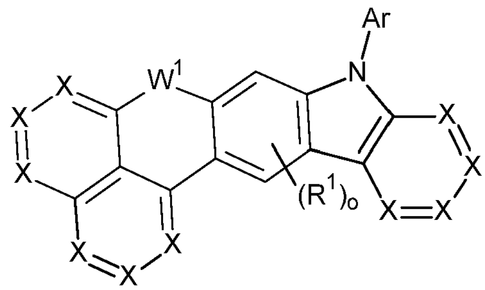

Formel (IVb)

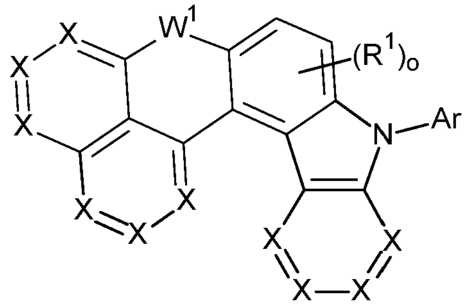

Formel (IVc)

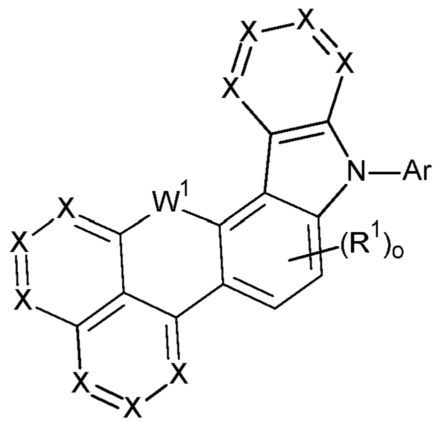

Formel (IVd)

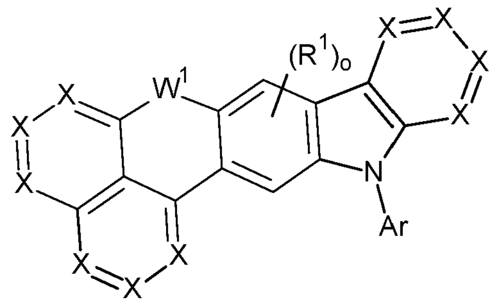

Formel (IVe)

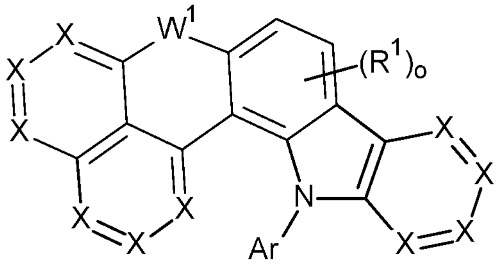

Formel (IVf)

wobei die Symbole $R^1$, Ar, $W^1$ und X die in Anspruch 1 genannte Bedeutung aufweisen und o 0, 1 oder 2 ist.

**5.** Verbindung gemäß mindestens einem der vorhergehenden Ansprüche, umfassend mindestens eine Struktur der Formel (Va), (Vb), (Vc), (Vd), (Ve) oder (Vf),

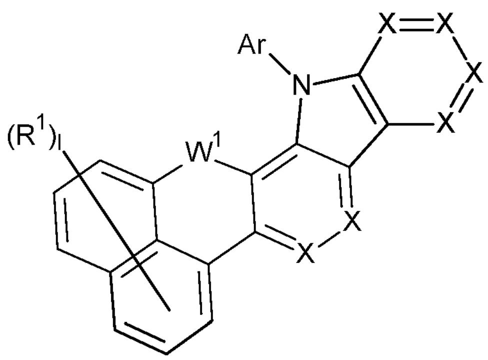

Formel (Va)

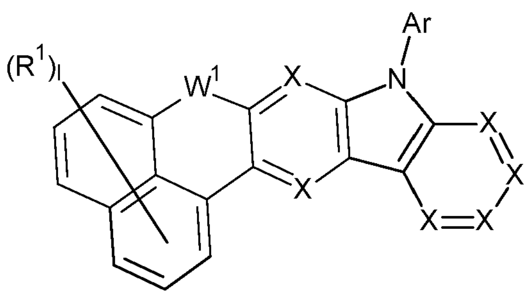

Formel (Vb)

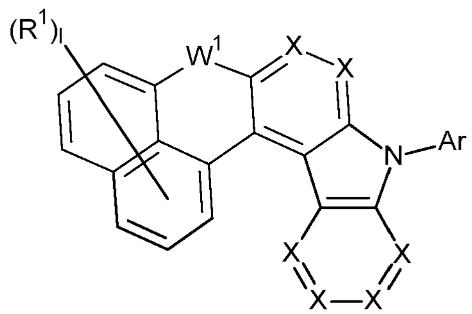

Formel (Vc)

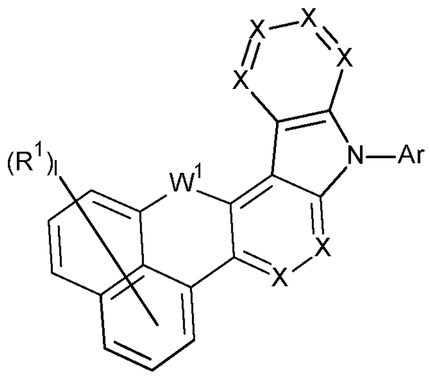

Formel (Vd)

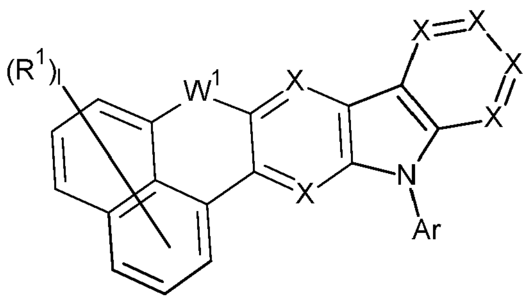

Formel (Ve)

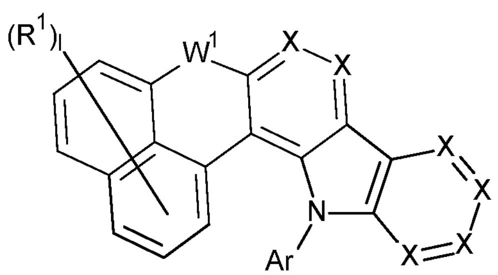

Formel (Vf)

wobei die verwendeten Symbole $R^1$, Ar, $W^1$ und X die in Anspruch 1 genannte Bedeutung aufweisen und l 0, 1, 2, 3, 4, 5 oder 6 ist.

6. Verbindung gemäß mindestens einem der vorhergehenden Ansprüche, umfassend mindestens eine Struktur der Formel (VIa), (VIb), (VIc), (VId), (VIe) oder (VIf),

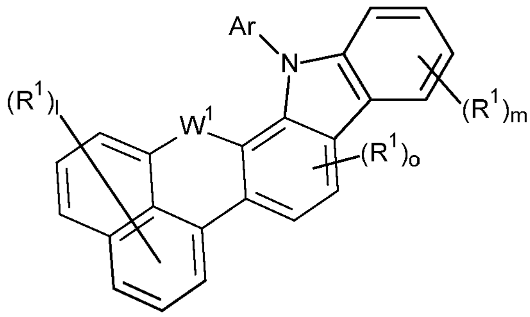

Formel (VIa)

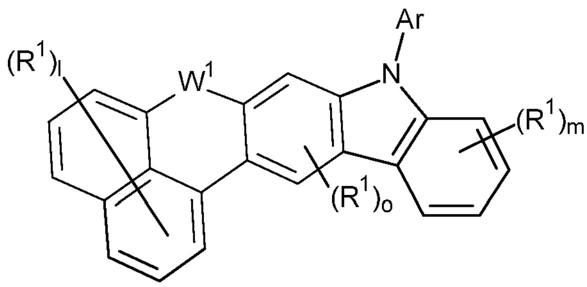

Formel (VIb)

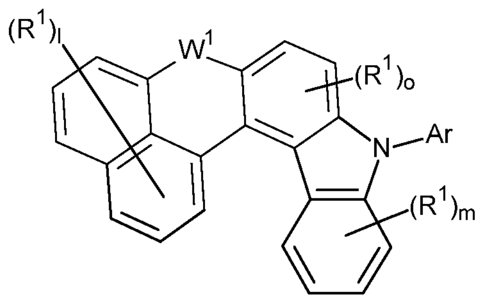

Formel (VIc)

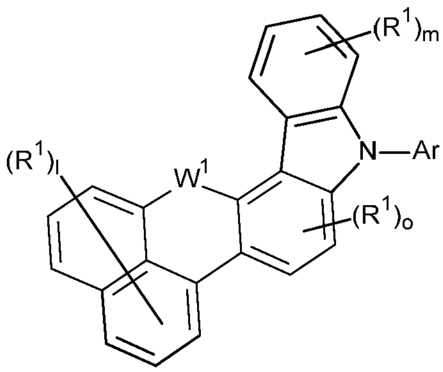

Formel (VId)

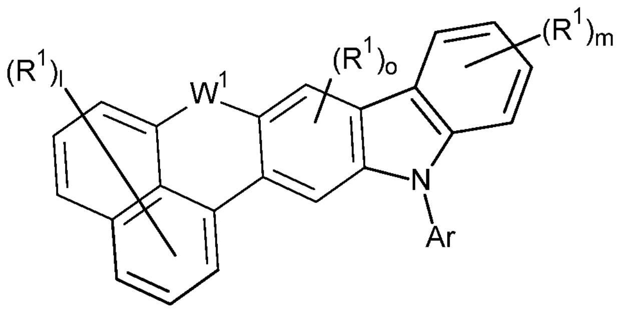

Formel (VIe)

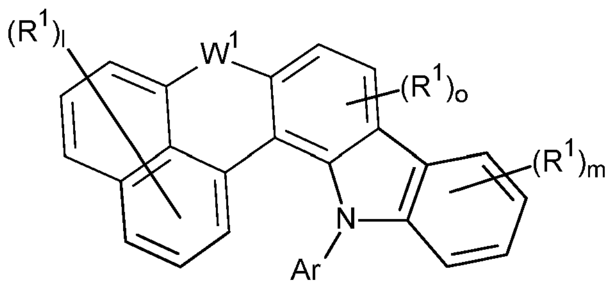

Formel (VIf)

wobei die Symbole $R^1$, Ar, $W^1$ und X die in Anspruch 1 genannte Bedeutung aufweisen, l 0, 1, 2, 3, 4, 5 oder 6, m 0, 1, 2, 3 oder 4 und o 0, 1 oder 2 ist.

7. Verbindung gemäß mindestens einem der vorhergehenden Ansprüche, **dadurch gekennzeichnet, dass** die Gruppe Ar eine Lochtransportgruppe umfasst oder darstellt.

8. Verbindung gemäß mindestens einem der Ansprüche 1 bis 7,
**dadurch gekennzeichnet, dass** die Gruppe Ar eine Elektronentransportgruppe umfasst oder darstellt.

9. Verbindung gemäß Anspruch 8, **dadurch gekennzeichnet, dass** die Gruppe Ar für eine Gruppe steht, die durch die Formel (QL) darstellbar ist,

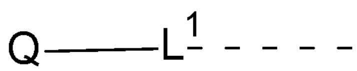

Formel (QL)

worin $L^1$ eine Bindung oder ein aromatisches oder heteroaromatisches Ringsystem mit 5 bis 40 aromatischen Ringatomen darstellt, welches durch einen oder mehrere Reste $R^1$ substituiert sein kann, und Q eine Elektronentransportgruppe ist, wobei der Rest $R^1$ die in Anspruch 1 genannte Bedeutung aufweist.

10. Oligomer, Polymer oder Dendrimer enthaltend eine oder mehrere Verbindungen nach einem der Ansprüche 1 bis 9, wobei statt eines Wasserstoffatoms oder eines Substituenten ein oder mehrere Bindungen der Verbindungen zum Polymer, Oligomer oder Dendrimer vorhanden sind.

11. Zusammensetzung enthaltend wenigstens eine Verbindung nach einem oder mehreren der Ansprüche 1 bis 9 oder ein Oligomer, Polymer oder Dendrimer nach Anspruch 10 und wenigstens eine weitere Verbindung ausgewählt aus der Gruppe bestehend aus fluoreszierenden Emittern, phosphoreszierenden Emittern, Emittern, die TADF zeigen, Hostmaterialien, Elektronentransportmaterialien, Elektroneninjektionsmaterialien, Lochleitermaterialien, Lochinjektionsmaterialien, Elektronenblockiermaterialien und Lochblockiermaterialien.

**12.** Formulierung, enthaltend mindestens eine Verbindung nach einem oder mehreren der Ansprüche 1 bis 9 oder ein Oligomer, Polymer oder Dendrimer nach Anspruch 10 oder eine Zusammensetzung nach Anspruch 11 und mindestens ein Lösemittel.

**13.** Verwendung einer Verbindung nach einem oder mehreren der Ansprüche 1 bis 9, eines Oligomers, Polymers oder Dendrimers nach Anspruch 10 oder einer Zusammensetzung nach Anspruch 11 in einer elektronischen Vorrichtung als Hostmaterial, Lochleitermaterial oder Elektronentransportmaterial.

**14.** Verfahren zur Herstellung einer Verbindung nach einem oder mehreren der Ansprüche 1 bis 9 oder eines Oligomers, Polymers und/oder Dendrimers nach Anspruch 10, **dadurch gekennzeichnet, dass** in einer Kupplungsreaktion eine Verbindung, umfassend mindestens eine stickstoffhaltige heterocyclische Gruppe, mit einer Verbindung, umfassend mindestens eine aromatische oder heteroaromatische Gruppe, verbunden wird.

**15.** Elektronische Vorrichtung, enthaltend mindestens eine Verbindung nach einem oder mehreren der Ansprüche 1 bis 9, ein Oligomer, Polymer oder Dendrimer nach Anspruch 10 oder eine Zusammensetzung gemäß Anspruch 11, wobei die elektronische Vorrichtung bevorzugt ausgewählt ist aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen optischen Detektoren, organischen Photorezeptoren, organischen Feld-Quench-Devices, lichtemittierenden elektrochemischen Zellen oder organischen Laserdioden.

## Claims

**1.** Compound comprising at least one structure of the formula (I),

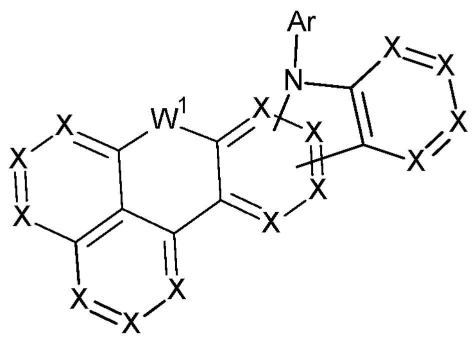

formula (I)

where the following applies to the symbols used:

X is on each occurrence, identically or differently, N or $CR^1$, preferably $CR^1$, or C if the indolo group is bonded to X;
$W^1$ is O, S, $C(R^1)_2$, or $Si(R^1)_2$;
$R^1$ is on each occurrence, identically or differently, H, D, F, Cl, Br, I, CN, $NO_2$, $N(Ar^1)_2$, $N(R^2)_2$, $C(=O)Ar^1$, $C(=O)R^2$, $P(=O)(Ar^1)_2$, $P(Ar^1)_2$, $B(Ar^1)_2$, $B(OR^2)_2$, $Si(Ar^1)_3$, $Si(R^2)_3$, a straight-chain alkyl, alkoxy or thioalkoxy group having 1 to 40 C atoms or a branched or cyclic alkyl, alkoxy or thioalkoxy group having 3 to 40 C atoms or an alkenyl group having 2 to 40 C atoms, which also includes cyclopentenyl, cyclohexenyl, cycloheptenyl, cyclooctenyl and cyclooctadienyl, which may in each case be substituted by one or more radicals $R^2$, where one or more non-adjacent $CH_2$ groups may be replaced by $-R^2C=CR^2-$, -C=C-, $Si(R^2)_2$, $Ge(R^2)_2$, $Sn(R^2)_2$, C=O, C=S, C=Se, $C=NR^2$, -C(=O)O-, $-C(=O)NR^2-$, $NR^2$, $P(=O)(R^2)$, -O-, -S-, SO or $SO_2$ and where one or more H atoms may be replaced by D, F, Cl, Br, I, CN or $NO_2$, or an aromatic or heteroaromatic ring system having 5 to 40 aromatic ring atoms, which may in each case be substituted by one or more radicals $R^2$, or an aryloxy or heteroaryloxy group having 5 to 40 aromatic ring atoms, which may be substituted by one or more radicals $R^2$, or an aralkyl or heteroaralkyl group having 5 to 40 aromatic ring atoms, which may be substituted by one or more radicals $R^2$, or a combination of these systems;

Ar is on each occurrence, identically or differently, an aromatic or heteroaromatic ring system having 5 to 60 aromatic ring atoms, which may be substituted by one or more radicals $R^1$;

$Ar^1$ is on each occurrence, identically or differently, an aromatic or heteroaromatic ring system having 5 to 30 aromatic ring atoms, which may be substituted by one or more radicals $R^2$, two radicals $Ar^1$ that are bonded to the same Si atom, N atom, P atom or B atom may also be bridged to one another by a single bond or a bridge selected from $B(R^2)$, $C(R^2)_2$, $Si(R^2)_2$, C=O, $C=NR^2$, $C=C(R^2)_2$, O, S, S=O, $SO_2$, $N(R^2)$, $P(R^2)$ and $P(=O)R^2$;

$R^2$ is on each occurrence, identically or differently, H, D, F, Cl, Br, I, CN, $B(OR^3)_2$, $NO_2$, $C(=O)R^3$, $CR^3=C(R^3)_2$, $C(=O)OR^3$, $C(=O)N(R^3)_2$, $Si(R^3)_3$, $P(R^3)_2$, $B(R^3)_2$, $N(R^3)_2$, $NO_2$, $P(=O)(R^3)_2$, $OSO_2R^3$, $OR^3$, $S(=O)R^3$, $S(=O)_2R^3$, a straight-chain alkyl, alkoxy or thioalkoxy group having 1 to 40 C atoms or a branched or cyclic alkyl, alkoxy or thioalkoxy group having 3 to 40 C atoms, which may in each case be substituted by one or more radicals $R^3$, where one or more non-adjacent $CH_2$ groups may be replaced by $-R^3C=CR^3-$, -C=C-, $Si(R^3)_2$, $Ge(R^3)_2$, $Sn(R^3)_2$, C=O, C=S, $C=NR^3$, -C(=O)O-, $-C(=O)NR^3-$, $NR^3$, $P(=O)(R^3)$, -O-, -S-, SO or $SO_2$ and where one or more H atoms may be replaced by D, F, Cl, Br, I, CN or $NO_2$, or an aromatic or heteroaromatic ring system having 5 to 40 aromatic ring atoms, which may in each case be substituted by one or more radicals $R^3$, or an aryloxy or heteroaryloxy group having 5 to 40 aromatic ring atoms, which may be substituted by one or more radicals $R^3$, or a combination of these systems; two or more, preferably adjacent, substituents $R^2$ may also form a mono- or polycyclic, aliphatic, hetero-aliphatic, aromatic or heteroaromatic ring system with one another;

$R^3$ is selected on each occurrence, identically or differently, from the group consisting of H, D, F, CN, an aliphatic hydrocarbon radical having 1 to 20 C atoms or an aromatic or heteroaromatic ring system having 5 to 30 aromatic ring atoms, in which one or more H atoms may be replaced by D, F, Cl, Br, I or CN and which may be substituted by one or more alkyl groups, in each case having 1 to 4 carbon atoms, which also includes cyclopropyl and cyclobutyl, two or more, preferably adjacent, substituents $R^3$ may also form a mono- or polycyclic, aliphatic, hetero-aliphatic, aromatic or heteroaromatic ring system with one another.

**2.** Compound according to Claim 1, comprising at least one structure of the formula (IIa), (IIb), (IIc), (IId), (IIe) or (IIf),

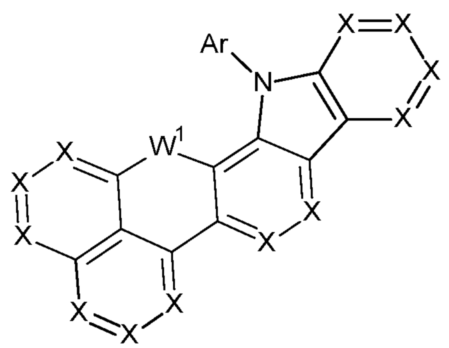

formula (IIa)

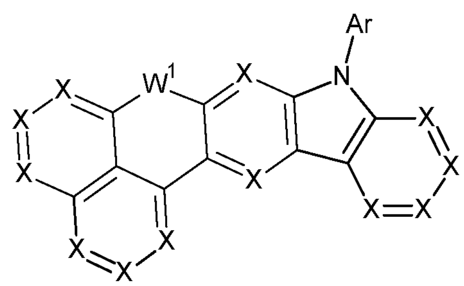

formula (IIb)

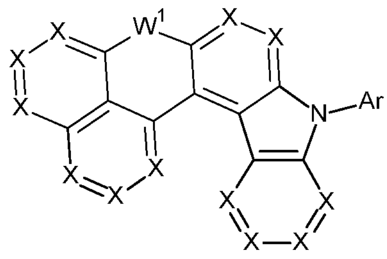

formula (IIc)

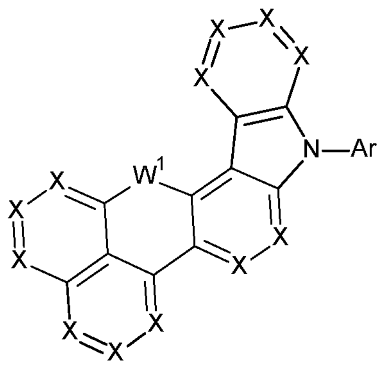

formula (IId)

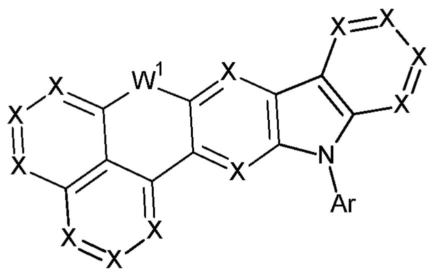

formula (IIe)

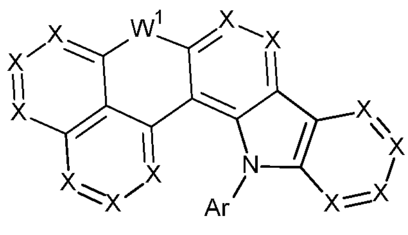

formula (IIf)

where the symbols Ar, $W^1$ and X used have the meaning given in Claim 1.

**3.** Compound according to Claim 1 or 2, comprising at least one structure of the formula (IIIa), (IIIb), (IIIc), (IIId), (IIIe) or (IIIf),

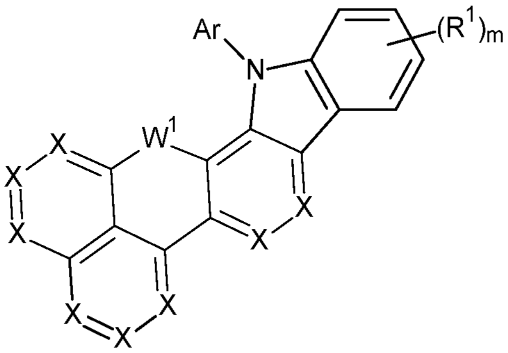

formula (IIIa)

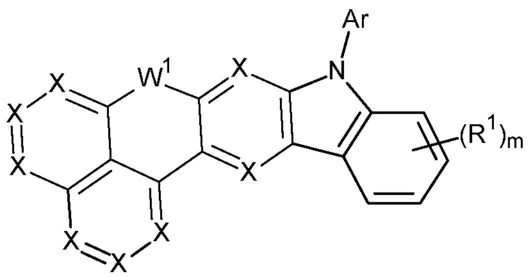

formula (IIIb)

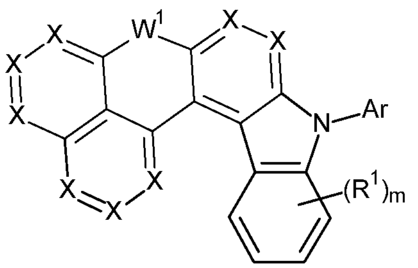

formula (IIIc)

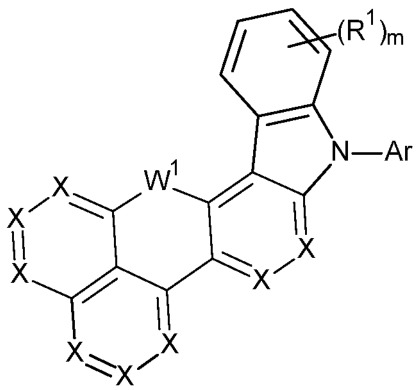

formula (IIId)

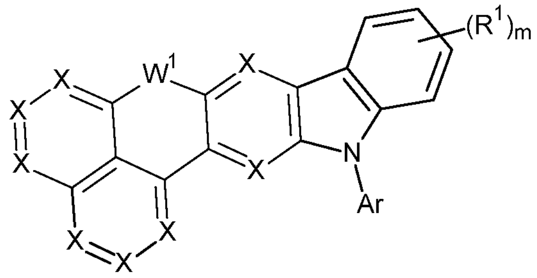

formula (IIIe)

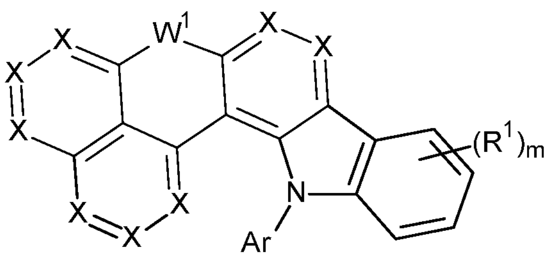

formula (IIIf)

where the symbols $R^1$, Ar, W' and X have the meaning given in Claim 1 and m is 0, 1, 2, 3 or 4.

**4.** Compound according to at least one of the preceding claims, comprising at least one structure of the formula (IVa), (IVb), (IVc), (IVd), (IVe) or (IVf),

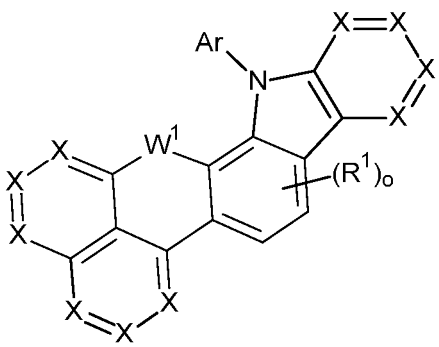

formula (IVa)

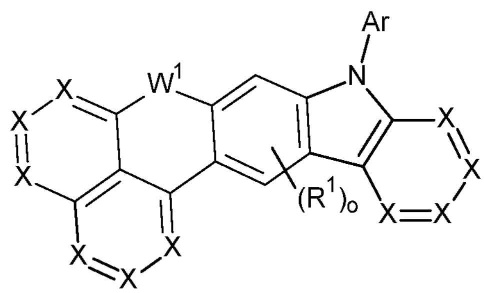

formula (IVb)

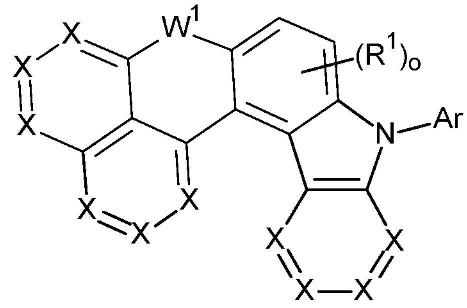

formula (IVc)

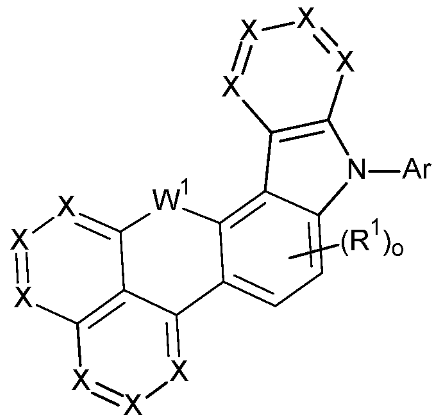

formula (IVd)

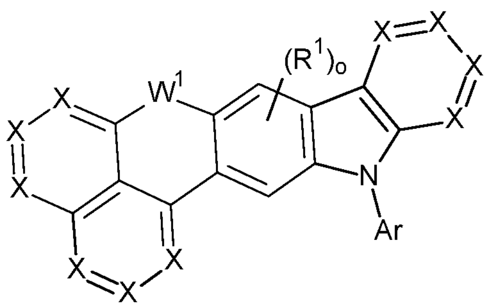

formula (IVe)

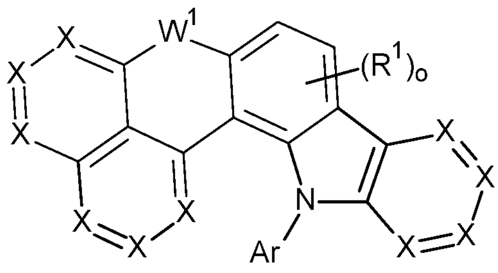

formula (IVf)

where the symbols $R^1$, Ar, W' and X have the meaning given in Claim 1 and o is 0, 1 or 2.

**5.** Compound according to at least one of the preceding claims, comprising at least one structure of the formula (Va),

(Vb), (Vc), (Vd), (Ve) or (Vf),

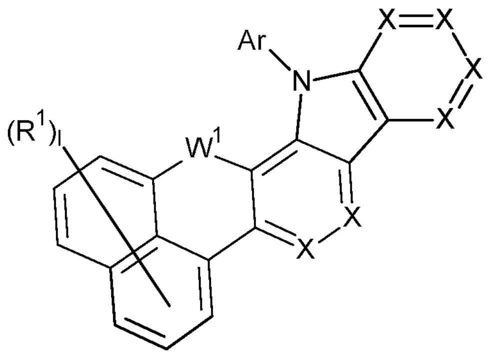

formula (Va)

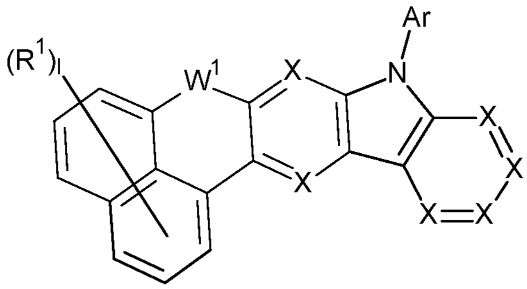

formula (Vb)

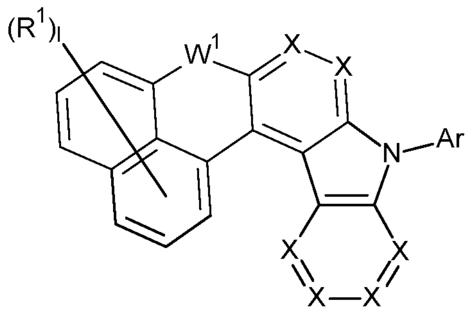

formula (Vc)

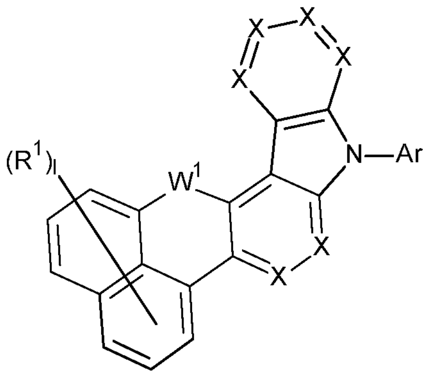

formula (Vd)

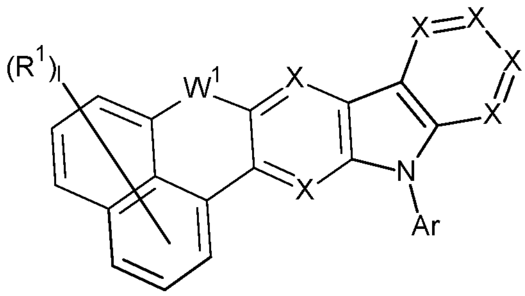

formula (Ve)

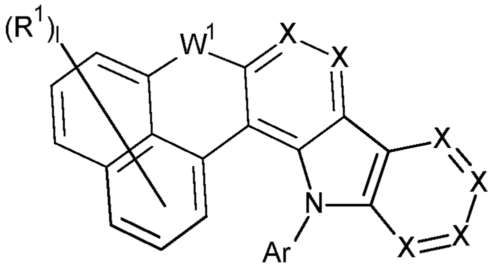

formula (Vf)

where the symbols $R^1$, Ar, W' and X used have the meaning given in Claim 1 and l is 0, 1, 2, 3, 4, 5 or 6.

**6.** Compound according to at least one of the preceding claims, comprising at least one structure of the formula (VIa), (VIb), (VIc), (VId), (VIe) or (VIf),

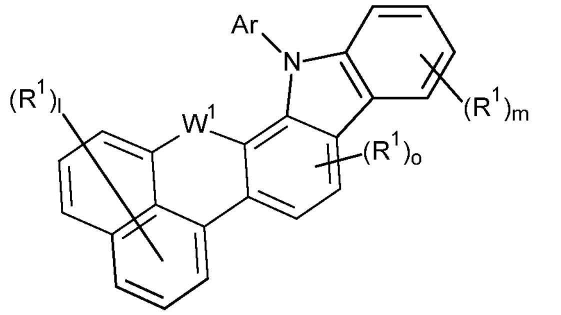

formula (VIa)

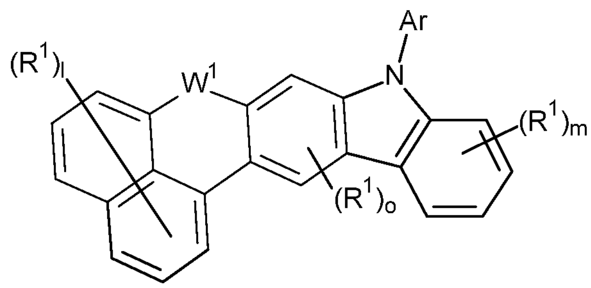

formula (VIb)

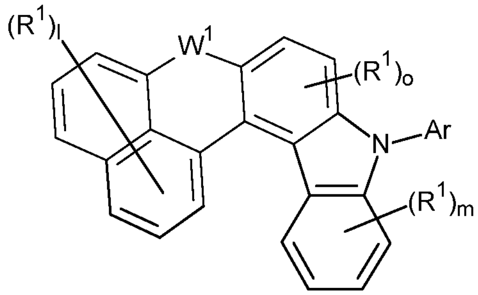

formula (VIc)

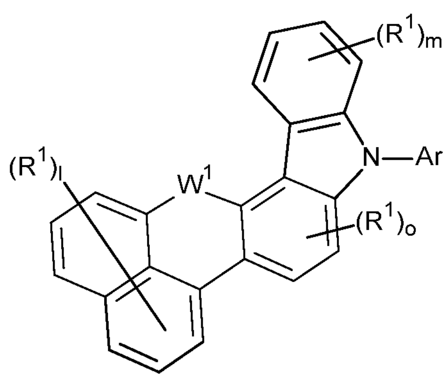

formula (VId)

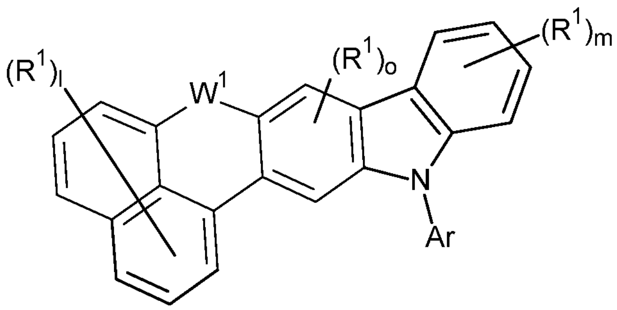

formula (VIe)

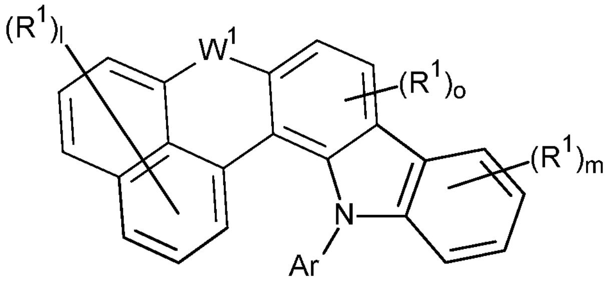

formula (VIf)

where the symbols $R^1$, Ar, W' and X have the meaning given in Claim 1, l is 0, 1, 2, 3, 4, 5 or 6, m is 0, 1, 2, 3 or 4 and o is 0, 1 or 2.

**7.** Compound according to at least one of the preceding claims, **characterised in that** the group Ar comprises or represents a hole-transport group.

**8.** Compound according to at least one of Claims 1 to 7, **characterised in that** the group Ar comprises or represents an electron-transport group.

**9.** Compound according to Claim 8, **characterised in that** the group Ar stands for a group which can be represented by the formula (QL),

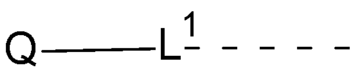

formula (QL)

in which $L^1$ represents a bond or an aromatic or heteroaromatic ring system having 5 to 40 aromatic ring atoms, which may be substituted by one or more radicals $R^1$, and Q is an electron-transport group, where the radical $R^1$ has the meaning given in Claim 1.

**10.** Oligomer, polymer or dendrimer containing one or more compounds according to one of Claims 1 to 9, in which, instead of a hydrogen atom or a substituent, one or more bonds are present from the compounds to the polymer, oligomer or dendrimer.

**11.** Composition comprising at least one compound according to one or more of Claims 1 to 9 or an oligomer, polymer or dendrimer according to Claim 10 and at least one further compound selected from the group consisting of fluorescent emitters, phosphorescent emitters, emitters which exhibit TADF, host materials, electron-transport materials, electron-injection materials, hole-conductor materials, hole-injection materials, electron-blocking materials and hole-blocking materials.

**12.** Formulation comprising at least one compound according to one or more of Claims 1 to 9 or an oligomer, polymer or dendrimer according to Claim 10 or a composition according to Claim 11 and at least one solvent.

**13.** Use of a compound according to one or more of Claims 1 to 9, an oligomer, polymer or dendrimer according to Claim 10 or a composition according to Claim 11 in an electronic device as host material, hole-conductor material or electron-transport material.

**14.** Process for the preparation of a compound according to one or more of Claims 1 to 9 or an oligomer, polymer or dendrimer according to Claim 10, **characterised in that** a compound comprising at least one nitrogen-containing heterocyclic group is connected to a compound comprising at least one aromatic or heteroaromatic group in a coupling reaction.

**15.** Electronic device containing at least one compound according to one or more of Claims 1 to 9, an oligomer, polymer or dendrimer according to Claim 10 or a composition according to Claim 11, where the electronic device is preferably selected from the group consisting of organic electroluminescent devices, organic integrated circuits, organic field-effect transistors, organic thin-film transistors, organic light-emitting transistors, organic solar cells, organic optical detectors, organic photoreceptors, organic field-quench devices, light-emitting electrochemical cells or organic laser diodes.

**Revendications**

**1.** Composé comprenant au moins une structure de formule (I),

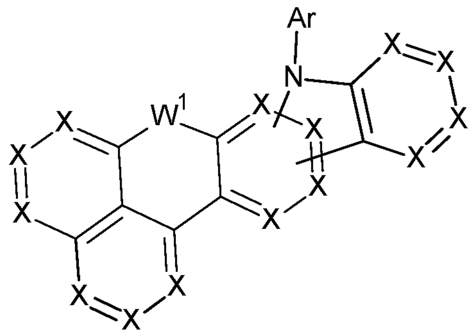

formule (I)

dans laquelle ce qui suit s'applique aux symboles utilisés :

X est à chaque occurrence, de manière identique ou différente, N ou $CR^1$, préférablement $CR^1$, ou C si le groupement indolo est lié à X ;
$W^1$ est O, S, $C(R^1)_2$, ou $Si(R^1)_2$ ;
$R^1$ est à chaque occurrence, de manière identique ou différente, H, D, F, Cl, Br, I, CN, $NO_2$, $N(Ar^1)_2$, $N(R^2)_2$, $C(=O)Ar^1$, $C(=O)R^2$, $P(=O)(Ar^1)_2$, $P(Ar^1)_2$, $B(Ar^1)_2$, $B(OR^2)_2$, $Si(Ar^1)_3$, $Si(R^2)_3$, un groupement alkyle, alcoxy ou thioalcoxy à chaîne linéaire ayant de 1 à 40 atomes de C ou un groupement alkyle, alcoxy ou thio-alcoxy ramifié ou cyclique ayant de 3 à 40 atomes de C ou un groupement alcényle ayant de 2 à 40 atomes de C, qui peut également comporter cyclopentényle, cyclohexényle, cyclo-heptényle, cyclooctényle et cyclooctadiényle, qui peut dans chaque cas être substitué par un ou plusieurs radicaux $R^2$, où un ou plusieurs groupements $CH_2$ non adjacents peuvent être remplacés par $-R^2C=CR^2-$, -C=C-, $Si(R^2)_2$, $Ge(R^2)_2$, $Sn(R^2)_2$, C=O, C=S, C=Se, $C=NR^2$, -C(=O)O-, $-C(=O)NR^2-$, $NR^2$, $P(=O)(R^2)$, -O-, -S-, SO ou $SO_2$ et où un ou plusieurs atomes de H peuvent être remplacés par D, F, Cl, Br, I, CN ou $NO_2$, ou un noyau aromatique ou hétéroaromatique ayant de 5 à 40 atomes de cycle aromatique, qui peut dans chaque cas être substitué par un ou plusieurs radicaux $R^2$, ou un groupement aryloxy ou hétéroaryloxy ayant de 5 à 40 atomes de cycle aromatique, qui peut être substitué par un ou plusieurs radicaux $R^2$, ou un groupement aralkyle ou hétéroaralkyle ayant de 5 à 40 atomes de cycle aromatique, qui peut être substitué par un ou plusieurs radicaux $R^2$, ou une combinaison de ces systèmes ;

Ar est à chaque occurrence, de manière identique ou différente, un noyau aromatique ou hétéroaromatique ayant de 5 à 60 atomes de cycle aromatique, qui peut être substitué par un ou plusieurs radicaux $R^1$ ;

$Ar^1$ est à chaque occurrence, de manière identique ou différente, un noyau aromatique ou hétéroaromatique ayant de 5 à 30 atomes de cycle aromatique, qui peut être substitué par un ou plusieurs radicaux $R^2$, deux radicaux $Ar^1$ qui sont liés au même atome de Si, atome de N, atome de P ou atome de B peuvent également être pontés l'un avec l'autre par une liaison simple ou un pont choisi parmi $B(R^2)$, $C(R^2)_2$, $Si(R^2)_2$, C=O, $C=NR^2$, $C=C(R^2)_2$, O, S, S=O, $SO_2$, $N(R^2)$, $P(R^2)$ et $P(=O)R^2$ ;

$R^2$ est à chaque occurrence, de manière identique ou différente, H, D, F, Cl, Br, I, CN, $B(OR^3)_2$, $NO_2$, $C(=O)R^3$, $CR^3=C(R^3)_2$, $C(=O)OR^3$, $C(=O)N(R^3)_2$, $Si(R^3)_3$, $P(R^3)_2$, $B(R^3)_2$, $N(R^3)_2$, $NO_2$, $P(=O)(R^3)_2$, $OSO_2R^3$, $OR^3$, $S(=O)R^3$, $S(=O)_2R^3$, un groupement alkyle, alcoxy ou thioalcoxy à chaîne linéaire ayant de 1 à 40 atomes de C ou un groupement alkyle, alcoxy ou thioalcoxy ramifié ou cyclique ayant de 3 à 40 atomes de C, qui peut dans chaque cas être substitué par un ou plusieurs radicaux $R^3$, où un ou plusieurs groupements $CH_2$ non adjacents peuvent être remplacés par $-R^3C=CR^3-$, -C=C-, $Si(R^3)_2$, $Ge(R^3)_2$, $Sn(R^3)_2$, C=O, C=S, $C=NR^3$, -C(=O)O-, $-C(=O)NR^3-$, $NR^3$, $P(=O)(R^3)$, -O-, -S-, SO ou $SO_2$ et où un ou plusieurs atomes de H peuvent être remplacés par D, F, Cl, Br, I, CN ou $NO_2$, ou un noyau aromatique ou hétéroaromatique ayant de 5 à 40 atomes de cycle aromatique, qui peut dans chaque cas être substitué par un ou plusieurs radicaux $R^3$, ou un groupement aryloxy ou hétéroaryloxy ayant de 5 à 40 atomes de cycle aromatique, qui peut être substitué par un ou plusieurs radicaux $R^3$, ou une combinaison de ces systèmes ; deux ou plus, substituants $R^2$, préférablement adjacents, peuvent également former un noyau mono- ou polycyclique, aliphatique, hétéroaliphatique, aromatique ou hétéroaromatique les uns avec les autres ;

$R^3$ est choisi à chaque occurrence, de manière identique ou différente, dans le groupe constitué par H, D, F, CN, un radical hydrocarboné aliphatique ayant de 1 à 20 atomes de C ou un noyau aromatique ou hétéroaromatique ayant de 5 à 30 atomes de cycle aromatique, où un ou plusieurs atomes de H peuvent être remplacés par D, F, Cl, Br, I ou CN et qui peut être substitué par un ou plusieurs groupements alkyle, dans chaque cas ayant de 1 à 4 atomes de carbone, pouvant également comporter cyclopropyle et cyclobutyle, deux ou plus, substituants $R^3$, préférablement adjacents, peuvent également former un noyau mono- ou polycyclique, aliphatique, hétéroaliphatique, aromatique ou hétéroaromatique les uns avec les autres.

2. Composé selon la revendication 1, comprenant au moins une structure de formule (IIa), (IIb), (IIc), (IId), (IIe) ou (IIf),

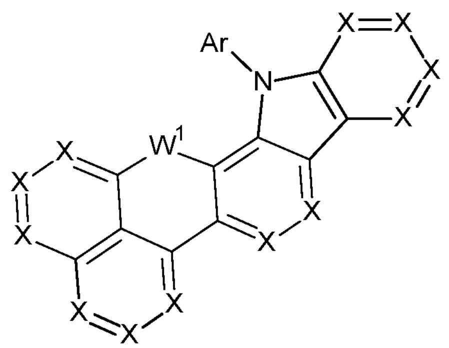

formule (IIa)

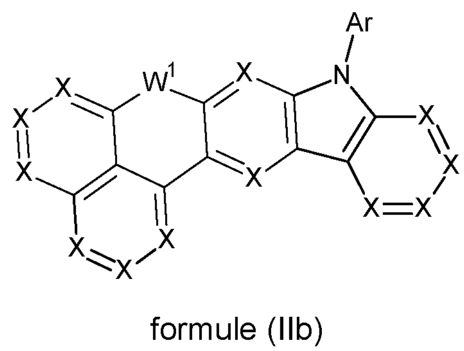

formule (IIb)

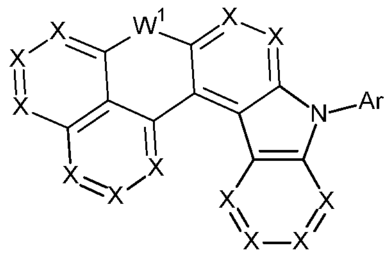

formule (IIc)

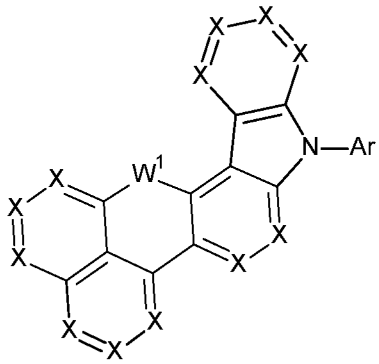

formule (IId)

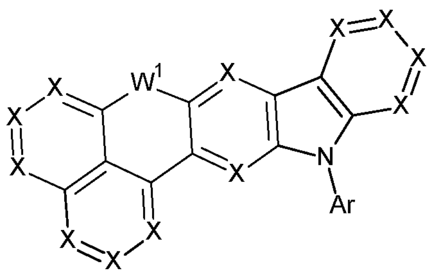

formule (IIe)

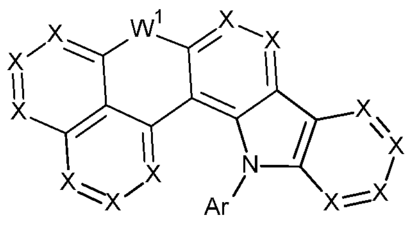

formule (IIf)

dans laquelle les symboles Ar, W' et X utilisés revêtent la signification donnée selon la revendication 1.

**3.** Composé selon la revendication 1 ou 2, comprenant au moins une structure de formule (IIIa), (IIIb), (IIIc), (IIId), (IIIe) ou (IIIf),

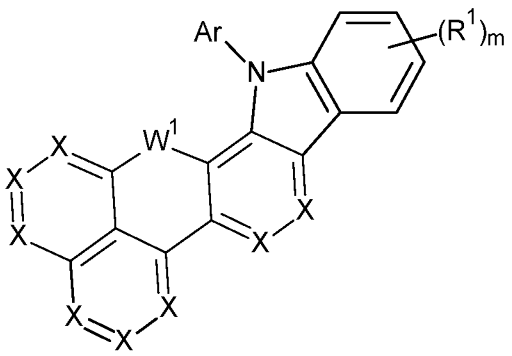

formule (IIIa)

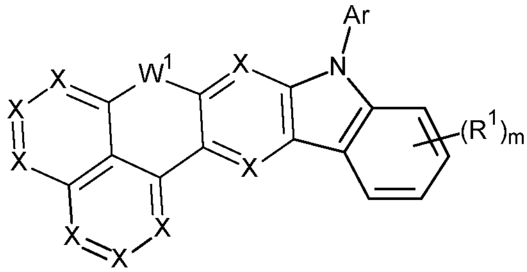

formule (IIIb)

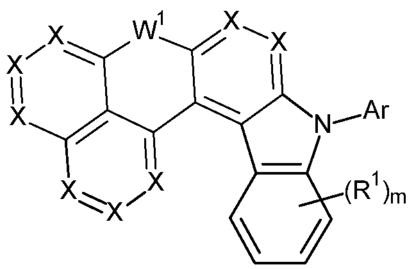

formule (IIIc)

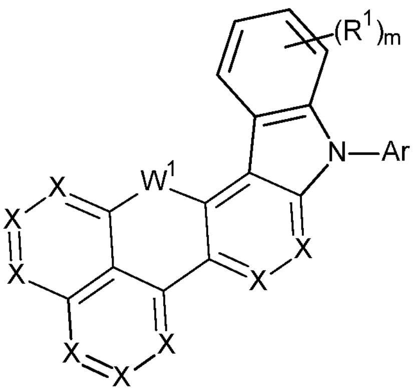

formule (IIId)

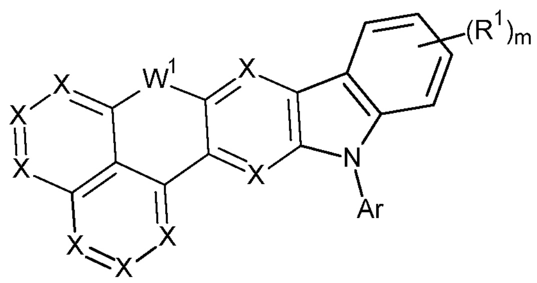

formule (IIIe)

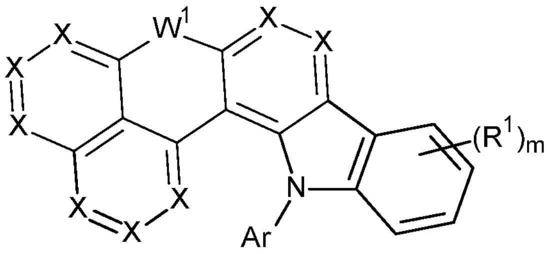

formule (IIIf)

dans laquelle les symboles $R^1$, Ar, W' et X revêtent la signification donnée selon la revendication 1 et m vaut 0, 1, 2, 3 ou 4.

4. Composé selon au moins l'une parmi les revendications précédentes, comprenant au moins une structure de formule (IVa), (IVb), (IVc), (IVd), (IVe) ou (IVf),

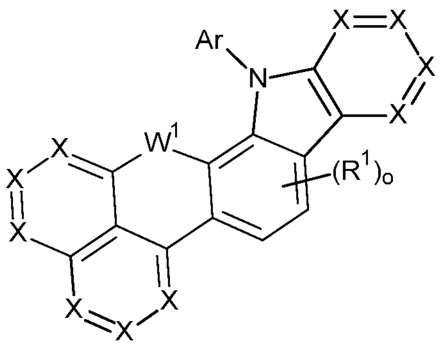

formule (IVa)

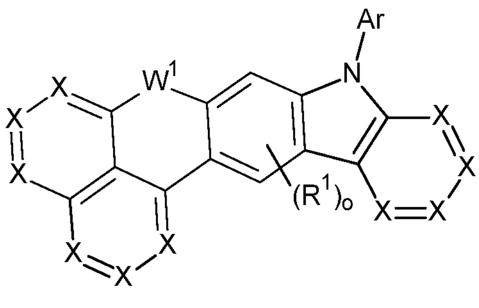

formule (IVb)

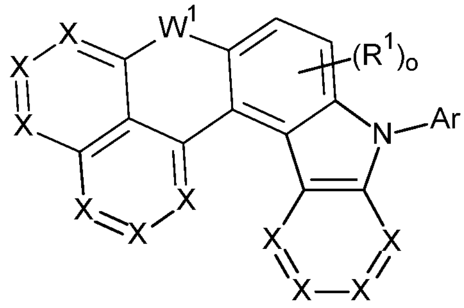

formule (IVc)

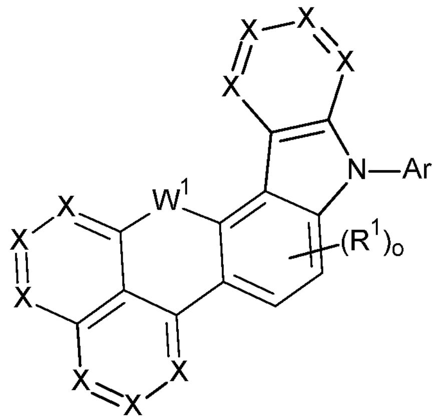

formule (IVd)

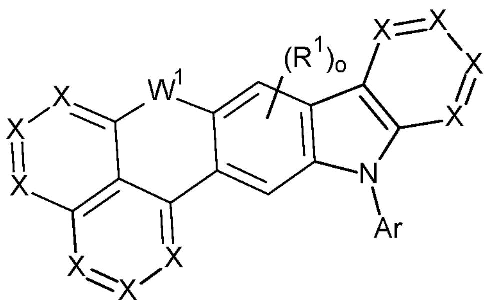

formule (IVe)

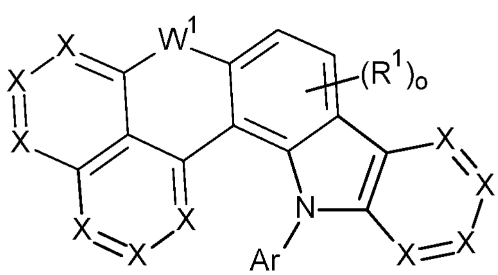

formule (IVf)

dans laquelle les symboles $R^1$, Ar, W' et X revêtent la signification donnée selon la revendication 1 et o vaut 0, 1 ou 2.

**5.** Composé selon au moins l'une parmi les revendications précédentes, comprenant au moins une structure de formule

(Va), (Vb), (Vc), (Vd), (Ve) ou (Vf),

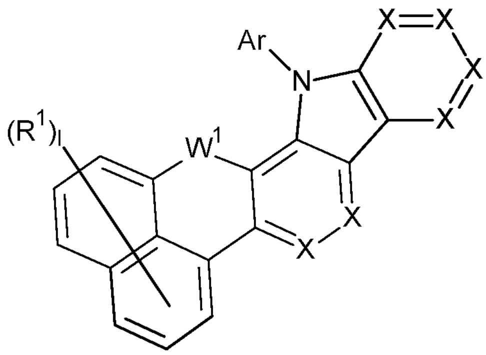

formule (Va)

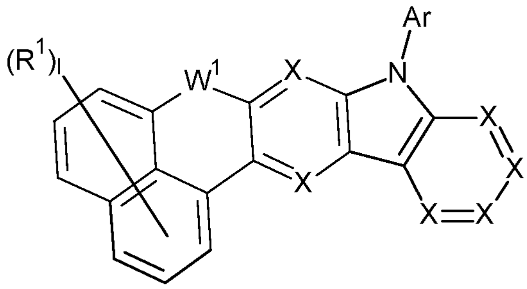

formule (Vb)

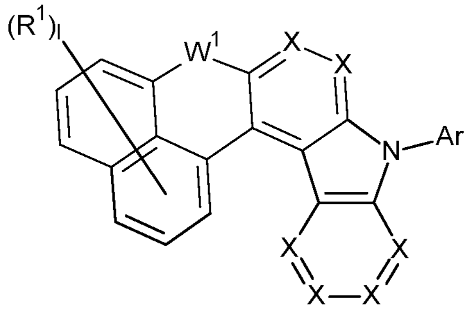

formule (Vc)

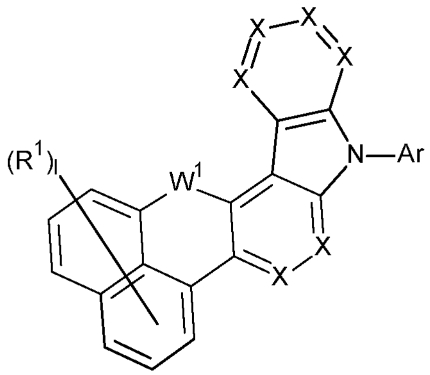

formule (Vd)

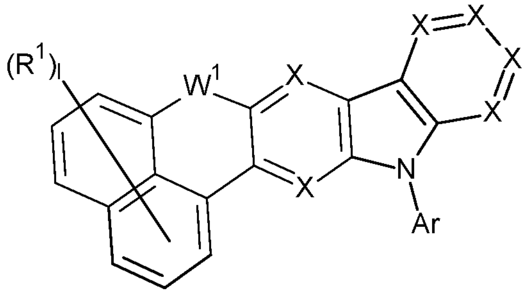

formule (Ve)

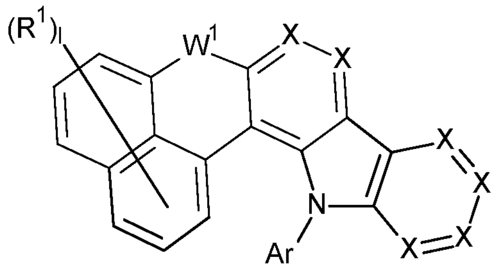

formule (Vf)

dans laquelle les symboles $R^1$, Ar, W' et X utilisés revêtent la signification donnée selon la revendication 1 et l vaut 0, 1, 2, 3, 4, 5 ou 6.

**6.** Composé selon au moins l'une parmi les revendications précédentes, comprenant au moins une structure de formule (VIa), (VIb), (VIc), (VId), (VIe) ou (VIf),

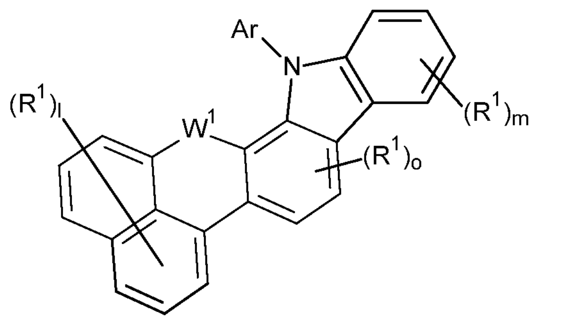

formule (VIa)

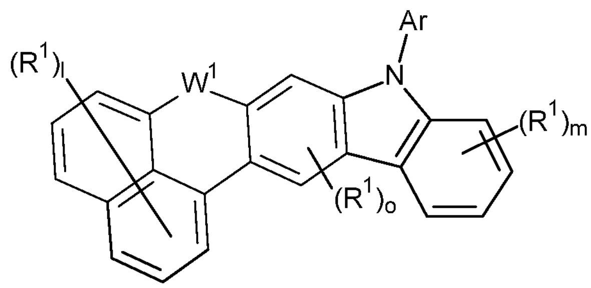

formule (VIb)

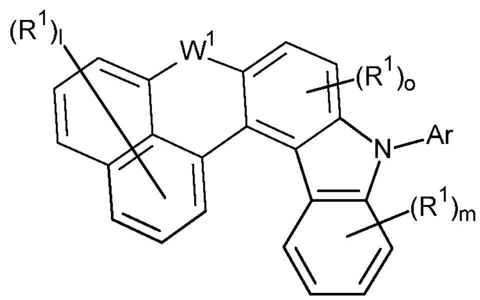

formule (VIc)

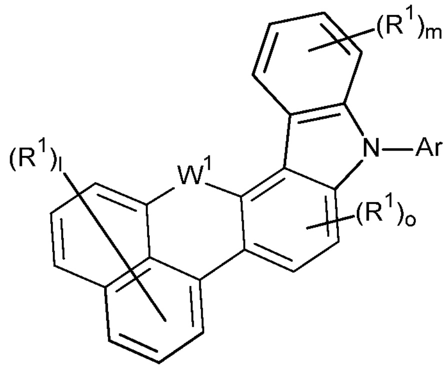

formule (VId)

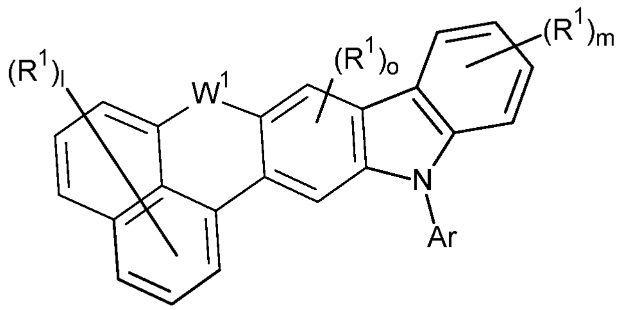

formule (VIe)

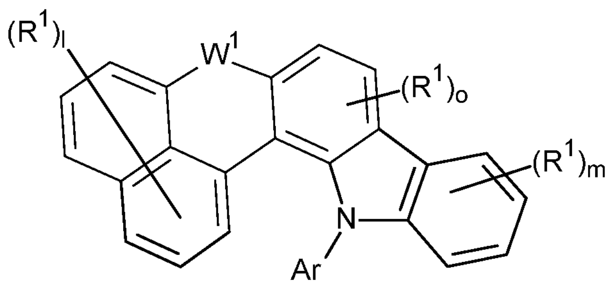

formule (VIf)

dans laquelle les symboles $R^1$, Ar, W' et X revêtent la signification donnée selon la revendication 1, l vaut 0, 1, 2, 3, 4, 5 ou 6, m vaut 0, 1, 2, 3 ou 4 et o vaut 0, 1 ou 2.

**7.** Composé selon au moins l'une parmi les revendications précédentes, **caractérisé en ce que** le groupement Ar comprend ou représente un groupement de transport de trous.

**8.** Composé selon au moins l'une parmi les revendications 1 à 7, **caractérisé en ce que** le groupement Ar comprend ou représente un groupement de transport d'électrons.

**9.** Composé selon la revendication 8, **caractérisé en ce que** le groupement Ar représente un groupement qui peut être représenté par la formule (QL),

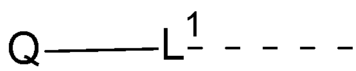

formule (QL)

dans laquelle L' représente une liaison ou un noyau aromatique ou hétéroaromatique ayant de 5 à 40 atomes de cycle aromatique, qui peut être substitué par un ou plusieurs radicaux $R^1$, et Q est un groupement de transport d'électrons, où le radical $R^1$ revêt la signification donnée selon la revendication 1.

**10.** Oligomère, polymère ou dendrimère contenant un ou plusieurs composés selon l'une parmi les revendications 1 à 9, où, au lieu d'un atome d'hydrogène ou d'un substituant, une ou plusieurs liaisons sont présentes entre les composés et le polymère, l'oligomère ou le dendrimère.

**11.** Composition comprenant au moins un composé selon l'une ou plusieurs parmi les revendications 1 à 9 ou un oligomère, polymère ou dendrimère selon la revendication 10 et au moins un autre composé choisi dans le groupe constitué par les émetteurs fluorescents, les émetteurs phosphorescents, les émetteurs présentant une TADF, les matériaux hôtes, les matériaux de transport d'électrons, les matériaux d'injection d'électrons, les matériaux conducteurs de trous, les matériaux d'injection de trous, les matériaux de blocage d'électrons et les matériaux de blocage de trous.

**12.** Formulation comprenant au moins un composé selon l'une ou plusieurs parmi les revendications 1 à 9 ou un oligomère, polymère ou dendrimère selon la revendication 10 ou une composition selon la revendication 11 et au moins un solvant.

**13.** Utilisation d'un composé selon l'une ou plusieurs parmi les revendications 1 à 9, d'un oligomère, polymère ou dendrimère selon la revendication 10 ou d'une composition selon la revendication 11, dans un dispositif électronique comme matériau hôte, matériau conducteur de trous ou matériau de transport d'électrons.

**14.** Procédé de préparation d'un composé selon l'une ou plusieurs parmi les revendications 1 à 9 ou d'un oligomère, polymère ou dendrimère selon la revendication 10, **caractérisé en ce qu'**un composé comprenant au moins un groupement hétérocyclique azoté est relié à un composé comprenant au moins un groupement aromatique ou hétéroaromatique dans une réaction de couplage.

**15.** Dispositif électronique contenant au moins un composé selon l'une ou plusieurs parmi les revendications 1 à 9, un oligomère, polymère ou dendrimère selon la revendication 10 ou une composition selon la revendication 11, le dispositif électronique étant préférablement choisi dans le groupe constitué par les dispositifs électroluminescents organiques, les circuits intégrés organiques, les transistors organiques à effet de champ, les transistors organiques en couche mince, les transistors organiques émetteurs de lumière, les cellules solaires organiques, les détecteurs optiques organiques, les photorécepteurs organiques, les dispositifs organiques à extinction de champ, les cellules électrochimiques émettrices de lumière ou les diodes laser organiques.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- KR 20150065383
- KR 20160062603 A
- KR 20110113468 A
- EP 842208 A
- WO 2000022026 A
- EP 707020 A
- EP 894107 A
- WO 2006061181 A
- WO 9218552 A
- WO 2004070772 A
- WO 2004113468 A
- EP 1028136 A
- WO 2005014689 A
- WO 2004041901 A
- WO 2004113412 A
- WO 2005040302 A
- WO 2005104264 A
- WO 2007017066 A
- US 7294849 B
- WO 0070655 A
- WO 200141512 A
- WO 200202714 A
- WO 200215645 A
- EP 1191613 A
- EP 1191612 A
- EP 1191614 A
- WO 05033244 A
- WO 05019373 A
- US 20050258742 A
- WO 2009146770 A
- WO 2010015307 A
- WO 2010031485 A
- WO 2010054731 A
- WO 2010054728 A
- WO 2010086089 A
- WO 2010099852 A
- WO 2010102709 A
- WO 2011032626 A
- WO 2011066898 A
- WO 2011157339 A
- WO 2012007086 A
- WO 2014008982 A
- WO 2014023377 A
- WO 2014094961 A
- WO 2014094960 A
- WO 2015036074 A
- WO 2015104045 A
- WO 2015117718 A
- WO 2016015815 A
- WO 2016124304 A
- WO 2017032439 A
- EP 16179378
- EP 16186313
- WO 2005011013 A
- WO 2004013080 A
- WO 2004093207 A
- WO 2006005627 A
- WO 2010006680 A
- WO 2014015935 A
- WO 2005039246 A
- US 20050069729 A
- JP 2004288381 A
- EP 1205527 A
- WO 2008086851 A
- WO 2007063754 A
- WO 2008056746 A
- WO 2010136109 A
- WO 2011000455 A
- EP 1617710 A
- EP 1617711 A
- EP 1731584 A
- JP 2005347160 A
- WO 2007137725 A
- WO 005111172 A
- WO 2006117052 A
- WO 2010015306 A
- EP 652273 A
- WO 2009062578 A
- WO 2010054729 A
- WO 2010054730 A
- US 20090136779 A
- WO 2010050778 A
- WO 2011042107 A
- WO 2011088877 A
- WO 2012143080 A
- WO 2012048781 A
- WO 2011116865 A
- WO 2011137951 A
- WO 2013064206 A
- WO 2014094963 A
- WO 2015192939 A
- WO 2015169412 A
- WO 2016015810 A
- WO 2016023608 A
- WO 2017148564 A
- WO 2017148565 A
- WO 2010108579 A
- WO 2005053051 A

- WO 2009030981 A


## In der Beschreibung aufgeführte Nicht-Patentliteratur


- **D. M. KOLLER et al.** *Nature Photonics,* 2008, 1-4
- **Z. B. M. S. ARNOLD et al.** *Appl. Phys. Lett.,* 2008, vol. 92, 053301